(19) Europäisches Patentamt
European Patent Office
Office européen des brevets

(11) EP 2 855 437 B1

(12) # EUROPÄISCHE PATENTSCHRIFT

(45) Veröffentlichungstag und Bekanntmachung des Hinweises auf die Patenterteilung:
**23.03.2016 Patentblatt 2016/12**

(21) Anmeldenummer: **13723813.5**

(22) Anmeldetag: **22.05.2013**

(51) Int Cl.:
***C07D 249/14*** (2006.01)
***C07D 257/06*** (2006.01)
***C07D 401/12*** (2006.01)
***C07D 403/12*** (2006.01)
***C07D 413/12*** (2006.01)
***A01N 43/653*** (2006.01)
***A01N 43/713*** (2006.01)
***A01P 13/00*** (2006.01)
***A01N 43/42*** (2006.01)
***A01N 43/80*** (2006.01)
***A01N 41/10*** (2006.01)

(86) Internationale Anmeldenummer:
**PCT/EP2013/060464**

(87) Internationale Veröffentlichungsnummer:
**WO 2013/174843 (28.11.2013 Gazette 2013/48)**

(54) **N-(TETRAZOL-5-YL)- UND N-(TRIAZOL-5-YL)ARYLCARBONSÄURETHIOAMIDE UND IHRE VERWENDUNG ALS HERBIZIDE**

N-(TETRAZOL-5-YL)- AND N-(TRIAZOL-5-YL)ARYL CARBOXYLIC ACID ATHIOAMIDES AND USE OF SAME AS HERBICIDES

THIOAMIDES DÝACIDE CARBOXYLIQUE DE N-(TÉTRAZOL-5-YL)- ET N-(TRIAZOL-5-YL)ARYLE ET LEUR UTILISATION COMME HERBICIDES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.05.2012 EP 12169234**

(43) Veröffentlichungstag der Anmeldung:
**08.04.2015 Patentblatt 2015/15**

(73) Patentinhaber: **Bayer CropScience AG**
**40789 Monheim am Rhein (DE)**

(72) Erfinder:
- **BRAUN, Ralf**
  **76857 Ramberg (DE)**
- **DÖRNER-RIEPING, Simon**
  **61267 Neu-Anspach (DE)**
- **AHRENS, Hartmut**
  **63329 Egelsbach (DE)**
- **KÖHN, Arnim**
  **55270 Klein-Winternheim (DE)**
- **WALDRAFF, Christian**
  **61118 Bad Vilbel (DE)**
- **DIETRICH, Hansjörg**
  **65835 Liederbach am Taunus (DE)**
- **SCHMUTZLER, Dirk**
  **65795 Hattersheim (DE)**
- **GATZWEILER, Elmar**
  **61231 Bad Nauheim (DE)**
- **ROSINGER, Christopher, Hugh**
  **65719 Hofheim (DE)**

(74) Vertreter: **BIP Patents**
**c/o Bayer Intellectual Property GmbH**
**Alfred-Nobel-Straße 10**
**40789 Monheim am Rhein (DE)**

(56) Entgegenhaltungen:
**EP-A1- 0 049 071**
**WO-A1-2012/028579**
**WO-A1-2013/064458**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 855 437 B1

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

Aus WO 2012/028579 A1 sind N-(Tetrazol-5-yl)- und N-(Triazol-5-yl)-Nicotinamide als Herbizide bekannt. Allerdings zeigen diese Wirkstoffe nicht immer eine ausreichende Wirkung gegen Schadpflanzen und/oder sie sind zum Teil nicht ausreichend verträglich mit einigen wichtigen Kulturpflanzen, wie Getreidearten, Mais oder Reis.

Aufgabe der vorliegenden Erfindung ist es daher, weitere herbizid wirksame Wirkstoffe bereitzustellen. Diese Aufgabe wird durch die nachfolgend beschriebenen erfindungsgemäßen N-(Tetrazol-5-yl)- und N-(Triazol-5-yl)arylcarbonsäurethioamide gelöst, die sich im Wesentlichen durch die Thioamid-Struktur von den aus dem Stand der Technik bekannten Verbindungen unterscheiden.

Ein Gegenstand der vorliegenden Erfindung sind somit N-(Tetrazol-5-yl)- und N-(Triazol-5-yl)arylcarbonsäurethioamide der Formel (I) oder deren Salze

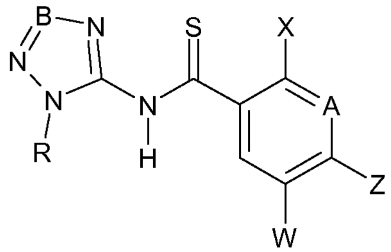

(I),

worin

A bedeutet N oder CY,

B bedeutet N oder CH,

X bedeutet Nitro, Halogen, Cyano, Formyl, Rhodano, $(C_1-C_6)$-Alkyl, Halogen-$(C_1-C_6)$-alkyl, $(C_2-C_6)$-Alkenyl, Halogen-$(C_2-C_6)$-alkenyl, $(C_2-C_6)$-Alkinyl, Halogen-$(C_3-C_6)$-alkinyl, $(C_3-C_6)$-Cycloalkyl, Halogen-$(C_3-C_6)$-cycloalkyl, $(C_3-C_6)$-Cycloalkyl-$(C_1-C_6)$-alkyl, Halogen-$(C_3-C_6)$-Cycloalkyl-$(C_1-C_6)$-alkyl, $COR^1$, $COOR^1$, $OCOOR^1$, $NR^1COOR^1$, $C(O)N(R^1)_2$, $NR^1C(O)N(R^1)_2$, $OC(O)N(R^1)_2$, $C(O)NR^1OR^1$, $OR^1$, $OCOR^1$, $OSO_2R^2$, $S(O)_nR^2$, $SO_2OR^1$, $SO_2N(R^1)_2$, $NR^1SO_2R^2$, $NR^1COR^1$, $(C_1-C_6)$-Alkyl-$S(O)_nR^2$, $(C_1-C_6)$-Alkyl-$OR^1$, $(C_1-C_6)$-Alkyl-$OCOR^1$, $(C_1-C_6)$-Alkyl-$OSO_2R^2$, $(C_1-C_6)$-Alkyl-$CO_2R^1$, $(C_1-C_6)$-Alkyl-$SO_2OR^1$, $(C_1-C_6)$-Alkyl-$CON(R^1)_2$, $(C_1-C_6)$-Alkyl-$SO_2N(R^1)_2$, $(C_1-C_6)$-Alkyl-$NR^1COR^1$, $(C_1-C_6)$-Alkyl-$NR^1SO_2R^2$, $NRiR_2$, $P(O)(OR^5)_2$, $CH_2P(O)(OR^5)_2$, $(C_1-C_6)$-Alkyl-Heteroaryl oder $(C_1-C_6)$-Alkyl-Heterocyclyl, wobei die beiden letztgenannten Reste jeweils durch s Reste aus der Gruppe bestehend aus Halogen, $(C_1-C_6)$-Alkyl, Halogen-$(C_1-C_6)$-alkyl, $S(O)_n$-$(C_1-C_6)$-Alkyl, $(C_1-C_6)$-Alkoxy und Halogen-$(C_1-C_6)$-alkoxy substituiert sind, und wobei Heterocyclyl n Oxogruppen trägt,

Y bedeutet Wasserstoff, Nitro, Halogen, Cyano, Rhodano, $(C_1-C_6)$-Alkyl, Halogen-$(C_1-C_6)$-alkyl, $(C_2-C_6)$-Alkenyl, Halogen-$(C_2-C_6)$-alkenyl, $(C_2-C_6)$-Alkinyl, Halogen-$(C_2-C_6)$-alkinyl, $(C_3-C_6)$-Cycloalkyl, $(C_3-C_6)$-Cycloalkenyl, Halogen-$(C_3-C_6)$-cycloalkyl, $(C_3-C_6)$-Cycloalkyl-$(C_1-C_6)$-alkyl, Halogen-$(C_3-C_6)$-Cycloalkyl-$(C_1-C_6)$-alkyl, $COR^1$, $COOR^1$, $OCOOR^1$, $NR^1COOR^1$, $C(O)N(R^1)_2$, $NR^1C(O)N(R^1)_2$, $OC(O)N(R^1)_2$, $CO(NOR^1)R^1$, $CHNOR^1$, $CH_2ONC(R^3)_2$, $NR^1SO_2R^2$, $NR^1COR^1$, $OR^1$, $OSO_2R^2$, $S(O)_nR^2$, $SO_2OR^1$, $SO_2N(R^1)_2$ $(C_1-C_6)$-Alkyl-$S(O)_nR^2$, $NS(O)R^6R^7$, $S(O)R^8NR^9$, $(C_1-C_6)$-Alkyl-$OR^1$, $(C_1-C_6)$-Alkyl-$OCOR^1$, $(C_1-C_6)$-Alkyl-OSO2$R^2$, $(C_1-C_6)$-Alkyl-$CO_2R^1$, $(C_1-C_6)$-Alkyl-CN, $(C_1-C_6)$-Alkyl-$SO_2OR^1$, $(C_1-C_6)$-Alkyl-$CON(R^1)_2$, $(C_1-C_6)$-Alkyl-$SO_2N(R^1)_2$, $(C_1-C_6)$-Alkyl-$NR^1COR^1$, $(C_1-C_6)$-Alkyl-$NR^1SO_2R^2$, $N(R^1)_2$, $P(O)(OR^5)_2$, $CH_2P(O)(OR^5)_2$, $(C_1-C_6)$-Alkyl-Phenyl, $(C_1-C_6)$-Alkyl-Heteroaryl, $(C_1-C_6)$-Alkyl-Heterocyclyl, Phenyl, Heteroaryl oder Heterocyclyl, wobei die sechs letztgenannten Reste jeweils durch s Reste aus der Gruppe bestehend aus Halogen, Nitro, Cyano, $(C_1-C_6)$-Alkyl, Halogen-$(C_1-C_6)$-alkyl, $(C_3-C_6)$-Cycloalkyl, $S(O)_n$-$(C_1-C_6)$-Alkyl, $(C_1-C_6)$-Alkoxy, Halogen-$(C_1-C_6)$-alkoxy, $(C_1-C_6)$-Alkoxy-$(C_1-C_4)$-alkyl und Cyanomethyl substituiert sind, und wobei Heterocyclyl n Oxogruppen trägt,

Z bedeutet Halogen, Cyano, Nitro, Rhodano, Halogen-$(C_1-C_6)$-alkyl, $(C_2-C_6)$-Alkenyl, Halogen-$(C_2-C_6)$-alkenyl, $(C_2-C_6)$-Alkinyl, Halogen-$(C_2-C_6)$-alkinyl, $(C_3-C_6)$-Cycloalkyl, Halogen-$(C_3-C_6)$-cycloalkyl, $(C_3-C_6)$-Cycloalkyl-$(C_1-C_6)$-alkyl, Halogen-$(C_3-C_6)$-cycloalkyl-$(C_1-C_6)$-alkyl, $COR^1$, $COOR^1$, $OCOOR^1$, $NR^1COOR^1$, $C(O)N(R^1)_2$, $NR^1C(O)N(R^1)_2$, $OC(O)N(R^1)_2$, $C(O)NR^1OR^1$, $OSO_2R^2$,$S(O)_nR^2$,$SO_2OR^1$, $SO_2N(R^1)_2$, $NR^1SO_2R^2$, $NR^1COR^1$,

$(C_1-C_6)$-Alkyl-$S(O)_nR^2$, $(C_1-C_6)$-Alkyl-$OR^1$, $(C_1-C_6)$-Alkyl-$OCOR^1$, $(C_1-C_6)$-Alkyl-$OSO_2R^2$, $(C_1-C_6)$-Alkyl-$CO_2R^1$, $(C_1-C_6)$-Alkyl-$SO_2OR^1$, $(C_1-C_6)$-Alkyl-$CON(R^1)_2$, $(C_1-C_6)$-Alkyl-$SO_2N(R^1)_2$, $(C_1-C_6)$-Alkyl-$NR^1COR^1$, $(C_1-C_6)$-Alkyl-$NR^1SO_2R^2$, $N(R^1)_2$, $P(O)(OR^5)_2$, Heteroaryl, Heterocyclyl oder Phenyl, wobei die drei letztgenannten Reste jeweils durch s Reste aus der Gruppe bestehend aus Halogen, Nitro, Cyano, $(C_1-C_6)$-Alkyl, Halogen-$(C_1-C_6)$-alkyl, $(C_3-C_6)$-Cycloalkyl, $S(O)_n$-$(C_1-C_6)$-Alkyl, $(C_1-C_6)$-Alkoxy oder Halogen-$(C_1-C_6)$-alkoxy substituiert sind, und wobei Heterocyclyl n Oxogruppen trägt, oder

Z kann auch Wasserstoff, $(C_1-C_6)$-Alkyl oder $(C_1-C_6)$-Alkoxy bedeuten, falls Y für den Rest $S(O)_nR^2$ steht,

W bedeutet Wasserstoff, $(C_1-C_6)$-Alkyl, Halogen-$(C_1-C_6)$-alkyl, $(C_2-C_6)$-Alkenyl, Halogen-$(C_2-C_6)$-alkenyl, $(C_2-C_6)$-Alkinyl, Halogen-$(C_2-C_6)$-alkinyl, $(C_3-C_7)$-Cycloalkyl, $(C_3-C_7)$-Halogencycloalkyl, $(C_1-C_6)$-Alkoxy, $(C_1-C_6)$-Halogenalkoxy, $S(O)_n$-$(C_1-C_6)$-Alkyl, $S(O)_n$-$(C_1-C_6)$-Halogenalkyl, $(C_1-C_6)$-Alkoxy-$(C_1-C_4)$-alkyl, $(C_1-C_6)$-Alkoxy-$(C_1-C_4)$-halogenalkyl, Halogen, Nitro, $NR^3COR^3$ oder Cyano,

R bedeutet $(C_1-C_8)$-Alkyl, Halogen-$(C_1-C_8)$-alkyl, $(C_2-C_8)$-Alkenyl, Halogen-$(C_2-C_8)$-alkenyl, $(C_2-C_8)$-Alkinyl oder Halogen-$(C_2-C_8)$-alkinyl, wobei diese sechs vorstehend genannten Reste jeweils durch s Reste aus der Gruppe bestehend aus Hydroxy, Nitro, Cyano, $SiR^5_3$, $PO(OR^5)_2$, $S(O)_n$-$(C_1-C_6)$-Alkyl, $S(O)_n$-$(C_1-C_6)$-Halogenalkyl, $(C_1-C_6)$-Alkoxy, Halogen-$(C_1-C_6)$-alkoxy, $N(R^3)_2$, $COR^3$, $COOR^3$, $OCOR^3$, $NR^3COR^3$, $NR^3SO_2R^4$, $O(C_1-C_2)$-Alkyl-$(C_3-C_6)$-Cycloalkyl, $(C_3-C_6)$-Cycloalkyl, Heteroaryl, Heterocyclyl, Phenyl, Q-Heteroaryl, Q-Heterocyclyl, Q-Phenyl und Q-Benzyl substituiert sind, wobei die sieben letztgenannten Reste jeweils durch s Reste aus der Gruppe bestehend aus Methyl, Ethyl, Methoxy, Trifluormethyl, Cyano und Halogen substituiert sind, und wobei Heterocyclyl n Oxogruppen trägt, oder

R bedeutet jeweils durch s Reste aus der Gruppe bestehend aus Halogen, Nitro, Cyano, $(C_1-C_6)$-Alkyl, Halogen-$(C_1-C_6)$-alkyl, $(C_3-C_6)$-Cycloalkyl, $S(O)_n$-$(C_1-C_6)$-Alkyl, $(C_1-C_6)$-Alkoxy, Halogen-$(C_1-C_6)$-alkoxy und $(C_1-C_6)$-Alkoxy-$(C_1-C_4)$-alkyl substituiertes $(C_3-C_7)$-Cycloalkyl, Heteroaryl, Heterocyclyl oder Phenyl, wobei Heterocyclyl n Oxogruppen trägt,

Q bedeutet O, S, oder $NR^3$,

$R^1$ bedeutet Wasserstoff, $(C_1-C_6)$-Alkyl, $(C_1-C_6)$-Halogenalkyl, $(C_2-C_6)$-Alkenyl, $(C_2-C_6)$-Halogenalkenyl, $(C_2-C_6)$-Alkinyl, $(C_2-C_6)$-Halogenalkinyl, $(C_3-C_6)$-Cycloalkyl, $(C_3-C_6)$-Cycloalkenyl, $(C_3-C_6)$-Halogencycloalkyl, $(C_1-C_6)$-Alkyl-O-$(C_1-C_6)$-alkyl, $(C_3-C_6)$-Cycloalkyl-$(C_1-C_6)$-alkyl, Phenyl, Phenyl-$(C_1-C_6)$-alkyl, Heteroaryl, $(C_1-C_6)$-Alkyl-Heteroaryl, Heterocycl, $(C_1-C_6)$-Alkyl-Heterocyclyl, $(C_1-C_6)$-Alkyl-O-Heteroaryl, $(C_1-C_6)$-Alkyl-O-Heterocyclyl, $(C_1-C_6)$-Alkyl-$NR^3$-Heteroaryl oder $(C_1-C_6)$-Alkyl-$NR^3$-Heterocyclyl, wobei die 21 letztgenannten Reste jeweils durch s Reste aus der Gruppe bestehend aus Cyano, Halogen, Nitro, Rhodano, $OR^3$, $S(O)_nR^4$, $N(R^3)_2$, $NR^3OR^3$, $COR^3$, $OCOR^3$, $SCOR^4$, $NR^3COR^3$, $NR^3SO_2R^4$, $CO_2R^3$, $COSR^4$, $CON(R^3)_2$ und $(C_1-C_4)$-Alkoxy-$(C_2-C_6)$-alkoxycarbonyl substituiert sind, und wobei Heterocyclyl n Oxogruppen trägt,

$R^2$ bedeutet $(C_1-C_6)$-Alkyl, $(C_1-C_6)$-Halogenalkyl, $(C_2-C_6)$-Alkenyl, $(C_2-C_6)$-Halogenalkenyl, $(C_2-C_6)$-Alkinyl, $(C_2-C_6)$-Halogenalkinyl, $(C_3-C_6)$-Cycloalkyl, $(C_3-C_6)$-Cycloalkenyl, $(C_3-C_6)$-Halogencycloalkyl, $(C_1-C_6)$-Alkyl-O-$(C_1-C_6)$-alkyl, $(C_3-C_6)$-Cycloalkyl-$(C_1-C_6)$-alkyl, Phenyl, Phenyl-$(C_1-C_6)$-alkyl, Heteroaryl, $(C_1-C_6)$-Alkyl-Heteroaryl, Heterocyclyl, $(C_1-C_6)$-Alkyl-Heterocyclyl, $(C_1-C_6)$-Alkyl-O-Heteroaryl, $(C_1-C_6)$-Alkyl-O-Heterocyclyl, $(C_1-C_6)$-Alkyl-$NR^3$-Heteroaryl oder $(C_1-C_6)$-Alkyl-$NR^3$-Heterocyclyl, wobei die 21 letztgenannten Reste jeweils durch s Reste aus der Gruppe bestehend aus Cyano, Halogen, Nitro, Rhodano, $OR^3$, $S(O)_nR^4$, $N(R^3)_2$, $NR^3OR^3$, $COR^3$, $OCOR^3$, $SCOR^4$, $NR^3COR^3$, $NR^3SO_2R^4$, $CO_2R^3$, $COSR^4$, $CON(R^3)_2$ und $(C_1-C_4)$-Alkoxy-$(C_2-C_6)$-alkoxycarbonyl substituiert sind, und wobei Heterocyclyl n Oxogruppen trägt,

$R^3$ bedeutet Wasserstoff, $(C_1-C_6)$-Alkyl, $(C_2-C_6)$-Alkenyl, $(C_2-C_6)$-Alkinyl, $(C_3-C_6)$-Cycloalkyl, $(C_3-C_6)$-Cycloalkyl-$(C_1-C_6)$-alkyl oder Phenyl,

$R^4$ bedeutet $(C_1-C_6)$-Alkyl, $(C_2-C_6)$-Alkenyl, $(C_2-C_6)$-Alkinyl, $(C_3-C_6)$-Cycloalkyl, $(C_3-C_6)$-Cycloalkyl-$(C_1-C_6)$-alkyl oder Phenyl,

$R^5$ bedeutet $(C_1-C_4)$-Alkyl,

$R^6$ und $R^7$ bedeuten unabhängig voneinander jeweils $(C_1-C_6)$-Alkyl, Halogen-$(C_1-C_6)$-alkyl, $(C_2-C_6)$-Alkenyl, Halogen-$(C_2-C_6)$-alkenyl, $(C_2-C_6)$-Alkinyl, Halogen-$(C_3-C_6)$-alkinyl, $(C_3-C_6)$-Cycloalkyl, Halogen-$(C_3-C_6)$-cycloalkyl,

$(C_3-C_6)$-Cycloalkyl-$(C_1-C_6)$-alkyl, Halogen-$(C_3-C_6)$-cycloalkyl-$(C_1-C_6)$-alkyl, $(C_1-C_6)$-Alkoxy-$(C_1-C_6)$-alkyl, Halogen-$(C_1-C_6)$-alkoxy-$(C_1-C_6)$-alkyl, Phenyl, Heteroaryl oder Heterocyclyl, wobei die drei letztgenannten Reste jeweils durch s Reste aus der Gruppe bestehend aus Nitro, Halogen, Cyano, Rhodano, $(C_1-C_6)$-Alkyl, Halogen-$(C_1-C_6)$-alkyl, $(C_3-C_6)$-Cycloalkyl, $R^1O(O)C$, $(R^1)_2N(O)C$, $R^1O$, $(R^1)_2N$, $R^2(O)_nS$, $R^1O(O)_2S$, $(R^1)_2N(O)_2S$ und $R^1O$-$(C_1-C_6)$-Alkyl substituiert sind, und wobei Heterocyclyl n Oxogruppen trägt, oder $R^6$ und $R^7$ bilden gemeinsam mit dem Schwefel-Atom, an dem sie gebunden sind, einen 3- bis 8-gliedrigen ungesättigten, teilgesättigten oder gesättigten Ring, der außer den Kohlenstoffatomen und außerdem Schwefelatom der Sulfoximinogruppe jeweils m Ringglieder aus der Gruppe bestehend aus $N(R^1)$, O und $S(O)_n$ enthält, und wobei dieser Ring jeweils durch s Reste aus der Gruppe bestehend aus Nitro, Halogen, Cyano, Rhodano, $(C_1-C_6)$-Alkyl, Halogen-$(C_1-C_6)$-alkyl, $(C_3-C_6)$-Cycloalkyl, $R^1O(O)C$, $(R^1)_2N(O)C$, $R^1O$, $(R^1)_2N$, $R^2(O)_nS$, $R^1O(O)_2S$, $(R^1)_2N(O)_2S$ und $R^1O$-$(C_1-C_6)$-Alkyl, substituiert ist, und wobei dieser Ring n Oxogruppen trägt,

$R^8$ bedeutet jeweils durch s Reste aus der Gruppe bestehend aus Nitro, Halogen, Cyano, Rhodano, $(C_3-C_6)$-Cycloalkyl, $R^1(O)C$, $R^1(R^1ON{=})C$, $R^1O(O)C$, $(R^1)_2N(O)C$, $R^1(R^1O)N(O)C$, $R^2(O)_2S(R^1)N(O)C$, $R^1O(O)_2S(R^1)N(O)C$, $(R^1)_2N(O)_2S(R^1)N(O)C$, $R^1S(O)C$, $R^1O$, $R^1(O)CO$, $R^2(O)_2SO$, $R^2O(O)CO$, $(R^1)_2N(O)CO$, $(R^1)_2N$, $R^1O(R^1)N$, $R^1(O)C(R^1)N$, $R^2(O)_2S(R^1)N$, $R^2O(O)C(R^1)N$, $(R^1)_2N(O)C(R^1)N$, $R^1O(O)_2S(R^1)N$, $(R^1)_2N(O)_2S(R^1)N$, $R^2(O)_nS$, $R^1C(O)S$, $R^1O(O)_2S$, $(R^1)_2N(O)_2S$, $R^1(O)C(R^1)N(O)_2S$, $R^2O(O)C(R^1)N(O)_2S$, $(R^1)_2N(O)C(R^1)N(O)_2S$ und $(R^5O)_2(O)P$ substituiertes $(C_1-C_6)$-Alkyl, $(C_2-C_6)$-Alkenyl oder $(C_2-C_6)$-Alkinyl,
oder

jeweils durch s Reste aus der Gruppe bestehend aus Nitro, Halogen, Cyano, Rhodano, $(C_1-C_6)$-Alkyl, Halogen-$(C_1-C_6)$-alkyl, $(C_3-C_6)$-Cycloalkyl, $R^1(O)C$, $R^1(R^1ON{=})C$, $R^1O(O)C$, $(R^1)_2N(O)C$, $R^1(R^1O)N(O)C$, $R^2(O)_2S(R^1)N(O)C$, $R^1O(O)_2S(R^1)N(O)C$, $(R^1)_2N(O)_2S(R^1)N(O)C$, $R^1S(O)C$, $R^1O$, $R^1(O)CO$, $R^2(O)_2SO$, $R^2O(O)CO$, $(R^1)_2N(O)CO$, $(R^1)_2N$, $R^1O(R^1)N$, $R^1(O)C(R^1)N$, $R^2(O)_2S(R^1)N$, $R^2O(O)C(R^1)N$, $(R^1)_2N(O)C(R^1)N$, $R^1O(O)_2S(R^1)N$, $(R^1)_2N(O)_2S(R^1)N$, $R^2(O)_nS$, $R^1C(O)S$, $R^1O(O)_2S$, $(R^1)_2N(O)_2S$, $R^1(O)C(R^1)N(O)_2S$, $R^2O(O)C(R^1)N(O)_2S$, $(R^1)_2N(O)C(R^1)N(O)_2S$, $(R^5O)_2(O)P$ und $R^1O$-$(C_1-C_6)$-Alkyl im cyclischen Teil substituiertes $(C_3-C_6)$-Cycloalkyl, $(C_3-C_6)$-Cycloalkenyl, Phenyl, Phenyl-$(C_1-C_6)$-alkyl, Heteroaryl, Heteroaryl-$(C_1-C_6)$-alkyl, Heterocyclyl, Heterocyclyl-$(C_1-C_6)$-alkyl, Phenyl-O-$(C_1-C_6)$-alkyl, Heteroaryl-O-$(C_1-C_6)$-alkyl, Heterocyclyl-O-$(C_1-C_6)$-alkyl, Phenyl-$N(R^1)$-$(C_1-C_6)$-alkyl, Heteroaryl- $N(R^1)$-$(C_1-C_6)$-alkyl, Heterocyclyl- $N(R^1)$-$(C_1-C_6)$-alkyl, Phenyl-$S(O)_n$-$(C_1-C_6)$-alkyl, Heteroaryl-$S(O)_n$-$(C_1-C_6)$-alkyl oder Heterocyclyl-$S(O)_n$-$(C_1-C_6)$-alkyl, und wobei Heterocyclyl n Oxogruppen trägt,

$R^9$ bedeutet Wasserstoff, Nitro, Halogen, Cyano, $(C_1-C_6)$-Alkyl, Halogen-$(C_1-C_6)$-alkyl, $(C_3-C_6)$-Alkenyl, Halogen-$(C_3-C_6)$-alkenyl, $(C_2-C_6)$-Alkinyl, Halogen-$(C_3-C_6)$-alkinyl, $(C_3-C_6)$-Cycloalkyl, Halogen-$(C_3-C_6)$-cycloalkyl, $(C_3-C_6)$-Cycloalkyl-$(C_1-C_6)$-alkyl, Halogen-$(C_3-C_6)$-Cycloalkyl-$(C_1-C_6)$-alkyl, $R^1(O)C$, $R^2O(O)C$, $(R^1)_2N(O)C$, $R^2S(O)C$, $(R^1)_2N(S)C$, $R^1(R^1O)N(O)C$, $R^2(O)_2S(R^1)N(O)C$, $(R^1)_2N(O)_2S(R^1)N(O)C$, $R^1O$, $(R^1)_2N$, $R^2(O)_nS$, $(R^2)_3Si$-$(C_1-C_6)$-Alkyl-$(O)_nS$, $R^1O(O)_2S$, $(R^1)_2N(O)_2S$, $R^1(O)C(R^1)N(O)_2S$, $R^2O(O)C(R^1)N(O)_2S$, $(R^1)_2N(O)C(R^1)N(O)_2S$, $R^2(O)_2S(R^1)N(O)_2S$, $(R^5O)_2(O)P$, $(R^2)_3Si$, $R^1(O)C$-$(C_1-C_6)$-Alkyl, $R^1O(O)C$-$(C_1-C_6)$-Alkyl, $(R^1)_2N(O)C$-$(C_1-C_6)$-Alkyl, $(R^1O)(R^1)N(O)C$-$(C_1-C_6)$-Alkyl, $R^2(O)_2S(R^1)N(O)C$-$(C_1-C_6)$-Alkyl, $R^1O(O)_2S(R^1)N(O)C$-$(C_1-C_6)$-Alkyl, $(R^1)_2N(O)_2S(R^1)N(O)C$-$(C_1-C_6)$-Alkyl, $R^1O$-$(C_1-C_6)$-Alkyl, $R^1(O)CO$-$(C_1-C_6)$-Alkyl, $R^2(O)_2SO$-$(C_1-C_6)$-Alkyl, $R^2O(O)CO$-$(C_1-C_6)$-Alkyl, $(R^1)_2N(O)CO$-$(C_1-C_6)$-Alkyl, $(R^1)_2N$-$(C_1-C_6)$-Alkyl, $R^1(O)C(R^1)N$-$(C_1-C_6)$-Alkyl, $R^2(O)_2S(R^1)N$-$(C_1-C_6)$-Alkyl, $R^2O(O)C(R^1)N$-$(C_1-C_6)$-Alkyl, $(R^1)_2N(O)C(R^1)N$-$(C_1-C_6)$-Alkyl, $R^1O(O)_2S(R^1)N$-$(C_1-C_6)$-Alkyl, $(R^1)_2N(O)_2S(R^1)N$-$(C_1-C_6)$-Alkyl, $R^2(O)_nS$-$(C_1-C_6)$-Alkyl, $R^1O(O)_2S$-$(C_1-C_6)$-Alkyl, $(R^1)_2N(O)_2S$-$(C_1-C_6)$-Alkyl, $R^1(O)C(R^1)N(O)_2S$-$(C_1-C_6)$-Alkyl, $R^2O(O)C(R^1)N(O)_2S$-$(C_1-C_6)$-Alkyl, $(R^1)_2N(O)C(R^1)N(O)_2S$-$(C_1-C_6)$-Alkyl, $(R^5O)_2(O)P$-$(C_1-C_6)$-Alkyl, $(R^2)_3Si$-$(C_1-C_6)$-Alkyl,
oder

jeweils durch s Reste aus der Gruppe bestehend aus Nitro, Halogen, Cyano, Rhodano, $(C_1-C_6)$-Alkyl, Halogen-$(C_1-C_6)$-alkyl, $(C_3-C_6)$-Cycloalkyl, $R^1O(O)C$, $(R^1)_2N(O)C$, $R^1O$, $(R^1)_2N$, $R^2(O)_nS$, $R^1O(O)_2S$, $(R^1)_2N(O)_2S$ und $R^1O$-$(C_1-C_6)$-Alkyl im cyclischen Teil substituiertes Phenyl, Heteroaryl, Heterocyclyl, Phenyl-$(C_1-C_6)$-alkyl, Heteroaryl-$(C_1-C_6)$-alkyl oder Heterocyclyl-$(C_1-C_6)$-alkyl, und wobei Heterocyclyl n Oxogruppen trägt,

m bedeutet 0, 1, 2, 3 oder 4,

n bedeutet 0, 1 oder 2,

s bedeutet 0, 1, 2 oder 3.

In der Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Analog bedeutet Alkenyl z.B. Allyl, 1-Methylprop-2-en-1-yl, 2-Methylprop-2-en-1-yl, But-2-en-1-yl, But-3-en-1-yl, 1-Methyl-but-3-en-1-yl und 1-Methyl-but-2-en-1-yl. Alkinyl bedeutet z.B. Propargyl, But-2-in-1-yl, But-3-in-1-yl, 1-Methyl-but-3-in-1-yl. Die Mehrfachbindung kann sich jeweils in beliebiger Position des ungesättigten Rests befinden. Cycloalkyl bedeutet ein carbocyclisches, gesättigtes Ringsystem mit drei bis sechs C-Atomen, z.B. Cyclopropyl, Cyclobutyl, Cyclopentyl oder Cyclohexyl. Analog bedeutet Cycloalkenyl eine monocyclische Alkenylgruppe mit drei bis sechs Kohlenstoffringgliedern, z.B. Cyclopropenyl, Cyclobutenyl, Cyclopentenyl und Cyclohexenyl, wobei sich die Doppelbindung an beliebiger Position befinden kann.

Halogen steht für Fluor, Chlor, Brom oder Iod.

Heterocyclyl bedeutet einen gesättigten, teilgesättigten oder vollständig ungesättigten cyclischen Rest, der 3 bis 6 Ringatome enthält, von denen 1 bis 4 aus der Gruppe bestehend aus Sauerstoff, Stickstoff und Schwefel stammen, und der zusätzlich durch einen Benzoring annelliert sein kann. Beispielsweise steht Heterocyclyl für Piperidinyl, Pyrrolidinyl, Tetrahydrofuranyl, Dihydrofuranyl und Oxetanyl,

Heteroaryl bedeutet einen aromatischen cyclischen Rest, der 3 bis 6 Ringatome enthält, von denen 1 bis 4 aus der Gruppe bestehend aus Sauerstoff, Stickstoff und Schwefel stammen, und der zusätzlich durch einen Benzoring annelliert sein kann. Beispielsweise steht Heteroaryl für Benzimidazol-2-yl, Furanyl, Imidazolyl, Isoxazolyl, Isothiazolyl, Oxazolyl, Pyrazinyl, Pyrimidinyl, Pyridazinyl, Pyridinyl, Benzisoxazolyl, Thiazolyl, Pyrrolyl, Pyrazolyl, Thiophenyl, 1,2,3-Oxadiazolyl, 1,2,4-Oxadiazolyl, 1,2,5-Oxadiazolyl, 1,3,4-Oxadiazolyl, 1,2,4-Triazolyl, 1,2,3-Triazolyl, 1,2,5-Triazolyl, 1,3,4-Triazolyl, 1,2,4-Triazolyl, 1,2,4-Thiadiazolyl, 1,3,4-Thiadiazolyl, 1,2,3-Thiadiazolyl, 1,2,5-Thiadiazolyl, 2H-1,2,3,4-Tetrazolyl, 1 H-1,2,3,4-Tetrazolyl, 1,2,3,4-Oxatriazolyl, 1,2,3,5-Oxatriazolyl, 1,2,3,4-Thiatriazolyl und 1,2,3,5-Thiatriazolyl.

Ist eine Gruppe mehrfach durch Reste substituiert, so ist darunter zu verstehen, daß diese Gruppe durch ein oder mehrere gleiche oder verschiedene der genannten Reste substituiert ist. Analoges gilt für den Aufbau von Ringsystemen durch verschiedene Atome und Elemente. Dabei sollen solche Verbindungen vom Anspruchsbegehren ausgenommen sein, von denen der Fachmann weiß, dass sie unter Normalbedingungen chemisch instabil sind.

Die Verbindungen der allgemeinen Formel (I) können je nach Art und Verknüpfung der Substituenten als Stereoisomere vorliegen. Sind beispielsweise ein oder mehrere asymmetrische Kohlenstoffatome vorhanden, so können Enantiomere und Diastereomere auftreten. Ebenso treten Stereoisomere auf, wenn n für 1 steht (Sulfoxide). Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereo-selektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfasst, jedoch nicht spezifisch definiert sind.

Die Verbindungen der Formel (I) können Salze bilden. Salzbildung kann durch Einwirkung einer Base auf Verbindungen der Formel (I) erfolgen. Geeignete Basen sind beispielsweise organische Amine, wie Trialkylamine, Morpholin, Piperidin und Pyridin sowie Ammonium-, Alkali- oder Erdalkalimetallhydroxide, -carbonate und -hydrogencarbonate, insbesondere Natrium- und Kaliumhydroxid, Natrium- und Kaliumcarbonat und Natrium- und Kaliumhydrogencarbonat. Diese Salze sind Verbindungen, in denen der acide Wasserstoff durch ein für die Landwirtschaft geeignetes Kation ersetzt wird, beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre Ammoniumsalze, zum Beispiel mit Kationen der Formel $[NR^aR^bR^cR^d]^+$, worin $R^a$ bis $R^d$ jeweils unabhängig voneinander einen organischen Rest, insbesondere Alkyl, Aryl, Aralkyl oder Alkylaryl darstellen. Infrage kommen auch Alkylsulfonium- und Alkylsulfoxoniumsalze, wie $(C_1$-$C_4)$-Trialkylsulfonium- und $(C_1$-$C_4)$-Trialkylsulfoxoniumsalze.

Die Verbindungen der Formel (I) können durch Anlagerung einer geeigneten anorganischen oder organischen Säure, wie beispielsweise Mineralsäuren, wie beispielsweise HCl, HBr, $H_2SO_4$, $H_3PO_4$ oder $HNO_3$, oder organische Säuren, z. B. Carbonsäuren, wie Ameisensäure, Essigsäure, Propionsäure, Oxalsäure, Milchsäure oder Salicylsäure oder Sulfonsäuren, wie zum Beispiel p-Toluolsulfonsäure, an eine basische Gruppe, wie z.B. Amino, Alkylamino, Dialkylamino, Piperidino, Morpholino oder Pyridino, Salze bilden.

Bevorzugt sind Verbindungen der allgemeinen Formel (I), worin

A bedeutet N oder CY,

B bedeutet N oder CH,

X bedeutet Nitro, Halogen, Cyano, Rhodano, $(C_1$-$C_6)$-Alkyl, Halogen-$(C_1$-$C_6)$-alkyl, $(C_2$-$C_6)$-Alkenyl, Halogen-$(C_2$-$C_6)$-alkenyl, $(C_2$-$C_6)$-Alkinyl, Halogen-$(C_3$-$C_6)$-alkinyl, $(C_3$-$C_6)$-Cycloalkyl, Halogen-$(C_3$-$C_6)$-cycloalkyl, $(C_1$-$C_6)$-Alkyl-O-$(C_1$-$C_6)$-alkyl, $(C_3$-$C_6)$-Cycloalkyl-$(C_1$-$C_6)$-alkyl, Halogen-$(C_3$-$C_6)$-cycloalkyl-$(C_1$-$C_6)$-alkyl, $COR^1$,

$OR^1$, $OCOR^1$, $OSO_2R^2$, $S(O)_nR^2$, $SO_2OR^1$, $SO_2N(R^1)_2$, $NR^1SO_2R^2$, $NR^1COR^1$, ($C_1$-$C_6$)-Alkyl-$S(O)_nR^2$, ($C_1$-$C_6$)-Alkyl-$OR^1$, ($C_1$-$C_6$)-Alkyl-$OCOR^1$, ($C_1$-$C_6$)-Alkyl-$OSO_2R^2$, ($C_1$-$C_6$)-Alkyl-$CO_2R^1$, ($C_1$-$C_6$)-Alkyl-$SO_2OR^1$, ($C_1$-$C_6$)-Alkyl-$CON(R^1)_2$, ($C_1$-$C_6$)-Alkyl-$SO_2N(R^1)_2$, ($C_1$-$C_6$)-Alkyl-$NR^1COR^1$ oder ($C_1$-$C_6$)-Alkyl-$NR^1SO_2R^2$, ($C_1$-$C_6$)-Alkyl-Heteroaryl oder ($C_1$-$C_6$)-Alkyl-Heterocyclyl, wobei die beiden letztgenannten Reste jeweils durch s Reste aus der Gruppe bestehend aus Halogen, ($C_1$-$C_6$)-Alkyl, Halogen-($C_1$-$C_6$)-alkyl, $S(O)_n$-($C_1$-$C_6$)-Alkyl, ($C_1$-$C_6$)-Alkoxy und Halogen-($C_1$-$C_6$)-alkoxy substituiert sind, und wobei Heterocyclyl n Oxogruppen trägt,

Y bedeutet Wasserstoff, Nitro, Halogen, Cyano, Rhodano, ($C_1$-$C_6$)-Alkyl, Halogen-($C_1$-$C_6$)-alkyl, ($C_2$-$C_6$)-Alkenyl, Halogen-($C_2$-$C_6$)-alkenyl, ($C_2$-$C_6$)-Alkinyl, Halogen-($C_3$-$C_6$)-alkinyl, ($C_3$-$C_6$)-Cycloalkyl, ($C_3$-$C_6$)-Cycloalkenyl, Halogen-($C_3$-$C_6$)-cycloalkyl, ($C_3$-$C_6$)-Cycloalkyl-($C_1$-$C_6$)-alkyl, Halogen-($C_3$-$C_6$)-Cycloalkyl-($C_1$-$C_6$)-alkyl, $COR^1$, $OR^1$, $COOR^1$, $CHNOR^1$, $CH_2ONC(R^3)_2$, $OSO_2R^2$, $S(O)_nR^2$, $SO_2OR^1$, $SO_2N(R^1)_2$, $NS(O)R^6R^7$, $S(O)R^8NR^9$, $N(R^1)_2$, $NR^1SO_2R^2$, $NR^1COR^1$, ($C_1$-$C_6$)-Alkyl-$S(O)_nR^2$, ($C_1$-$C_6$)-Alkyl-$OR^1$, ($C_1$-$C_6$)-Alkyl-$OCOR^1$, ($C_1$-$C_6$)-Alkyl-$OSO_2R^2$, ($C_1$-$C_6$)-Alkyl-$CO_2R^1$, ($C_1$-$C_6$)-Alkyl-$SO_2OR^1$, ($C_1$-$C_6$)-Alkyl-$CON(R^1)_2$, ($C_1$-$C_6$)-Alkyl-$SO_2N(R^1)_2$, ($C_1$-$C_6$)-Alkyl-$NR^1COR^1$, ($C_1$-$C_6$)-Alkyl-$NR^1SO_2R^2$, ($C_1$-$C_6$)-Alkyl-Phenyl, ($C_1$-$C_6$)-Alkyl-Heteroaryl, ($C_1$-$C_6$)-Alkyl-Heterocyclyl, Phenyl, Heteroaryl oder Heterocyclyl, wobei die sechs letztgenannten Reste jeweils durch s Reste aus der Gruppe bestehend Halogen, Nitro, Cyano, ($C_1$-$C_6$)-Alkyl, Halogen-($C_1$-$C_6$)-alkyl, ($C_3$-$C_6$)-Cycloalkyl, $S(O)_n$-($C_1$-$C_6$)-Alkyl, ($C_1$-$C_6$)-Alkoxy, Halogen-($C_1$-$C_6$)-alkoxy, ($C_1$-$C_6$)-Alkoxy-($C_1$-$C_4$)-alkyl und Cyanomethyl substituiert sind, und wobei Heterocyclyl n Oxogruppen trägt,

Z bedeutet Halogen, Cyano, Nitro, Rhodano, Halogen-($C_1$-$C_6$)-alkyl, ($C_2$-$C_6$)-Alkenyl, Halogen-($C_2$-$C_6$)-alkenyl, ($C_2$-$C_6$)-Alkinyl, Halogen-($C_3$-$C_6$)-alkinyl, ($C_3$-$C_6$)-Cycloalkyl, Halogen-($C_3$-$C_6$)-cycloalkyl, ($C_3$-$C_6$)-Cycloalkyl-($C_1$-$C_6$)-alkyl, Halogen-($C_3$-$C_6$)-cycloalkyl-($C_1$-$C_6$)-alkyl, $COR^1$, $COOR^1$, $C(O)N(R^1)_2$, $C(O)NR^1OR^1$, $OSO_2R^2$, $S(O)_nR^2$, $SO_2OR^1$, $SO_2N(R^1)_2$, $NR^1SO_2R^2$, $NR^1COR^1$, ($C_1$-$C_6$)-Alkyl-$S(O)_nR^2$, ($C_1$-$C_6$)-Alkyl-$OR^1$, ($C_1$-$C_6$)-Alkyl-$OCOR^1$, ($C_1$-$C_6$)-Alkyl-$OSO_2R^2$, ($C_1$-$C_6$)-Alkyl-$CO_2R^1$, ($C_1$-$C_6$)-Alkyl-$SO_2OR^1$, ($C_1$-$C_6$)-Alkyl-$CON(R^1)_2$, ($C_1$-$C_6$)-Alkyl-$SO_2N(R^1)_2$, ($C_1$-$C_6$)-Alkyl-$NR^1COR^1$, ($C_1$-$C_6$)-Alkyl-$NR^1SO_2R^2$, 1,2,4-Triazol-1-yl, oder

Z kann auch Wasserstoff, ($C_1$-$C_6$)-Alkyl oder ($C_1$-$C_6$)-Alkoxy bedeuten, falls Y für den Rest $S(O)_nR^2$ steht,

W bedeutet Wasserstoff, ($C_1$-$C_6$)-Alkyl, Halogen-($C_1$-$C_6$)-alkyl, ($C_1$-$C_6$)-Alkoxy, ($C_1$-$C_6$)-Halogenalkoxy, $S(O)_n$-($C_1$-$C_6$)-alkyl, $S(O)_n$-($C_1$-$C_6$)-halogenalkyl, ($C_1$-$C_6$)-Alkoxy-($C_1$-$C_4$)-alkyl, Halogen, Nitro oder Cyano,

R bedeutet ($C_1$-$C_8$)-Alkyl, Halogen-($C_1$-$C_8$)-alkyl, ($C_2$-$C_8$)-Alkenyl, Halogen-($C_2$-$C_8$)-alkenyl, ($C_2$-$C_8$)-Alkinyl, Halogen-($C_2$-$C_8$)-alkinyl, wobei diese sechs vorstehend genannten Reste jeweils durch s Reste aus der Gruppe bestehend aus Nitro, Cyano, $SiR^5_3$, $P(OR^5)_3$, $S(O)_n$-($C_1$-$C_6$)-Alkyl, ($C_1$-$C_6$)-Alkoxy, Halogen-($C_1$-$C_6$)-alkoxy, $N(R^3)_2$, $COR^3$, $COOR^3$, $OCOR^3$, $NR^3COR^3$, $NR^3SO_2R^4$, ($C_3$-$C_6$)-Cycloalkyl, Heteroaryl, Heterocyclyl, Phenyl, Q-Heteroaryl, Q-Heterocyclyl, Q-Phenyl und Q-Benzyl substituiert sind, wobei die sieben letztgenannten Reste jeweils durch s Reste aus der Gruppe bestehend aus Methyl, Ethyl, Methoxy, Trifluormethyl, Cyano und Halogen substituiert sind, und wobei Heterocyclyl n Oxogruppen trägt, oder

R bedeutet jeweils durch s Reste aus der Gruppe bestehend aus Halogen, Nitro, Cyano, ($C_1$-$C_6$)-Alkyl, Halogen-($C_1$-$C_6$)-alkyl, ($C_3$-$C_6$)-Cycloalkyl, $S(O)_n$-($C_1$-$C_6$)-Alkyl, ($C_1$-$C_6$)-Alkoxy, Halogen-($C_1$-$C_6$)-alkoxy und ($C_1$-$C_6$)-Alkoxy-($C_1$-$C_4$)-alkyl substituiertes ($C_3$-$C_7$)-Cycloalkyl, Heteroaryl, Heterocyclyl oder Phenyl,

Q bedeutet O, S, oder $NR^3$,

$R^1$ bedeutet Wasserstoff, ($C_1$-$C_6$)-Alkyl, ($C_2$-$C_6$)-Alkenyl, ($C_2$-$C_6$)-Alkinyl, ($C_3$-$C_6$)-Cycloalkyl, ($C_3$-$C_6$)-Cycloalkyl-($C_1$-$C_6$)-alkyl, ($C_1$-$C_6$)-Alkyl-O-($C_1$-$C_6$)-alkyl, Phenyl, Phenyl-($C_1$-$C_6$)-alkyl, Heteroaryl, ($C_1$-$C_6$)-Alkyl-Heteroaryl, Heterocyclyl, ($C_1$-$C_6$)-Alkyl-Heterocyclyl, ($C_1$-$C_6$)-Alkyl-O-Heteroaryl, ($C_1$-$C_6$)-Alkyl-O-Heterocyclyl, ($C_1$-$C_6$)-Alkyl-$NR^3$-Heteroaryl oder ($C_1$-$C_6$)-Alkyl-$NR^3$-Heterocyclyl, wobei die sechzehn letztgenannten Reste jeweils durch s Reste aus der Gruppe bestehend aus Cyano, Halogen, Nitro, $OR^3$, $S(O)_nR^4$, $N(R^3)_2$, $NR^3OR^3$, $COR^3$, $OCOR^3$, $NR^3COR^3$, $NR^3SO_2R^4$, $CO_2R^3$, $CON(R^3)_2$ und ($C_1$-$C_4$)-Alkoxy-($C_2$-$C_6$)-alkoxycarbonyl substituiert sind, und wobei Heterocyclyl n Oxogruppen trägt,

$R^2$ bedeutet ($C_1$-$C_6$)-Alkyl, ($C_2$-$C_6$)-Alkenyl, ($C_2$-$C_6$)-Alkinyl, ($C_3$-$C_6$)-Cycloalkyl, ($C_3$-$C_6$)-Cycloalkyl-($C_1$-$C_6$)-alkyl, ($C_1$-$C_6$)-Alkyl-O-($C_1$-$C_6$)-alkyl, Phenyl, Phenyl-($C_1$-$C_6$)-alkyl, Heteroaryl, ($C_1$-$C_6$)-Alkyl-Heteroaryl, Heterocyclyl, ($C_1$-$C_6$)-Alkyl-Heterocyclyl, ($C_1$-$C_6$)-Alkyl-O-Heteroaryl, ($C_1$-$C_6$)-Alkyl-O-Heterocyclyl, ($C_1$-$C_6$)-Alkyl-$NR^3$-Heteroaryl oder ($C_1$-$C_6$)-Alkyl-$NR^3$-Heterocyclyl, wobei diese sechzehn letztgenannten Reste jeweils durch s Reste aus der

Gruppe bestehend aus Cyano, Halogen, Nitro, $OR^3$, $S(O)_nR^4$, $N(R^3)_2$, $NR^3OR^3$, $NR^3SO_2R^4$, $COR^3$, $OCOR^3$, $NR^3COR^3$, $CO_2R^3$, $CON(R^3)_2$ und $(C_1-C_4)$-Alkoxy-$(C_2-C_6)$-alkoxycarbonyl substituiert sind, und wobei Heterocyclyl n Oxogruppen trägt,

$R^3$ bedeutet Wasserstoff, $(C_1-C_6)$-Alkyl, $(C_2-C_6)$-Alkenyl, $(C_2-C_6)$-Alkinyl, $(C_3-C_6)$-Cycloalkyl oder $(C_3-C_6)$-Cycloalkyl-$(C_1-C_6)$-alkyl,

$R^4$ bedeutet $(C_1-C_6)$-Alkyl, $(C_2-C_6)$-Alkenyl oder $(C_2-C_6)$-Alkinyl,

$R^5$ bedeutet Methyl oder Ethyl,

$R^6$ und $R^7$ bedeuten unabhängig voneinander jeweils $(C_1-C_6)$-Alkyl, Halogen-$(C_1-C_6)$-alkyl, $(C_3-C_6)$-Cycloalkyl, Halogen-$(C_3-C_6)$-cycloalkyl, $(C_3-C_6)$-Cycloalkyl-$(C_1-C_6)$-alkyl, Halogen-$(C_3-C_6)$-CyCloalkyl-$(C_1-C_6)$-alkyl, $(C_1-C_6)$-Alkoxy-$(C_1-C_6)$-alkyl, Halogen-$(C_1-C_6)$-alkoxy-$(C_1-C_6)$-alkyl, Phenyl, Heteroaryl oder Heterocyclyl, wobei die drei letztgenannten Reste jeweils durch s Reste aus der Gruppe bestehend aus Nitro, Halogen, $(C_1-C_6)$-Alkyl, Halogen-$(C_1-C_6)$-alkyl, $R^1O(O)C$, $(R^1)_2N(O)C$, $R^1O$, $(R^1)_2N$, $R^2(O)_nS$, und $R^1O$-$(C_1-C_6)$-Alkyl substituiert sind, und wobei Heterocyclyl n Oxogruppen trägt,
oder $R^6$ und $R^7$ bilden gemeinsam mit dem Schwefel-Atom, an dem sie gebunden sind, einen 3- bis 8-gliedrigen ungesättigten, teilgesättigten oder gesättigten Ring, der außer den Kohlenstoffatomen und außer dem Schwefelatom der Sulfoximinogruppe jeweils m Ringglieder aus der Gruppe bestehend aus $N(R^1)$, O und $S(O)_n$ enthält, und wobei dieser Ring jeweils durch s Reste aus der Gruppe bestehend aus Halogen, $(C_1-C_6)$-Alkyl, Halogen-$(C_1-C_6)$-alkyl, $R^1O(O)C$, $(R^1)_2N(O)C$, $R^1O$, $(R^1)_2N$, $R^2(O)_nS$, $R^1O(O)_2S$, $(R^1)_2N(O)_2S$ und $R^1O$-$(C_1-C_6)$-Alkyl, substituiert ist, und wobei dieser Ring n Oxogruppen trägt,

$R^8$ bedeutet jeweils durch s Reste aus der Gruppe bestehend aus Halogen, Cyano, $(C_3-C_6)$-Cycloalkyl, $R^1(O)C$, $R^1(R^1ON{=})C$, $R^1O(O)C$, $(R^1)_2N(O)C$, $R^2(O)_2S(R^1)N(O)C$, $R^1O$, $(R^1)_2N$, $R^1(O)C(R^1)N$, $R^2(O)_2S(R^1)N$, $R^2O(O)C(R^1)N$, $(R^1)_2N(O)C(R^1)N$, $R^2(O)_nS$, $R^1O(O)_2S$, $(R^1)_2N(O)_2S$, $R^1(O)C(R^1)N(O)_2S$, $R^2O(O)C(R^1)N(O)_2S$ und $(R^1)_2N(O)C(R^1)N(O)_2S$ substituiertes $(C_1-C_6)$-Alkyl oder
jeweils durch s Reste aus der Gruppe bestehend aus Halogen, $(C_1-C_6)$-Alkyl, Halogen-$(C_1-C_6)$-alkyl, $(C_3-C_6)$-Cycloalkyl, $R^1O(O)C$ und $(R^1)_2N(O)C$ substituiertes $(C_3-C_6)$-Cycloalkyl,

$R^9$ bedeutet Wasserstoff, Nitro, Cyano, $(C_1-C_6)$-Alkyl, Halogen-$(C_1-C_6)$-alkyl, $(C_3-C_6)$-Cycloalkyl, Halogen-$(C_3-C_6)$-cycloalkyl, $(C_3-C_6)$-Cycloalkyl-$(C_1-C_6)$-alkyl, Halogen-$(C_3-C_6)$-cycloalkyl-$(C_1-C_6)$-alkyl, $R^1(O)C$, $R^2O(O)C$, $(R^1)_2N(O)C$, $R^2(O)_2S$, $R^1(O)C$-$(C_1-C_6)$-Alkyl, $R^1O(O)C$-$(C_1-C_6)$-Alkyl, $(R^1)_2N(O)C$-$(C_1-C_6)$-Alkyl, $R^1O$-$(C_1-C_6)$-Alkyl, $(R^1)_2N$-$(C_1-C_6)$-Alkyl oder $R^2(O)_nS$-$(C_1-C_6)$-Alkyl,

m bedeutet 0, 1 oder 2,

n bedeutet 0, 1 oder 2,

s bedeutet 0, 1, 2 oder 3.

Besonders bevorzugt sind Verbindungen der allgemeinen Formel (I), worin

A bedeutet N oder CY,

B bedeutet N oder CH,

X bedeutet Nitro, Halogen, Cyano, $(C_1-C_6)$-Alkyl, Halogen-$(C_1-C_6)$-alkyl, $(C_3-C_6)$-Cycloalkyl, $OR^1$, $S(O)_nR^2$, $(C_1-C_6)$-Alkyl-$S(O)_nR^2$, $(C_1-C_6)$-Alkyl-$OR^1$, $(C_1-C_6)$-Alkyl-$CON(R^1)_2$, $(C_1-C_6)$-Alkyl-$SO_2N(R^1)_2$, $(C_1-C_6)$-Alkyl-$NR^1COR^1$, $(C_1-C_6)$-Alkyl-$NR^1SO_2R^2$, $(C_1-C_6)$-Alkyl-Heteroaryl oder $(C_1-C_6)$-Alkyl-Heterocyclyl, wobei die beiden letztgenannten Reste jeweils durch s Reste Halogen, $(C_1-C_6)$-Alkyl, Halogen-$(C_1-C_6)$-alkyl, $S(O)_n$-$(C_1-C_6)$-Alkyl, $(C_1-C_6)$-Alkoxy und Halogen-$(C_1-C_6)$-alkoxy substituiert sind, und wobei Heterocyclyl n Oxogruppen trägt,

Y Wasserstoff, Nitro, Halogen, Cyano, $(C_1-C_6)$-Alkyl, $(C_1-C_6)$-Halogenalkyl, $OR^1$, $S(O)_nR^2$, $SO_2N(R^1)_2$, $NS(O)R^6R^7$, $S(O)R^8NR^9$, $N(R^1)_2$, $CHNOR^1$, $CH_2ONC(R^3)_2$, $NR^1SO_2R^2$, $NR^1COR^1$, $(C_1-C_6)$-Alkyl-$S(O)_nR^2$, $(C_1-C_6)$-Alkyl-$OR^1$, $(C_1-C_6)$-Alkyl-$CON(R^1)_2$, $(C_1-C_6)$-Alkyl-$SO_2N(R^1)_2$, $(C_1-C_6)$-Alkyl-$NR^1COR^1$, $(C_1-C_6)$-Alkyl-$NR^1SO_2R^2$, $(C_1-C_6)$-Alkyl-Phenyl, $(C_1-C_6)$-Alkyl-Heteroaryl, $(C_1-C_6)$-Alkyl-Heterocyclyl, Phenyl, Heteroaryl oder Heterocyclyl, wobei die

sechs letztgenannten Reste jeweils durch s Reste aus der Gruppe bestehend Halogen, Nitro, Cyano, $(C_1-C_6)$-Alkyl, Halogen-$(C_1-C_6)$-alkyl, $(C_3-C_6)$-Cycloalkyl, $S(O)_n-(C_1-C_6)$-Alkyl, $(C_1-C_6)$-Alkoxy, Halogen-$(C_1-C_6)$-alkoxy, $(C_1-C_6)$-Alkoxy-$(C_1-C_4)$-alkyl und Cyanomethyl substituiert sind, und wobei Heterocyclyl n Oxogruppen trägt,

Z bedeutet Halogen, Cyano, Halogen-$(C_1-C_6)$-alkyl, $(C_3-C_6)$-Cycloalkyl, $S(O)_nR^2$, 1,2,4-Triazol-1-yl, oder

Z kann auch Wasserstoff, Methyl, Methoxy oder Ethoxy bedeuten, falls Y für den Rest $S(O)_nR^2$ steht,

W bedeutet Wasserstoff, Methyl, Ethyl, Methoxymethyl, Methoxy, Fluor, Chlor oder $S(O)_nCH_3$,

R bedeutet $(C_1-C_8)$-Alkyl, Halogen-$(C_1-C_8)$-alkyl, $(C_2-C_8)$-Alkenyl, Halogen-$(C_2-C_8)$-alkenyl, $(C_2-C_8)$-Alkinyl, Halogen-$(C_2-C_8)$-alkinyl, wobei diese sechs vorstehend genannten Reste jeweils durch s Reste aus der Gruppe bestehend aus Cyano, $S(O)_n-(C_1-C_6)$-Alkyl, $(C_1-C_6)$-Alkoxy, Halogen-$(C_1-C_6)$-alkoxy, $COR^3$, $COOR^3$, $OCOR^3$, $NR^3COR^3$, $NR^3SO_2R^4$, $(C_3-C_6)$-Cycloalkyl, Heteroaryl, Heterocyclyl und Phenyl substituiert sind, wobei die drei letztgenannten Reste jeweils durch s Reste aus der Gruppe bestehend aus Methyl, Ethyl, Methoxy, Trifluormethyl, Cyano und Halogen substituiert sind, und wobei Heterocyclyl 0 bis 2 Oxogruppen trägt, oder

R bedeutet durch s Reste aus der Gruppe bestehend aus Halogen, Nitro, Cyano, $(C_1-C_6)$-Alkyl, Halogen-$(C_1-C_6)$-alkyl, $(C_3-C_6)$-Cycloalkyl, $S(O)_n-(C_1-C_6)$-Alkyl, $(C_1-C_6)$-Alkoxy, Halogen-$(C_1-C_6)$-alkoxy, $(C_1-C_6)$-Alkoxy-$(C_1-C_4)$-alkyl substituiertes Phenyl,

$R^1$ bedeutet Wasserstoff, $(C_1-C_6)$-Alkyl, $(C_2-C_6)$-Alkenyl, $(C_2-C_6)$-Alkinyl, $(C_3-C_6)$-Cycloalkyl, $(C_3-C_6)$-Cycloalkyl-$(C_1-C_6)$-alkyl, $(C_1-C_6)$-Alkyl-O-$(C_1-C_6)$-alkyl, Phenyl, Phenyl-$(C_1-C_6)$-alkyl, Heteroaryl, $(C_1-C_6)$-Alkyl-Heteroaryl, Heterocyclyl, $(C_1-C_6)$-Alkyl-Heterocyclyl, $(C_1-C_6)$-Alkyl-O-Heteroaryl, $(C_1-C_6)$-Alkyl-O-Heterocyclyl, $(C_1-C_6)$-Alkyl-$NR^3$-Heteroaryl oder $(C_1-C_6)$-Alkyl-$NR^3$-Heterocyclyl, wobei die sechzehn letztgenannten Reste jeweils durch s Reste aus der Gruppe bestehend aus Cyano, Halogen, Nitro, $OR^3$, $S(O)_nR^4$, $N(R^3)_2$, $NR^3OR^3$, $COR^3$, $OCOR^3$, $NR^3COR^3$, $NR^3SO_2R^4$, $CO_2R^3$, $CON(R^3)_2$ und $(C_1-C_4)$-Alkoxy-$(C_2-C_6)$-alkoxycarbonyl substituiert sind, und wobei Heterocyclyl n Oxogruppen trägt,

$R^2$ bedeutet jeweils durch s Reste aus der Gruppe bestehend aus Halogen und $OR^3$ substituiertes $(C_1-C_6)$-Alkyl, $(C_3-C_6)$-Cycloalkyl oder $(C_3-C_6)$-Cycloalkyl-$(C_1-C_6)$-alkyl,

$R^3$ bedeutet Wasserstoff oder $(C_1-C_6)$-Alkyl,

$R^4$ bedeutet $(C_1-C_6)$-Alkyl,

$R^6$ und $R^7$ bedeuten unabhängig voneinander jeweils Methyl, Ethyl oder n-Propyl, oder

$R^6$ und $R^7$ bilden gemeinsam mit dem Schwefel-Atom, an dem sie gebunden sind, einen 5- oder 6-gliedrigen gesättigten Ring, der außer den Kohlenstoffatomen und außer dem Schwefelatom der Sulfoximinogruppe m Sauerstoffatome enthält,

$R^8$ bedeutet Methyl, Ethyl oder n-Propyl,

$R^9$ bedeutet Wasserstoff oder Cyano,

m bedeutet 0 oder 1,

n bedeutet 0, 1 oder 2,

s bedeutet 0, 1, 2 oder 3.

In allen nachfolgend genannten Formeln haben die Substituenten und Symbole, sofern nicht anders definiert, dieselbe Bedeutung wie unter Formel (I) beschrieben.

Erfindungsgemäße Verbindungen können beispielsweise nach der in Schema 1 angegebenen Methode durch Umsetzung eines N-(Tetrazol-5-yl)- und N-(Triazol-5-yl)benzamides und Nicotinamides (II) mit einem schwefelübertragendem Reagenz, wie z.B. Phosphorpentasulfid oder Lawesson's Reagenz hergestellt werden:

Schema 1

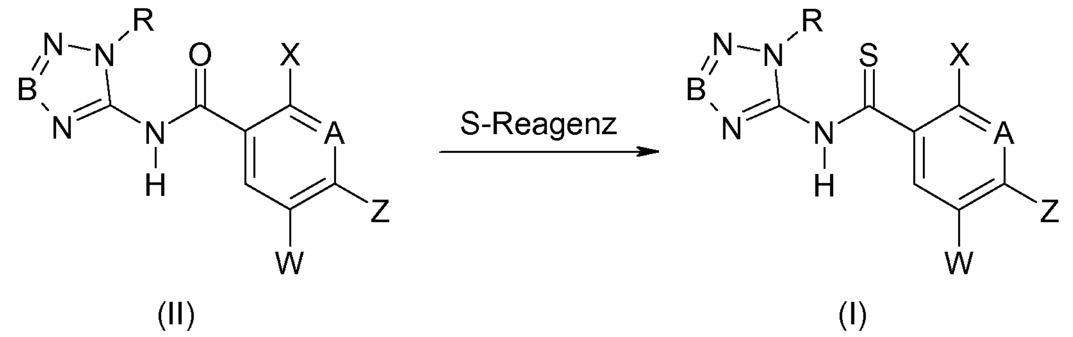


Die N-(Tetrazol-5-yl)- und N-(Triazol-5-yl)benzamide und Nicotinamide der Formel (II) können beispielsweise nach den in WO 2012/028579 A1, EP 11158253 und EP 11159115 beschriebenen Methoden hergestellt werden.

Es kann zweckmäßig sein, Reaktionsschritte in ihrer Reihenfolgezu ändern.So sind Benzoesäuren, die ein Sulfoxid tragen, nicht ohne weiteres in ihre Säurechloride zu überführen. Hier bietet sich an, zunächst auf Thioether-Stufe das Amid zu herzustellen und danach den Thioether zum Sulfoxid zu oxidieren.

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, Iowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasenunterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Die erfindungsgemäßen Verbindungen der Formel (I) (und/oder deren Salze), im folgenden zusammen als "erfindungsgemäße Verbindungen" bezeichnet, weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder

Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen in wirtschaftlich bedeutenden transgenen Kulturen von Nutz- und Zierpflanzen, z. B. von Getreide wie Weizen, Gerste, Roggen, Hafer, Hirse, Reis und Mais oder auch Kulturen von Zuckerrübe, Baumwolle, Soja, Raps, Kartoffel, Tomate, Erbse und anderen Gemüsesorten.Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Bevorzugt ist die Anwendung der erfindungsgemäßen Verbindungen oder deren Salze in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten. Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen

- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91 /13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt, siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY, oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet. Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber

nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227, Wolter et al., Proc. Natl. Acad. Sci. USA 85 (1988), 846-850, Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydroxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973, K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y., C. Marsden, "Solvents Guide", 2nd Ed., Interscience, N.Y. 1963, McCutcheon's "Detergents and Emulsifiers Annual", MC Publ. Corp., Ridgewood N.J., Sisley and Wood, "Encyclopedia of Surface Active Agents", Chem. Publ. Co. Inc., N.Y. 1964, Schönfeldt, "Grenzflächenaktive Äthylenoxidaddukte", Wiss. Verlagsgesell., Stuttgart 1976, Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix. Geeignete Safener sind beispielsweise Mefenpyr-diethyl, Cyprosulfamid, Isoxadifen-ethyl, Cloquintocet-mexyl und Dichlormid.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der

Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London, J.E. Browning, "Agglomeration", Chemical and Engineering 1967, Seiten 147 ff, "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung.

A. Chemische Beispiele

Synthese von 2-Methyl-3-methylsulfonyl-4-trifluormetyl-N-(1-propyltetrazol-5-yl)-thiobenzamid (Nr. 1-9)

196 mg (0.5 mmol) 2-Methyl-3-methylsulfonyl-4-trifluormetyl-N-(1-propyltetrazol-5-yl)-benzamid und 404 mg (1 mmol) Lawesson's Reagenz werden in 5 ml Dioxan 5 h unter Rückfluss erhitzt. Nach beendeter Reaktion wird mit 1ml Wasser versetzt, die Mischung eingeengt und der Rückstand über HPLC chromatographisch gereinigt, wobei 158 mg 2-Methyl-3-methylsulfonyl-4-trifluormetyl-N-(1-propyltetrazol-5-yl)-thiobenzamid gewonnen werden (Ausbeute: 78%).

Die in den nachfolgenden Tabellen aufgeführten Beispiele wurden analog oben genannten Methoden hergestellt beziehungsweise sind analog oben genannten Methoden erhältlich. Die in den nachfolgenden Tabellen aufgeführten Verbindungen sind ganz besonders bevorzugt.

Die verwendeten Abkürzungen bedeuten:

Et = Ethyl     Me = Methyl     n-Pr = n-Propyl     c-Pr = c-Propyl

Tabelle 1: Verbindungen der allgemeinen Formel (I), worin A für CY und B für N und W für Wasserstoff steht

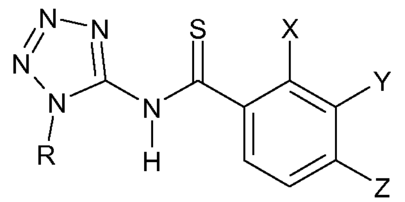

| Bsp.-Nr. | R | X | Y | Z | Physikalische Daten ($^1$H-NMR, DMSO-$d_6$,400 MHz) |
|---|---|---|---|---|---|
| 1-1 | Me | Cl | H | $SO_2Me$ | |
| 1-2 | Me | $SO_2Me$ | H | $CF_3$ | 8.26 (s,1H), 8.23 (d,1H), 7.89 (d,1H), 4.08 (s,3H), 3.48 (s,3H) |
| 1-3 | Me | Me | SMe | $CF_3$ | |
| 1-4 | $MeOC_2H_4$ | Me | SMe | $CF_3$ | |
| 1-5 | Me | Me | SOMe | $CF_3$ | |
| 1-6 | Et | Me | SOMe | $CF_3$ | |
| 1-7 | Me | Me | $SO_2Me$ | $CF_3$ | 8.00 (d,1H), 7.94 (d,1H), 4.05 (s,3H), 3.44 (s,3H), 2.78 (s,3H) |
| 1-8 | Et | Me | $SO_2Me$ | $CF_3$ | 7.99 (d,1 H), 7.92 (d,1 H), 4.36 (q,2H), 3.43 (s,3H), 2.79 (s,3H), 1.49 (t,3H) |
| 1-9 | Pr | Me | $SO_2Me$ | $CF_3$ | 7.99 (d,1 H), 7.90 (d,1 H), 4.29 (t,2H), 3.43 (s,3H), 2.79 (s,3H), 1.90 (m,2H), 0.89 (t,3H) |
| 1-10 | $MeOC_2H_4$ | Me | $SO_2Me$ | $CF_3$ | |
| 1-11 | Me | Me | SEt | $CF_3$ | |
| 1-12 | Et | Me | SEt | $CF_3$ | |
| 1-13 | Me | Me | SOEt | $CF_3$ | |
| 1-14 | Et | Me | SOEt | $CF_3$ | |
| 1-15 | Me | Me | $SO_2Et$ | $CF_3$ | |
| 1-16 | Et | Me | $SO_2Et$ | $CF_3$ | |

(fortgesetzt)

| Bsp.-Nr. | R | X | Y | Z | Physikalische Daten ($^1$H-NMR, DMSO-$d_6$,400 MHz) |
|---|---|---|---|---|---|
| 1-17 | Me | Me | $SO_2Me$ | Cl | |
| 1-18 | Me | Me | SEt | Cl | |
| 1-19 | Me | Me | SOEt | Cl | |
| 1-20 | Et | Me | SOEt | Cl | |
| 1-21 | Me | Me | $SO_2Et$ | Cl | |
| 1-22 | Me | Me | SMe | Br | |
| 1-23 | Me | Me | SEt | Br | |
| 1-24 | Me | Me | 4,5-Dihydro-1,2-oxazol-3-yl | $SO_2Me$ | |
| 1-25 | Et | Me | 4,5-Dihydro-1,2-oxazol-3-yl | $SO_2Me$ | |
| 1-26 | Me | Me | Pyrazol-1-yl | $SO_2Me$ | 8.07 (d,1H), 7.78 (d,1H), 7.91 (d,1H), 7.86 (d,1H), 6.60 (t,1 H), 4.04 (s,3H), 3.03 (s,3H), 1.92 (s,3H) |
| 1-27 | Et | Me | Pyrazol-1-yl | $SO_2Me$ | |
| 1-28 | Et | Me | F | $SO_2Me$ | 13.0 (brs,1H), 7.80 (t,1H), 7.56 (d,1H), 4.35 (q,3H), 3.38 (s,3H), 2.39 (s,3H), 1.50 (t,3H) |
| 1-29 | Me | Me | Cl | $SO_2Me$ | 12.8 (brs,1H), 8.01 (d,1H), 7.70 (d,1H), 4.05 (s,3H), 3.43 (s,3H), 2.49 (s,3H) |
| 1-30 | Me | Me | OMe | $SO_2Me$ | 7.79 (d,1H), 7.36 (d,1H), 4.10 (s,3H), 3.95 (s,3H), 3.23 (s,3H), 2.47 (s,3H) |
| 1-31 | Me | Me | SMe | $SO_2Me$ | |
| 1-32 | Me | Me | $SO_2Me$ | $SO_2Me$ | |
| 1-33 | Et | Me | $SO_2Me$ | $SO_2Me$ | |
| 1-34 | Me | Me | $SO_2Et$ | $SO_2Me$ | |
| 1-35 | Et | Me | $SO_2Et$ | $SO_2Me$ | |
| 1-36 | Me | Et | SMe | $CF_3$ | |
| 1-37 | Me | Et | SOMe | $CF_3$ | |
| 1-38 | Me | Et | $SO_2Me$ | $CF_3$ | |
| 1-39 | Me | Et | SMe | Cl | |
| 1-40 | Et | Et | SMe | Cl | |
| 1-41 | Me | Et | SOMe | Cl | |
| 1-42 | Me | Et | SMe | Br | |
| 1-43 | Me | Et | $SO_2Me$ | Br | |
| 1-44 | Me | Pr | SMe | $CF_3$ | |
| 1-45 | Me | Pr | SOMe | $CF_3$ | |
| 1-46 | Me | c-Pr | SMe | $CF_3$ | |
| 1-47 | Me | OMe | SMe | $CHF_2$ | |

(fortgesetzt)

| Bsp.-Nr. | R | X | Y | Z | Physikalische Daten ($^1$H-NMR, DMSO-$d_6$,400 MHz) |
|---|---|---|---|---|---|
| 1-48 | Et | OMe | SMe | $CHF_2$ | |
| 1-49 | Me | OMe | SOMe | $CHF_2$ | |
| 1-50 | Et | OMe | SOMe | $CHF_2$ | |
| 1-51 | Me | OMe | $SO_2Me$ | $CHF_2$ | |
| 1-52 | Et | OMe | $SO_2Me$ | $CHF_2$ | |
| 1-53 | Me | OMe | SEt | $CHF_2$ | |
| 1-54 | Me | OMe | SMe | $CF_3$ | 12.95 (brs,1H), 7.73 (d,1H), 7.66 (d,1H), 4.04 (s,3H), 3.98 (s,3H), 2.48 (s,3H) |
| 1-55 | Et | OMe | SMe | $CF_3$ | |
| 1-56 | Me | OMe | SOMe | $CF_3$ | |
| 1-57 | Et | OMe | SOMe | $CF_3$ | |
| 1-58 | Me | OMe | $SO_2Me$ | $CF_3$ | |
| 1-59 | Et | OMe | $SO_2Me$ | $CF_3$ | |
| 1-60 | Me | OMe | SEt | $CF_3$ | |
| 1-61 | Me | Cl | SMe | H | |
| 1-62 | Me | Cl | SMe | Me | |
| 1-63 | Me | Cl | $SO_2Me$ | Me | 7.65 (d,1H), 7.34 (d,1H), 4.10 (s,3H), 3.32 (s,3H), 2.79 (s,3H) |
| 1-64 | Me | Cl | $SO_2Et$ | Me | |
| 1-65 | Me | Cl | $SO_2Me$ | $CF_3$ | |
| 1-66 | Me | Cl | $OC_2H_4OMe$ | Cl | |
| 1-67 | Me | Cl | SMe | Cl | |
| 1-68 | Et | Cl | SMe | Cl | |
| 1-69 | Me | Cl | SOMe | Cl | |
| 1-70 | Et | Cl | SOMe | Cl | |
| 1-71 | Me | Cl | $SO_2Me$ | Cl | |
| 1-72 | Et | Cl | $SO_2Me$ | Cl | |
| 1-73 | Me | Cl | $SO_2Et$ | Cl | |
| 1-74 | Me | Cl | $CH_2OMe$ | $SO_2Me$ | 8.05 (d,1H), 7.65 (d,1H), 5.07 (s,2H), 4.09 (s,3H), 3.51 (s,3H), 3.24 (s,3H) |
| 1-75 | Me | Cl | $CH_2OCH_2CF_3$ | $SO_2Me$ | 13.2 (brs,1H), 8.09 (d,1H), 7.91 (d,1H), 5.26 (s,2H), 4.31 (q,2H), 4.05 (s,3H), 3.37 (s,3H) |
| 1-76 | Et | Cl | $CH_2OCH_2CF_3$ | $SO_2Me$ | |
| 1-77 | Me | Cl | $CH_2OC_2H_4OMe$ | $SO_2Me$ | |
| 1-78 | Me | Cl | 4,5-Dihydro-1,2-oxazol-3-yl | $SO_2Me$ | |
| 1-79 | Me | Cl | 4,5-Dihydro-1,2-oxazol-3-yl | $SO_2Et$ | |

(fortgesetzt)

| Bsp.-Nr. | R | X | Y | Z | Physikalische Daten ($^1$H-NMR, DMSO-$d_6$,400 MHz) |
|---|---|---|---|---|---|
| 1-80 | Me | Cl | 5-Methoxymethy-4,5-dihydro-1,2-oxazol-3-yl | $SO_2Et$ | |
| 1-81 | Me | Cl | OMe | $SO_2Me$ | 7.93 (d,1H), 7.48 (d,1H), 4.11 (s,3H), 4.10 (s,3H), 3.26 (s, 3H) |
| 1-82 | Me | Cl | OMe | $SO_2Et$ | |
| 1-83 | Me | Cl | OEt | $SO_2Me$ | 7.94 (d,1H), 7.46 (d,1H), 4.34 (q,2H), 4.10 (s,3H), 3.28 (s,3H), 1.53 (t,3H) |
| 1-84 | Me | Cl | OEt | $SO_2Et$ | |
| 1-85 | Me | Cl | OPr | $SO_2Me$ | |
| 1-86 | Me | Cl | OPr | $SO_2Et$ | |
| 1-87 | Me | Cl | Oi-Bu | $SO_2Me$ | |
| 1-88 | Me | Cl | $OCH_2$c-Pr | $SO_2Me$ | 7.93 (d,1H), 7.46 (d,1H), 4.11 (d,2H), 4.09 (s,3H), 3.32 (s,3H), 1.40-1.55 (m,1H), 0.64-0.71 (m,2H), 0.43-0.50 (m,2H) |
| 1-89 | Me | Cl | $OCH_2$c-Pr | $SO_2Et$ | |
| 1-90 | Me | Cl | $OC_2H_4OMe$ | $SO_2Me$ | |
| 1-91 | Me | Cl | $OC_3H_6OMe$ | $SO_2Me$ | 7.92 (d,1H), 7.47 (d,1H), 4.35 (t,2H), 4.10 (s,3H), 3.62 (t,2H), 3.37 (s,3H), 3.26 (s,3H), 2.18 (quin,2H) |
| 1-92 | Me | Cl | SMe | $SO_2Me$ | |

Tabelle 2: Verbindungen der allgemeinen Formel (I), worin A für CY und B für CH und W für Wasserstoff steht

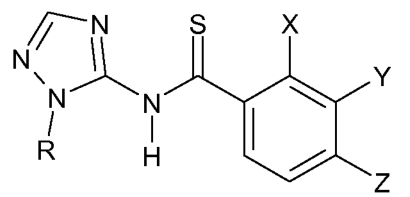

| Bsp.-Nr. | R | X | Y | Z | Physikalische Daten ($^1$H-NMR, DMSO-$d_6$,400 MHz) |
|---|---|---|---|---|---|
| 2-1 | Me | Me | $SO_2Me$ | $CF_3$ | |
| 2-2 | Me | Me | 4,5-Dihydro-1,2-oxazol-3-yl | $SO_2Me$ | |
| 2-3 | Me | Me | Pyrazol-1-yl | $SO_2Me$ | |
| 2-4 | Me | Me | $SO_2Me$ | $SO_2Me$ | 8.20 (brs,1H, 8.19 (d,1H), 7.88 (brs,1H), 3.79 (s,3H), 3.58 (s,3H), 3.56 (s,3H), 2.71 (s,3H) |
| 2-5 | Me | Cl | $SO_2Me$ | Cl | |
| 2-6 | Me | Cl | 4,5-Dihydro-1,2-oxazol-3-yl | $SO_2Me$ | |

(fortgesetzt)

| Bsp.-Nr. | R | X | Y | Z | Physikalische Daten ($^1$H-NMR, DMSO-$d_6$,400 MHz) |
|---|---|---|---|---|---|
| 2-7 | Me | Cl | 4,5-Dihydro-1,2-oxazol-3-yl | $SO_2Et$ | |
| 2-8 | Me | Cl | $OC_2HaOMe$ | $SO_2Me$ | |

Tabelle 3: Verbindungen der allgemeinen Formel (I), worin A für CY und B für N steht

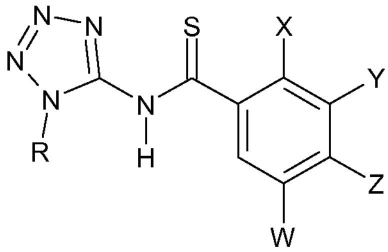

| Bsp.-Nr. | R | X | Y | Z | W | Physikalische Daten ($^1$H-NMR, DMSO-$d_6$,400 MHz) |
|---|---|---|---|---|---|---|
| 3-1 | Me | Cl | H | SMe | Me | 12.85 (brs,1H), 7.47 (s,1H), 7.27 (s,1H), 4.02 (s,3H), 2.56 (s,3H), 2.26 (s,3H) |
| 3-2 | Me | Cl | SMe | H | Me | |
| 3-3 | Me | Cl | $SO_2Me$ | H | Me | |
| 3-4 | Et | Cl | $SO_2Me$ | H | Me | |
| 3-5 | Me | Cl | Me | SMe | Me | |
| 3-6 | Et | Cl | Me | $SO_2Me$ | Me | |
| 3-7 | Me | Br | $SO_2Me$ | H | Me | |
| 3-8 | Me | Cl | OMe | SMe | OMe | 13.0 (brs,1H), 7.08 (s,1H), 4.05 (s,3H), 3.91 (s,3H), 3.84 (s,3H), 2.40 (s,3H) |

Tabelle 4: Verbindungen der allgemeinen Formel (I), worin A und B jeweils N bedeuten und W für Wasserstoff steht

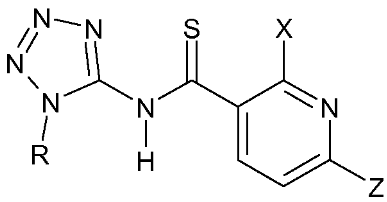

| Bsp.-Nr. | R | X | Z | Physikalische Daten ($^1$H-NMR, DMSO-$d_6$,400 MHz) |
|---|---|---|---|---|
| 4-1 | Me | Me | $CF_3$ | 13.1 (brs,1H), 8.17 (d,1H), 7.88 (d,1H), 4.06 (s,3H), 2.68 (s,3H) |
| 4-2 | Me | $CH_2OMe$ | $CF_3$ | 13.1 (brs,1H), 8.25 (d,1H), 8.02 (d,1H), 4.79 (s,2H), 4.05 (s,3H), 3.17 (s,3H) |
| 4-3 | Et | $CH_2OMe$ | $CF_3$ | |
| 4-4 | Me | $CH_2OC_2H_4OMe$ | $CF_3$ | |

(fortgesetzt)

| Bsp.-Nr. | R | X | Z | Physikalische Daten ($^1$H-NMR, DMSO-$d_6$,400 MHz) |
|---|---|---|---|---|
| 4-5 | Et | $CH_2OC_2H_4OMe$ | $CF_3$ | |
| 4-6 | Me | $CH_2OCH_2$c-Pr | $CF_3$ | |
| 4-7 | Me | Cl | $CF_3$ | 8.38 (d,1H), 8.09 (d,1H), 4.03 (s,3H) |
| 4-8 | Me | Br | $CF_3$ | |
| 4-9 | Me | $SO_2Me$ | $CF_3$ | |
| 4-10 | Me | SMe | SMe | 7.73 (d,1H), 7.12 (d,1H), 4.02 (s,3H), 2.60 (s,3H), 2.54 (s,3H) |

B. Formulierungsbeispiele

a) Ein Stäubemittel wird erhalten, indem man 10 Gew.-Teile einer Verbindung der Formel (I) und/oder deren Salze und 90 Gew.-Teile Talkum als Inertstoff mischt und in einer Schlagmühle zerkleinert.

b) Ein in Wasser leicht dispergierbares, benetzbares Pulver wird erhalten, indem man 25 Gewichtsteile einer Verbindung der Formel (I) und/oder deren Salze, 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat wird erhalten, indem man 20 Gew.-Teile einer Verbindung der Formel (I) und/oder deren Salze mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.

d) Ein emulgierbares Konzentrat wird erhalten aus 15 Gew.-Teilen einer Verbindung der Formel (I) und/oder deren Salze, 75 Gew.-Teilen Cyclohexanon als Lösungsmittel und 10 Gew.-Teilen oxethyliertes Nonylphenol als Emulgator.

e) Ein in Wasser dispergierbares Granulat wird erhalten indem man

75 Gew.-Teile einer Verbindung der Formel (I) und/oder deren Salze,
10 Gew.-Teile ligninsulfonsaures Calcium,
5 Gew.-Teile Natriumlaurylsulfat,
3 Gew.-Teile Polyvinylalkohol und
7 Gew.-Teile Kaolin

mischt, auf einer Stiftmühle mahlt und das Pulver in einem Wirbelbett durch Aufsprühen von Wasser als Granulierflüssigkeit granuliert.

f) Ein in Wasser dispergierbares Granulat wird auch erhalten, indem man

25 Gew.-Teile einer Verbindung der Formel (I) und/oder deren Salze,
5 Gew.-Teile 2,2'-dinaphthylmethan-6,6'-disulfonsaures Natrium
2 Gew.-Teile oleoylmethyltaurinsaures Natrium,
1 Gew.-Teil Polyvinylalkohol,
17 Gew.-Teile Calciumcarbonat und
50 Gew.-Teile Wasser

auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

C. Biologische Beispiele

1. Herbizide Wirkung gegen Schadpflanzen im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die Oberfläche der Abdeckerde appliziert. Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen). Dabei zeigen beispielsweise die Verbindungen Nr. 1-8, 1-26 und 4-7 bei einer Aufwandmenge von 80 g/ha eine mindestens 80%-ige Wirkung gegen Alopecurus myosuroides, Avena fatua, Cyperus serotinus, Echinochloa crus galli, Lolium multiflorum, Setaria viridis, Abutilon theophrasti, Amaranthus retroflexus, Matricaria inodora, Pharbitis purpureum, Stellaria media, Viola tricolorund Veronica persica.

2. Herbizide Wirkung gegen Schadpflanzen im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen). Dabei zeigen beispielsweise die Verbindungen Nr. 1-8 und 1-83 bei einer Aufwandmenge von 80 g/ha eine mindestens 80%-ige Wirkung gegen Alopecurus myosuroides, Avena fatua, Cyperus serotinus, Echinochloa crus galli, Lolium multiflorum, Setaria viridis, Abutilon theophrasti, Amaranthus retroflexus, Matricaria inodora, Pharbitis purpureum, Polygonum convolvulus, Stellaria media, Viola tricolorund Veronica persica.

3. Vergleichsversuche

Zur Demonstration der Überlegenheit der erfindungsgemäßen Verbindungen wurden zahlreiche erfindungsgemäße mit den strukturell ähnlichsten und aus WO 2012/028579 A1 bekannten Verbindungen bei unterschiedlichen Aufwandmengen, Schadpflanzen und Kulturpflanzen beispielhaft verglichen. Diese Vergleichsversuche wurden unter den oben genannten Bedingungen im Vorauflauf und Nachauflauf durchgeführt. In den nachfolgenden Tabellen ist jeweils in der oberen Zeile die erfindungsgemäße und in der unteren Zeile die aus WO 2012/028579 A1 Verbindung genannt.

Die hier verwendeten Abkürzungen bedeuten:

| Schadpflanzen | | | |
|---|---|---|---|
| ABUTH | Abutilon theophrasti | ALOMY | Alopecurus myosuroides |
| AMARE | Amaranthus retroflexus | AVEFA | Avena fatua |
| CYPES | Cyperus serotinus | ECHCG | Echinochloa crus galli |
| LOLMU | Lolium multiflorum | PHBPU | Pharbitis purpureum |
| POLCO | Polygonum convolvulus | SETVI | Setaria viridis |

| *Kulturpflanzen* | | | |
|---|---|---|---|
| *BRSNW* | *Brassica napus (Raps)* | *ORYSA* | *Oryza sativa* (*Reis*) |
| *TRZAS* | *Triticum aestivum* (*Weizen*) | *ZEAMX* | *Zea mays* (*Mais*) |

Tabelle A: Wirkung im Vorauflauf

| Verbindung | Dosierung [g/ha] | *Schädigung von Kulturpflanzen* | Herbizide Wirkung gegen | |
|---|---|---|---|---|
| | | *BRSNW* | ABUTH | PHBPU |
| 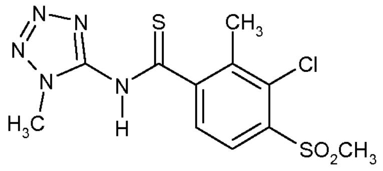 Nr. 1-29 | 20 | 0% | 90% | 20% |
| 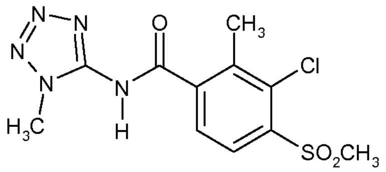 Nr. 4-173 | 20 | *20%* | 70% | 0% |

Tabelle B: Wirkung im Vorauflauf

| Verbindung | Dosierung [g/ha] | *Schädigung von Kulturpflanzen* | Herbizide Wirkung gegen | |
|---|---|---|---|---|
| | | *OR YSA* | CYPES | ECHCG |
| 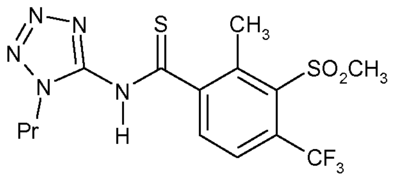 Nr. 1-9 | 20 | *0%* | 100% | 20% |
| 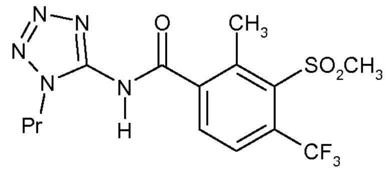 Nr. 7-032 | 20 | *50%* | 0% | 0% |

Tabelle C: Wirkung im Vorauflauf

| Verbindung | Dosierung [g/ha] | *Schädigung von Kulturpflanzen* | Herbizide Wirkung gegen | |
|---|---|---|---|---|
| | | *ZEAMX* | AVEFA | POLCO |
| 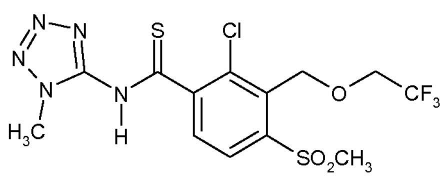 Nr. 1-75 | 320 | *0%* | 90% | 90% |

(fortgesetzt)

| Verbindung | Dosierung [g/ha] | *Schädigung von Kulturpflanzen* | Herbizide Wirkung gegen | |
|---|---|---|---|---|
| | | *ZEAMX* | AVEFA | POLCO |
| 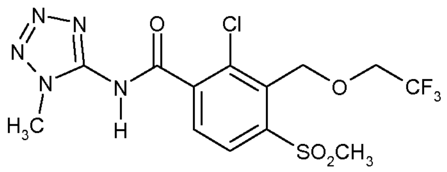 Nr. 4-260 | 320 | *20%* | 70% | 50% |

Tabelle D: Wirkung im Vorauflauf

| Verbindung | Dosierung [g/ha] | *Schädigung von Kulturpflanzen* | Herbizide Wirkung gegen | |
|---|---|---|---|---|
| | | *TRZAS* | PHBPU | POLCO |
| 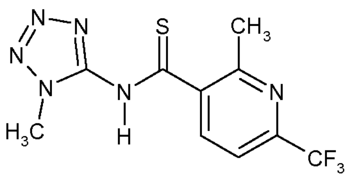 Nr. 4-1 | 320 | *0%* | 80% | 80% |
| 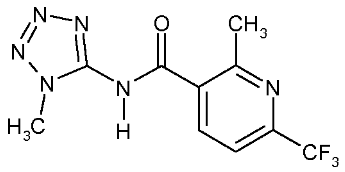 Nr. 8-19 | 320 | *20%* | 60% | 60% |

Tabelle E: Wirkung im Vorauflauf

| Verbindung | Dosierung [g/ha] | *Schädigung von Kulturpflanzen* | Herbizide Wirkung gegen | |
|---|---|---|---|---|
| | | *BRSNW* | ABUTH | AMARE |
| 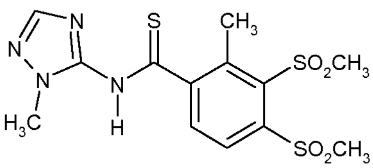 Nr. 2-4 | 320 | *0%* | 80% | 90% |
| 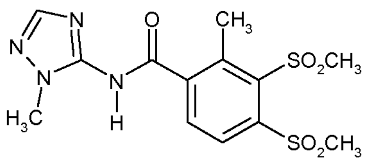 Nr. 1-188 | 320 | *20%* | 0% | 40% |

Tabelle F: Wirkung im Vorauflauf

| Verbindung | Dosierung [g/ha] | *Schädigung von Kulturpflanzen* | | Herbizide Wirkung gegen | |
|---|---|---|---|---|---|
| | | *BRSNW* | *ORYSA* | ECHCG | POLCO |
| 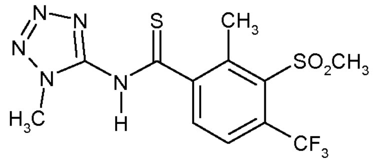 | 20 | *0%* | *20%* | 100% | 60% |
| 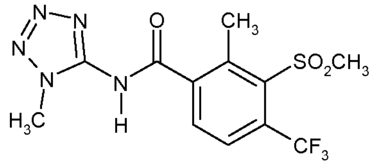 | 20 | *30%* | *50%* | 20% | 0% |

Tabelle G: Wirkung im Nachauflauf

| Verbindung | Dosierung [g/ha] | *Schädigung von Kulturpflanzen* | | Herbizide Wirkung gegen | |
|---|---|---|---|---|---|
| | | *ORYSA* | *TRZAS* | PHBPU | POLCO |
| 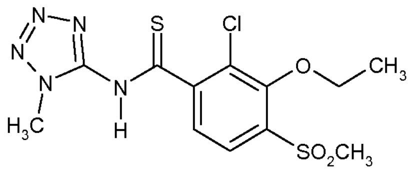 Nr. 1-83 | 5 | *10%* | 0% | 80% | 60% |
| 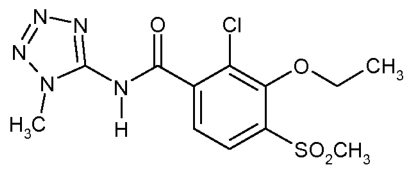 Nr. 4-278 | 5 | *40%* | *30%* | 20% | 40% |

Tabelle H: Wirkung im Nachauflauf

| Verbindung | Dosierung [g/ha] | Herbizide Wirkung gegen | | |
|---|---|---|---|---|
| | | ECHCG | SETVI | AMARE |
| 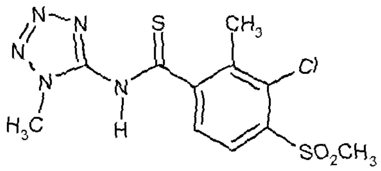 Nr. 1-29 | 5 | 70% | 40% | 40% |

(fortgesetzt)

| Verbindung | Dosierung [g/ha] | Herbizide Wirkung gegen | | |
|---|---|---|---|---|
| | | ECHCG | SETVI | AMARE |
| 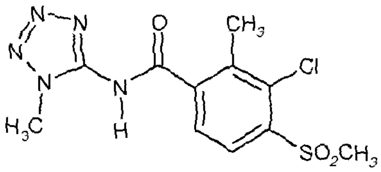 Nr. 4-173 | 5 | 10% | 0% | 20% |

Tabelle I: Wirkung im Nachauflauf

| Verbindung | Dosierung [g/ha] | *Schädigung von* Kulturpflanzen, *ZEAMX* | Herbizide Wirkung gegen | |
|---|---|---|---|---|
| | | | ALOMY | LOLMU |
| 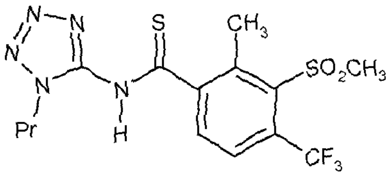 Nr. 1-9 | 80 | 0% | 80% | 20% |
| 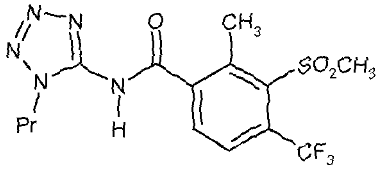 Nr. 7-032 | 80 | 20% | 30% | 0% |

Tabelle J: Wirkung im Nachauflauf

| Verbindung | Dosierung [g/ha] | *Schädigung von Kulturpflanzen TRZAS* | Herbizide Wirkung gegen POLCO |
|---|---|---|---|
| 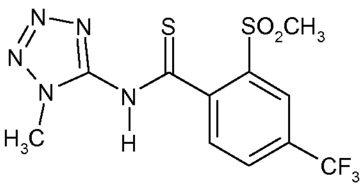 Nr. 1-2 | 80 | *20%* | 90% |
| 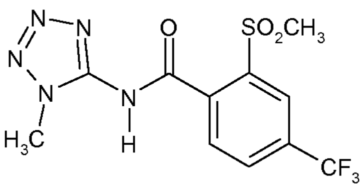 Nr. 4-25 | 80 | 60% | 70% |

Tabelle K: Wirkung im Nachauflauf

| Verbindung | Dosierung [g/ha] | *Schädigung von Kulturpflanzen* | Herbizide Wirkung gegen | |
|---|---|---|---|---|
| | | *ZEAMX* | AVEFA | LOLMU |
| 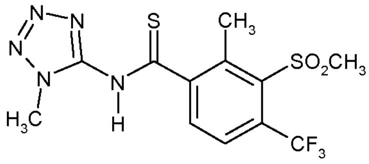 Nr. 1-7 | 80 | *50%* | 90% | 90% |
| 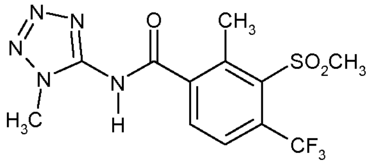 Nr. 4-137 | 80 | *70%* | 70% | 70% |

Die Ergenisse dieser orientierenden Vergleichsversuche zeigen, dass die erfindungsgemäßen Verbindungen mit einer Thioamid-Struktur höhere herbizide Wirkung und geringere Schädigung an Kulturpflanzen aufweisen als die strukturell ähnlichsten und aus WO 2012/028579 A1 bekannten Verbindungen mit einer Amid-Struktur.

## Patentansprüche

**1.** N-(Tetrazol-5-yl)- und N-(Triazol-5-yl)arylcarbonsäurethioamide der Formel (I) oder deren Salze

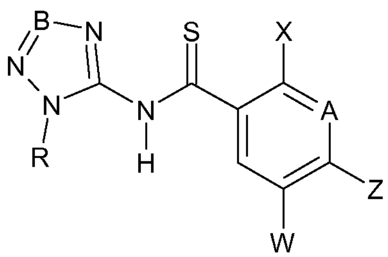

(I),

worin

A bedeutet N oder CY,
B bedeutet N oder CH,
X bedeutet Nitro, Halogen, Cyano, Formyl, Rhodano, $(C_1-C_6)$-Alkyl, Halogen-$(C_1-C_6)$-alkyl, $(C_2-C_6)$-Alkenyl, Halogen-$(C_2-C_6)$-alkenyl, $(C_2-C_6)$-Alkinyl, Halogen-$(C_3-C_6)$-alkinyl, $(C_3-C_6)$-Cycloalkyl, Halogen-$(C_3-C_6)$-cycloalkyl, $(C_3-C_6)$-Cycloalkyl-$(C_1-C_6)$-alkyl, Halogen-$(C_3-C_6)$-CyCloalkyl-$(C_1-C_6)$-alkyl, $COR^1$, $COOR^1$, $OCOOR^1$, $NR^1COOR^1$, $C(O)N(R^1)_2$, $NR^1C(O)N(R^1)_2$, $OC(O)N(R^1)_2$, $C(O)NR^1OR^1$, $OR^1$, $OCOR^1$, $OSO_2R^2$, $S(O)_nR^2$, $SO_2OR^1$, $SO_2N(R^1)_2$, $NR^1SO_2R^2$, $NR^1COR^1$, $(C_1-C_6)$-Alkyl-$S(O)_mR^2$, $(C_1-C_6)$-Alkyl-$OR^1$, $(C_1-C_6)$-Alkyl-$OCOR^1$, $(C_1-C_6)$-Alkyl-$OSO_2R^2$, $(C_1-C_6)$-Alkyl-$CO_2R^1$, $(C_1-C_6)$-Alkyl-$SO_2OR^1$, $(C_1-C_6)$-Alkyl-$CON(R^1)_2$, $(C_1-C_6)$-Alkyl-$SO_2N(R^1)_2$, $(C_1-C_6)$-Alkyl-$NR^1COR^1$, $(C_1-C_6)$-Alkyl-$NR^1SO_2R^2$, $NR_1R_2$, $P(O)(OR^5)_2$, $CH_2P(O)(OR^5)_2$, $(C_1-C_6)$-Alkyl-Heteroaryl oder $(C_1-C_6)$-Alkyl-Heterocyclyl, wobei die beiden letztgenannten Reste jeweils durch s Reste aus der Gruppe bestehend aus Halogen, $(C_1-C_6)$-Alkyl, Halogen-$(C_1-C_6)$-alkyl, $S(O)_n$-$(C_1-C_6)$-Alkyl, $(C_1-C_6)$-Alkoxy und Halogen-$(C_1-C_6)$-alkoxy substituiert sind, und wobei Heterocyclyl n Oxogruppen trägt,
Y bedeutet Wasserstoff, Nitro, Halogen, Cyano, Rhodano, $(C_1-C_6)$-Alkyl, Halogen-$(C_1-C_6)$-alkyl, $(C_2-C_6)$-Alkenyl, Halogen-$(C_2-C_6)$-alkenyl, $(C_2-C_6)$-Alkinyl, Halogen-$(C_2-C_6)$-alkinyl, $(C_3-C_6)$-Cycloalkyl, $(C_3-C_6)$-Cycloalke-

nyl, Halogen-($C_3$-$C_6$)-cycloalkyl, ($C_3$-$C_6$)-Cycloalkyl-($C_1$-$C_6$)-alkyl, Halogen-($C_3$-$C_6$)-CyCloalkyl-($C_1$-$C_6$)-alkyl, $COR^1$, $COOR^1$, $OCOOR^1$, $NR^1COOR^1$, $C(O)N(R^1)_2$, $NR^1C(O)N(R^1)_2$, $OC(O)N(R^1)_2$, $CO(NOR^1)R^1$, $CHNOR^1$, $CH_2ONC(R^3)_2$, $NR^1SO_2R^2$, $NR^1COR^1$, $OR^1$, $OSO_2R^2$, $S(O)_nR^2$, $SO_2OR^1$, $SO_2N(R^1)_2$ ($C_1$-$C_6$)-Alkyl-$S(O)_nR^2$, $NS(O)R^6R^7$, $S(O)R^8NR^9$, ($C_1$-$C_6$)-Alkyl-$OR^1$, ($C_1$-$C_6$)-Alkyl-$OCOR^1$, ($C_1$-$C_6$)-Alkyl-OSO2$R^2$, ($C_1$-$C_6$)-Alkyl-$CO_2R^1$, ($C_1$-$C_6$)-Alkyl-CN, ($C_1$-$C_6$)-Alkyl-$SO_2OR^1$, ($C_1$-$C_6$)-Alkyl-$CON(R^1)_2$, ($C_1$-$C_6$)-Alkyl-$SO_2N(R^1)_2$, ($C_1$-$C_6$)-Alkyl-$NR^1COR^1$, ($C_1$-$C_6$)-Alkyl-$NR^1SO_2R^2$, $N(R^1)_2$, $P(O)(OR^5)_2$, $CH_2P(O)(OR^5)_2$, ($C_1$-$C_6$)-Alkyl-Phenyl, ($C_1$-$C_6$)-Alkyl-Heteroaryl, ($C_1$-$C_6$)-Alkyl-Heterocyclyl, Phenyl, Heteroaryl oder Heterocyclyl, wobei die sechs letztgenannten Reste jeweils durch s Reste aus der Gruppe bestehend aus Halogen, Nitro, Cyano, ($C_1$-$C_6$)-Alkyl, Halogen-($C_1$-$C_6$)-alkyl, ($C_3$-$C_6$)-Cycloalkyl, $S(O)_n$-($C_1$-$C_6$)-Alkyl, ($C_1$-$C_6$)-Alkoxy, Halogen-($C_1$-$C_6$)-alkoxy, ($C_1$-$C_6$)-Alkoxy-($C_1$-$C_4$)-alkyl und Cyanomethyl substituiert sind, und wobei Heterocyclyl n Oxogruppen trägt,

Z bedeutet Halogen, Cyano, Nitro, Rhodano, Halogen-($C_1$-$C_6$)-alkyl, ($C_2$-$C_6$)-Alkenyl, Halogen-($C_2$-$C_6$)-alkenyl, ($C_2$-$C_6$)-Alkinyl, Halogen-($C_2$-$C_6$)-alkinyl, ($C_3$-$C_6$)-Cycloalkyl, Halogen-($C_3$-$C_6$)-cycloalkyl, ($C_3$-$C_6$)-Cycloalkyl-($C_1$-$C_6$)-alkyl, Halogen-($C_3$-$C_6$)-Cycloalkyl-($C_1$-$C_6$)-alkyl, $COR^1$, $COOR^1$, $OCOOR^1$, $NR^1COOR^1$, $C(O)N(R^1)_2$, $NR^1C(O)N(R^1)_2$, $OC(O)N(R^1)_2$, $C(O)NR^1OR^1$, $OSO_2R^2$, $S(O)_nR^2$, $SO_2OR^1$, $SO_2N(R^1)_2$, $NR^1SO_2R^2$, $NR^1COR^1$, ($C_1$-$C_6$)-Alkyl-$S(O)_nR^2$, ($C_1$-$C_6$)-Alkyl-$OR^1$, ($C_1$-$C_6$)-Alkyl-$OCOR^1$, ($C_1$-$C_6$)-Alkyl-$OSO_2R^2$, ($C_1$-$C_6$)-Alkyl-$CO_2R^1$, ($C_1$-$C_6$)-Alkyl-$SO_2OR^1$, ($C_1$-$C_6$)-Alkyl-$CON(R^1)_2$, ($C_1$-$C_6$)-Alkyl-$SO_2N(R^1)_2$, ($C_1$-$C_6$)-Alkyl-$NR^1COR^1$, ($C_1$-$C_6$)-Alkyl-$NR^1SO_2R^2$, $N(R^1)_2$, $P(O)(OR^5)_2$, Heteroaryl, Heterocyclyl oder Phenyl, wobei die drei letztgenannten Reste jeweils durch s Reste aus der Gruppe bestehend aus Halogen, Nitro, Cyano, ($C_1$-$C_6$)-Alkyl, Halogen-($C_1$-$C_6$)-alkyl, ($C_3$-$C_6$)-Cycloalkyl, $S(O)_n$-($C_1$-$C_6$)-Alkyl, ($C_1$-$C_6$)-Alkoxy oder Halogen-($C_1$-$C_6$)-alkoxy substituiert sind, und wobei Heterocyclyl n Oxogruppen trägt, oder

Z kann auch Wasserstoff, ($C_1$-$C_6$)-Alkyl oder ($C_1$-$C_6$)-Alkoxy bedeuten, falls Y für den Rest $S(O)_nR^2$ steht,

W bedeutet Wasserstoff, ($C_1$-$C_6$)-Alkyl, Halogen-($C_1$-$C_6$)-alkyl, ($C_2$-$C_6$)-Alkenyl, Halogen-($C_2$-$C_6$)-alkenyl, ($C_2$-$C_6$)-Alkinyl, Halogen-($C_2$-$C_6$)-alkinyl, ($C_3$-$C_7$)-Cycloalkyl, ($C_3$-$C_7$)-Halogencycloalkyl, ($C_1$-$C_6$)-Alkoxy, ($C_1$-$C_6$)-Halogenalkoxy, $S(O)_n$-($C_1$-$C_6$)-Alkyl, $S(O)_n$-($C_1$-$C_6$)-Halogenalkyl, ($C_1$-$C_6$)-Alkoxy-($C_1$-$C_4$)-alkyl, ($C_1$-$C_6$)-Alkoxy-($C_1$-$C_4$)-halogenalkyl, Halogen, Nitro, $NR^3COR^3$ oder Cyano,

R bedeutet ($C_1$-$C_8$)-Alkyl, Halogen-($C_1$-$C_8$)-alkyl, ($C_2$-$C_8$)-Alkenyl, Halogen-($C_2$-$C_8$)-alkenyl, ($C_2$-$C_8$)-Alkinyl oder Halogen-($C_2$-$C_8$)-alkinyl, wobei diese sechs vorstehend genannten Reste jeweils durch s Reste aus der Gruppe bestehend aus Hydroxy, Nitro, Cyano, $SiR^5_3$, $PO(OR^5)_2$, $S(O)_n$-($C_1$-$C_6$)-Alkyl, $S(O)_n$-($C_1$-$C_6$)-Halogenalkyl, ($C_1$-$C_6$)-Alkoxy, Halogen-($C_1$-$C_6$)-alkoxy, $N(R^3)_2$, $COR^3$, $COOR^3$, $OCOR^3$, $NR^3COR^3$, $NR^3SO_2R^4$, O($C_1$-$C_2$)-Alkyl-($C_3$-$C_6$)-Cycloalkyl, ($C_3$-$C_6$)-Cycloalkyl, Heteroaryl, Heterocyclyl, Phenyl, Q-Heteroaryl, Q-Heterocyclyl, Q-Phenyl und Q-Benzyl substituiert sind, wobei die sieben letztgenannten Reste jeweils durch s Reste aus der Gruppe bestehend aus Methyl, Ethyl, Methoxy, Trifluormethyl, Cyano und Halogen substituiert sind, und wobei Heterocyclyl n Oxogruppen trägt, oder R bedeutet jeweils durch s Reste aus der Gruppe bestehend aus Halogen, Nitro, Cyano, ($C_1$-$C_6$)-Alkyl, Halogen-($C_1$-$C_6$)-alkyl, ($C_3$-$C_6$)-Cycloalkyl, $S(O)_n$-($C_1$-$C_6$)-Alkyl, ($C_1$-$C_6$)-Alkoxy, Halogen-($C_1$-$C_6$)-alkoxy und ($C_1$-$C_6$)-Alkoxy-($C_1$-$C_4$)-alkyl substituiertes ($C_3$-$C_7$)-Cycloalkyl, Heteroaryl, Heterocyclyl oder Phenyl, wobei Heterocyclyl n Oxogruppen trägt,

Q bedeutet O, S, oder $NR^3$,

$R^1$ bedeutet Wasserstoff, ($C_1$-$C_6$)-Alkyl, ($C_1$-$C_6$)-Halogenalkyl, ($C_2$-$C_6$)-Alkenyl, ($C_2$-$C_6$)-Halogenalkenyl, ($C_2$-$C_6$)-Alkinyl, ($C_2$-$C_6$)-Halogenalkinyl, ($C_3$-$C_6$)-Cycloalkyl, ($C_3$-$C_6$)-Cycloalkenyl, ($C_3$-$C_6$)-Halogencycloalkyl, ($C_1$-$C_6$)-Alkyl-O-($C_1$-$C_6$)-alkyl, ($C_3$-$C_6$)-Cycloalkyl-($C_1$-$C_6$)-alkyl, Phenyl, Phenyl-($C_1$-$C_6$)-alkyl, Heteroaryl, ($C_1$-$C_6$)-Alkyl-Heteroaryl, Heterocycl, ($C_1$-$C_6$)-Alkyl-Heterocyclyl, ($C_1$-$C_6$)-Alkyl-O-Heteroaryl, ($C_1$-$C_6$)-Alkyl-O-Heterocyclyl, ($C_1$-$C_6$)-Alkyl-$NR^3$-Heteroaryl oder ($C_1$-$C_6$)-Alkyl-$NR^3$-Heterocyclyl, wobei die 21 letztgenannten Reste jeweils durch s Reste aus der Gruppe bestehend aus Cyano, Halogen, Nitro, Rhodano, $OR^3$, $S(O)_nR^4$, $N(R^3)_2$, $NR^3OR^3$, $COR^3$, $OCOR^3$, $SCOR^4$, $NR^3COR^3$, $NR^3SO_2R^4$, $CO_2R^3$, $COSR^4$, $CON(R^3)_2$ und ($C_1$-$C_4$)-Alkoxy-($C_2$-$C_6$)-alkoxycarbonyl substituiert sind, und wobei Heterocyclyl n Oxogruppen trägt,

$R^2$ bedeutet ($C_1$-$C_6$)-Alkyl, ($C_1$-$C_6$)-Halogenalkyl, ($C_2$-$C_6$)-Alkenyl, ($C_2$-$C_6$)-Halogenalkenyl, ($C_2$-$C_6$)-Alkinyl, ($C_2$-$C_6$)-Halogenalkinyl, ($C_3$-$C_6$)-Cycloalkyl, ($C_3$-$C_6$)-Cycloalkenyl, ($C_3$-$C_6$)-Halogencycloalkyl, ($C_1$-$C_6$)-Alkyl-O-($C_1$-$C_6$)-alkyl, ($C_3$-$C_6$)-Cycloalkyl-($C_1$-$C_6$)-alkyl, Phenyl, Phenyl-($C_1$-$C_6$)-alkyl, Heteroaryl, ($C_1$-$C_6$)-Alkyl-Heteroaryl, Heterocyclyl, ($C_1$-$C_6$)-Alkyl-Heterocyclyl, ($C_1$-$C_6$)-Alkyl-O-Heteroaryl, ($C_1$-$C_6$)-Alkyl-O-Heterocyclyl, ($C_1$-$C_6$)-Alkyl-$NR^3$-Heteroaryl oder ($C_1$-$C_6$)-Alkyl-$NR^3$-Heterocyclyl, wobei die 21 letztgenannten Reste jeweils durch s Reste aus der Gruppe bestehend aus Cyano, Halogen, Nitro, Rhodano, $OR^3$, $S(O)_nR^4$, $N(R^3)_2$, $NR^3OR^3$, $COR^3$, $OCOR^3$, $SCOR^4$, $NR^3COR^3$, $NR^3SO_2R^4$, $CO_2R^3$, $COSR^4$, $CON(R^3)_2$ und ($C_1$-$C_4$)-Alkoxy-($C_2$-$C_6$)-alkoxycarbonyl substituiert sind, und wobei Heterocyclyl n Oxogruppen trägt,

$R^3$ bedeutet Wasserstoff, ($C_1$-$C_6$)-Alkyl, ($C_2$-$C_6$)-Alkenyl, ($C_2$-$C_6$)-Alkinyl, ($C_3$-$C_6$)-Cycloalkyl, ($C_3$-$C_6$)-Cycloalkyl-($C_1$-$C_6$)-alkyl oder Phenyl,

$R^4$ bedeutet ($C_1$-$C_6$)-Alkyl, ($C_2$-$C_6$)-Alkenyl, ($C_2$-$C_6$)-Alkinyl, ($C_3$-$C_6$)-Cycloalkyl, ($C_3$-$C_6$)-Cycloalkyl-($C_1$-$C_6$)-alkyl oder Phenyl,

$R^5$ bedeutet ($C_1$-$C_4$)-Alkyl,

$R^6$ und $R^7$ bedeuten unabhängig voneinander jeweils ($C_1$-$C_6$)-Alkyl, Halogen-($C_1$-$C_6$)-alkyl, ($C_2$-$C_6$)-Alkenyl, Halogen-($C_2$-$C_6$)-alkenyl, ($C_2$-$C_6$)-Alkinyl, Halogen-($C_3$-$C_6$)-alkinyl, ($C_3$-$C_6$)-Cycloalkyl, Halogen-($C_3$-$C_6$)-cycloalkyl, ($C_3$-$C_6$)-Cycloalkyl-($C_1$-$C_6$)-alkyl, Halogen-($C_3$-$C_6$)-cycloalkyl-($C_1$-$C_6$)-alkyl, ($C_1$-$C_6$)-Alkoxy-($C_1$-$C_6$)-alkyl, Halogen-($C_1$-$C_6$)-alkoxy-($C_1$-$C_6$)-alkyl, Phenyl, Heteroaryl oder Heterocyclyl, wobei die drei letztgenannten Reste jeweils durch s Reste aus der Gruppe bestehend aus Nitro, Halogen, Cyano, Rhodano, ($C_1$-$C_6$)-Alkyl, Halogen-($C_1$-$C_6$)-alkyl, ($C_3$-$C_6$)-Cycloalkyl, $R^1O(O)C$, $(R^1)_2N(O)C$, $R^1O$, $(R^1)_2N$, $R^2(O)_nS$, $R^1O(O)_2S$, $(R^1)_2N(O)_2S$ und $R^1O$-($C_1$-$C_6$)-Alkyl substituiert sind, und wobei Heterocyclyl n Oxogruppen trägt, oder $R^6$ und $R^7$ bilden gemeinsam mit dem Schwefel-Atom, an dem sie gebunden sind, einen 3- bis 8-gliedrigen ungesättigten, teilgesättigten oder gesättigten Ring, der außer den Kohlenstoffatomen und außerdem Schwefelatom der Sulfoximinogruppe jeweils m Ringglieder aus der Gruppe bestehend aus $N(R^1)$, O und $S(O)_n$ enthält, und wobei dieser Ring jeweils durch s Reste aus der Gruppe bestehend aus Nitro, Halogen, Cyano, Rhodano, ($C_1$-$C_6$)-Alkyl, Halogen-($C_1$-$C_6$)-alkyl, ($C_3$-$C_6$)-Cycloalkyl, $R^1O(O)C$, $(R^1)_2N(O)C$, $R^1O$, $(R^1)_2N$, $R^2(O)_nS$, $R^1O(O)_2S$, $(R^1)_2N(O)_2S$ und $R^1O$-($C_1$-$C_6$)-Alkyl, substituiert ist, und wobei dieser Ring n Oxogruppen trägt,
$R^8$ bedeutet jeweils durch s Reste aus der Gruppe bestehend aus Nitro, Halogen, Cyano, Rhodano, ($C_3$-$C_6$)-Cycloalkyl, $R^1(O)C$, $R^1(R^1ON{=})C$, $R^1O(O)C$, $(R^1)_2N(O)C$, $R^1(R^1O)N(O)C$, $R^2(O)_2S(R^1)N(O)C$, $R^1O(O)_2S(R^1)N(O)C$, $(R^1)_2N(O)_2S(R^1)N(O)C$, $R^1S(O)C$, $R^1O$, $R^1(O)CO$, $R^2(O)_2SO$, $R^2O(O)CO$, $(R^1)_2N(O)CO$, $(R^1)_2N$, $R^1O(R^1)N$, $R^1(O)C(R^1)N$, $R^2(O)_2S(R^1)N$, $R^2O(O)C(R^1)N$, $(R^1)_2N(O)C(R^1)N$, $R^1O(O)_2S(R^1)N$, $(R^1)_2N(O)_2S(R^1)N$, $R^2(O)_nS$, $R^1C(O)S$, $R^1O(O)_2S$, $(R^1)_2N(O)_2S$, $R^1(O)C(R^1)N(O)_2S$, $R^2O(O)C(R^1)N(O)_2S$, $(R^1)_2N(O)C(R^1)N(O)_2S$ und $(R^5O)_2(O)P$ substituiertes ($C_1$-$C_6$)-Alkyl, ($C_2$-$C_6$)-Alkenyl oder ($C_2$-$C_6$)-Alkinyl,
oder
jeweils durch s Reste aus der Gruppe bestehend aus Nitro, Halogen, Cyano, Rhodano, ($C_1$-$C_6$)-Alkyl, Halogen-($C_1$-$C_6$)-alkyl, ($C_3$-$C_6$)-Cycloalkyl, $R^1(O)C$, $R^1(R^1ON{=})C$, $R^1O(O)C$, $(R^1)_2N(O)C$, $R^1(R^1O)N(O)C$, $R^2(O)_2S(R^1)N(O)C$, $R^1O(O)_2S(R^1)N(O)C$, $(R^1)_2N(O)_2S(R^1)N(O)C$, $R^1S(O)C$, $R^1O$, $R^1(O)CO$, $R^2(O)_2SO$, $R^2O(O)CO$, $(R^1)_2N(O)CO$, $(R^1)_2N$, $R^1O(R^1)N$, $R^1(O)C(R^1)N$, $R^2(O)_2S(R^1)N$, $R^2O(O)C(R^1)N$, $(R^1)_2N(O)C(R^1)N$, $R^1O(O)_2S(R^1)N$, $(R^1)_2N(O)_2S(R^1)N$, $R^2(O)_nS$, $R^1C(O)S$, $R^1O(O)_2S$, $(R^1)_2N(O)_2S$, $R^1(O)C(R^1)N(O)_2S$, $R^2O(O)C(R^1)N(O)_2S$, $(R^1)_2N(O)C(R^1)N(O)_2S$, $(R^5O)_2(O)P$ und $R^1O$-($C_1$-$C_6$)-Alkyl im cyclischen Teil substituiertes ($C_3$-$C_6$)-Cycloalkyl, ($C_3$-$C_6$)-Cycloalkenyl, Phenyl, Phenyl-($C_1$-$C_6$)-alkyl, Heteroaryl, Heteroaryl-($C_1$-$C_6$)-alkyl, Heterocyclyl, Heterocyclyl-($C_1$-$C_6$)-alkyl, Phenyl-O-($C_1$-$C_6$)-alkyl, Heteroaryl-O-($C_1$-$C_6$)-alkyl, Heterocyclyl-O-($C_1$-$C_6$)-alkyl, Phenyl-$N(R^1)$-($C_1$-$C_6$)-alkyl, Heteroaryl- $N(R^1)$-($C_1$-$C_6$)-alkyl, Heterocyclyl-$N(R^1)$-($C_1$-$C_6$)-alkyl, Phenyl-$S(O)_n$-($C_1$-$C_6$)-alkyl, Heteroaryl-S(O)n-($C_1$-$C_6$)-alkyl oder Heterocyclyl-S(O)n-($C_1$-$C_6$)-alkyl, und wobei Heterocyclyl n Oxogruppen trägt,
$R^9$ bedeutet Wasserstoff, Nitro, Halogen, Cyano, ($C_1$-$C_6$)-Alkyl, Halogen-($C_1$-$C_6$)-alkyl, ($C_3$-$C_6$)-Alkenyl, Halogen-($C_3$-$C_6$)-alkenyl, ($C_2$-$C_6$)-Alkinyl, Halogen-($C_3$-$C_6$)-alkinyl, ($C_3$-$C_6$)-Cycloalkyl, Halogen-($C_3$-$C_6$)-cycloalkyl, ($C_3$-$C_6$)-Cycloalkyl-($C_1$-$C_6$)-alkyl, Halogen-($C_3$-$C_6$)-cycloalkyl-($C_1$-$C_6$)-alkyl, $R^1(O)C$, $R^2O(O)C$, $(R^1)_2N(O)C$, $R^2S(O)C$, $(R^1)_2N(S)C$, $R^1(R^1O)N(O)C$, $R^2(O)_2S(R^1)N(O)C$, $(R^1)_2N(O)_2S(R^1)N(O)C$, $R^1O$, $(R^1)_2N$, $R^2(O)_nS$, $(R^2)_3Si$-($C_1$-$C_6$)-Alkyl-$(O)_nS$, $R^1O(O)_2S$, $(R^1)_2N(O)_2S$, $R^1(O)C(R^1)N(O)_2S$, $R^2O(O)C(R^1)N(O)_2S$, $(R^1)_2N(O)C(R^1)N(O)_2S$, $R^2(O)_2S(R^1)N(O)_2S$, $(R^5O)_2(O)P$, $(R^2)_3Si$, $R^1(O)C$-($C_1$-$C_6$)-Alkyl, $R^1O(O)C$-($C_1$-$C_6$)-Alkyl, $(R^1)_2N(O)C$-($C_1$-$C_6$)-Alkyl, $(R^1O)(R^1)N(O)C$-($C_1$-$C_6$)-Alkyl, $R^2(O)_2S(R^1)N(O)C$-($C_1$-$C_6$)-Alkyl, $R^1O(O)_2S(R^1)N(O)C$-($C_1$-$C_6$)-Alkyl, $(R^1)_2N(O)_2S(R^1)N(O)C$-($C_1$-$C_6$)-Alkyl, $R^1O$-($C_1$-$C_6$)-Alkyl, $R^1(O)CO$-($C_1$-$C_6$)-Alkyl, $R^2(2)_2SO$-($C_1$-$C_6$)-Alkyl, $R^2O(O)CO$-($C_1$-$C_6$)-Alkyl, $(R^1)_2N(O)CO$-($C_1$-$C_6$)-Alkyl, $(R^1)_2N$-($C_1$-$C_6$)-Alkyl, $R^1(O)C(R^1)N$-($C_1$-$C_6$)-Alkyl, $R^2(O)_2S(R^1)N$-($C_1$-$C_6$)-Alkyl, $R^2O(O)C(R^1)N$-($C_1$-$C_6$)-Alkyl, $(R^1)_2N(O)C(R^1)N$-($C_1$-$C_6$)-Alkyl, $R^1O(O)_2S(R^1)N$-($C_1$-$C_6$)-Alkyl, $(R^1)_2N(O)_2S(R^1)N$-($C_1$-$C_6$)-Alkyl, $R^2(O)_nS$-($C_1$-$C_6$)-Alkyl, $R^1O(O)_2S$-($C_1$-$C_6$)-Alkyl, $(R^1)_2N(O)_2S$-($C_1$-$C_6$)-Alkyl, $R^1(O)C(R^1)N(O)_2S$-($C_1$-$C_6$)-Alkyl, $R^2O(O)C(R^1)N(O)_2S$-($C_1$-$C_6$)-Alkyl, $(R^1)_2N(O)C(R^1)N(O)_2S$-($C_1$-$C_6$)-Alkyl, $(R^5O)_2(O)P$-($C_1$-$C_6$)-Alkyl, $(R^2)_3Si$-($C_1$-$C_6$)-Alkyl,
oder
jeweils durch s Reste aus der Gruppe bestehend aus Nitro, Halogen, Cyano, Rhodano, ($C_1$-$C_6$)-Alkyl, Halogen-($C_1$-$C_6$)-alkyl, ($C_3$-$C_6$)-Cycloalkyl, $R^1O(O)C$, $(R^1)_2N(O)C$, $R^1O$, $(R^1)_2N$, $R^2(O)_nS$, $R^1O(O)_2S$, $(R^1)_2N(O)_2S$ und $R^1O$-($C_1$-$C_6$)-Alkyl im cyclischen Teil substituiertes Phenyl, Heteroaryl, Heterocyclyl, Phenyl-($C_1$-$C_6$)-alkyl, Heteroaryl-($C_1$-$C_6$)-alkyl oder Heterocyclyl-($C_1$-$C_6$)-alkyl, und wobei Heterocyclyl n Oxogruppen trägt,
m bedeutet 0, 1, 2, 3 oder 4,
n bedeutet 0, 1 oder 2,
s bedeutet 0, 1, 2 oder 3.

**2.** N-(Tetrazol-5-yl)- und N-(Triazol-5-yl)arylcarbonsäurethioamide nach Anspruch 1, worin

A bedeutet N oder CY,
B bedeutet N oder CH,

X bedeutet Nitro, Halogen, Cyano, Rhodano, $(C_1-C_6)$-Alkyl, Halogen-$(C_1-C_6)$-alkyl, $(C_2-C_6)$-Alkenyl, Halogen-$(C_2-C_6)$-alkenyl, $(C_2-C_6)$-Alkinyl, Halogen-$(C_3-C_6)$-alkinyl, $(C_3-C_6)$-Cycloalkyl, Halogen-$(C_3-C_6)$-cycloalkyl, $(C_1-C_6)$-Alkyl-O-$(C_1-C_6)$-alkyl, $(C_3-C_6)$-Cycloalkyl-$(C_1-C_6)$-alkyl, Halogen-$(C_3-C_6)$-cycloalkyl-$(C_1-C_6)$-alkyl, $COR^1$, $OR^1$, $OCOR^1$, $OSO_2R^2$, $S(O)_nR^2$, $SO_2OR^1$, $SO_2N(R^1)_2$, $NR^1SO_2R^2$, $NR^1COR^1$, (C1-C6)-Alkyl-$S(O)_nR^2$, $(C_1-C_6)$-Alkyl-$OR^1$, $(C_1-C_6)$-Alkyl-$OCOR^1$, $(C_1-C_6)$-Alkyl-$OSO_2R^2$, $(C_1-C_6)$-Alkyl-$CO_2R^1$, $(C_1-C_6)$-Alkyl-$SO_2OR^1$, $(C_1-C_6)$-Alkyl-$CON(R^1)_2$, $(C_1-C_6)$-Alkyl-$SO_2N(R^1)_2$, $(C_1-C_6)$-Alkyl-$NR^1COR^1$ oder $(C_1-C_6)$-Alkyl-$NR^1SO_2R^2$, $(C_1-C_6)$-Alkyl-Heteroaryl oder $(C_1-C_6)$-Alkyl-Heterocyclyl, wobei die beiden letztgenannten Reste jeweils durch s Reste aus der Gruppe bestehend aus Halogen, $(C_1-C_6)$-Alkyl, Halogen-$(C_1-C_6)$-alkyl, $S(O)_n$-$(C_1-C_6)$-Alkyl, $(C_1-C_6)$-Alkoxy und Halogen-$(C_1-C_6)$-alkoxy substituiert sind, und wobei Heterocyclyl n Oxogruppen trägt,

Y bedeutet Wasserstoff, Nitro, Halogen, Cyano, Rhodano, $(C_1-C_6)$-Alkyl, Halogen-$(C_1-C_6)$-alkyl, $(C_2-C_6)$-Alkenyl, Halogen-$(C_2-C_6)$-alkenyl, $(C_2-C_6)$-Alkinyl, Halogen-$(C_3-C_6)$-alkinyl, $(C_3-C_6)$-Cycloalkyl, $(C_3-C_6)$-Cycloalkenyl, Halogen-$(C_3-C_6)$-cycloalkyl, $(C_3-C_6)$-Cycloalkyl-$(C_1-C_6)$-alkyl, Halogen-$(C_3-C_6)$-Cycloalkyl-$(C_1-C_6)$-alkyl, $COR^1$, $OR^1$, $COOR^1$, $CHNOR^1$, $CH_2ONC(R^3)_2$, $OSO_2R^2$, $S(O)_nR^2$, $SO_2OR^1$, $SO_2N(R^1)_2$, $NS(O)R^6R^7$, $S(O)R^8NR^9$, $N(R^1)_2$, $NR^1SO_2R^2$, $NR^1COR^1$, $(C_1-C_6)$-Alkyl-$S(O)_nR^2$, $(C_1-C_6)$-Alkyl-$OR^1$, $(C_1-C_6)$-Alkyl-$OCOR^1$, $(C_1-C_6)$-Alkyl-$OSO_2R^2$, $(C_1-C_6)$-Alkyl-$CO_2R^1$, $(C_1-C_6)$-Alkyl-$SO_2OR^1$, $(C_1-C_6)$-Alkyl-$CON(R^1)_2$, $(C_1-C_6)$-Alkyl-$SO_2N(R^1)_2$, $(C_1-C_6)$-Alkyl-$NR^1COR^1$, $(C_1-C_6)$-Alkyl-$NR^1SO_2R^2$, $(C_1-C_6)$-Alkyl-Phenyl, $(C_1-C_6)$-Alkyl-Heteroaryl, $(C_1-C_6)$-Alkyl-Heterocyclyl, Phenyl, Heteroaryl oder Heterocyclyl, wobei die sechs letztgenannten Reste jeweils durch s Reste aus der Gruppe bestehend Halogen, Nitro, Cyano, $(C_1-C_6)$-Alkyl, Halogen-$(C_1-C_6)$-alkyl, $(C_3-C_6)$-Cycloalkyl, $S(O)_n$-$(C_1-C_6)$-Alkyl, $(C_1-C_6)$-Alkoxy, Halogen-$(C_1-C_6)$-alkoxy, $(C_1-C_6)$-Alkoxy-$(C_1-C_4)$-alkyl und Cyanomethyl substituiert sind, und wobei Heterocyclyl n Oxogruppen trägt,

Z bedeutet Halogen, Cyano, Nitro, Rhodano, Halogen-$(C_1-C_6)$-alkyl, $(C_2-C_6)$-Alkenyl, Halogen-$(C_2-C_6)$-alkenyl, $(C_2-C_6)$-Alkinyl, Halogen-$(C_3-C_6)$-alkinyl, $(C_3-C_6)$-Cycloalkyl, Halogen-$(C_3-C_6)$-cycloalkyl, $(C_3-C_6)$-Cycloalkyl-$(C_1-C_6)$-alkyl, Halogen-$(C_3-C_6)$-cycloalkyl-$(C_1-C_6)$-alkyl, $COR^1$, $COOR^1$, $C(O)N(R^1)_2$, $C(O)NR^1OR^1$, $OSO_2R^2$, $S(O)_nR^2$, $SO_2OR^1$, $SO_2N(R^1)_2$, $NR^1SO_2R^2$, $NR^1COR^1$, $(C_1-C_6)$-Alkyl-$S(O)_nR^2$, $(C_1-C_6)$-Alkyl-$OR^1$, $(C_1-C_6)$-Alkyl-$OCOR^1$, $(C_1-C_6)$-Alkyl-$OSO_2R^2$, $(C_1-C_6)$-Alkyl-$CO_2R^1$, $(C_1-C_6)$-Alkyl-$SO_2OR^1$, $(C_1-C_6)$-Alkyl-$CON(R^1)_2$, $(C_1-C_6)$-Alkyl-$SO_2N(R^1)_2$, $(C_1-C_6)$-Alkyl-$NR^1COR^1$, $(C_1-C_6)$-Alkyl-$NR^1SO_2R^2$, 1,2,4-Triazol-1-yl, oder

Z kann auch Wasserstoff, $(C_1-C_6)$-Alkyl oder $(C_1-C_6)$-Alkoxy bedeuten, falls Y für den Rest $S(O)_nR^2$ steht,

W bedeutet Wasserstoff, $(C_1-C_6)$-Alkyl, Halogen-$(C_1-C_6)$-alkyl, $(C_1-C_6)$-Alkoxy, $(C_1-C_6)$-Halogenalkoxy, $S(O)_n$-$(C_1-C_6)$-alkyl, $S(O)_n$-$(C_1-C_6)$-halogenalkyl, $(C_1-C_6)$-Alkoxy-$(C_1-C_4)$-alkyl, Halogen, Nitro oder Cyano,

R bedeutet $(C_1-C_8)$-Alkyl, Halogen-$(C_1-C_8)$-alkyl, $(C_2-C_8)$-Alkenyl, Halogen-$(C_2-C_8)$-alkenyl, $(C_2-C_8)$-Alkinyl, Halogen-$(C_2-C_8)$-alkinyl, wobei diese sechs vorstehend genannten Reste jeweils durch s Reste aus der Gruppe bestehend aus Nitro, Cyano, $SiR^5_3$, $P(OR^5)_3$, $S(O)_n$-$(C_1-C_6)$-Alkyl, $(C_1-C_6)$-Alkoxy, Halogen-$(C_1-C_6)$-alkoxy, $N(R^3)_2$, $COR^3$, $COOR^3$, $OCOR^3$, $NR^3COR^3$, $NR^3SO_2R^4$, $(C_3-C_6)$-Cycloalkyl, Heteroaryl, Heterocyclyl, Phenyl, Q-Heteroaryl, Q-Heterocyclyl, Q-Phenyl und Q-Benzyl substituiert sind, wobei die sieben letztgenannten Reste jeweils durch s Reste aus der Gruppe bestehend aus Methyl, Ethyl, Methoxy, Trifluormethyl, Cyano und Halogen substituiert sind, und wobei Heterocyclyl n Oxogruppen trägt, oder

R bedeutet jeweils durch s Reste aus der Gruppe bestehend aus Halogen, Nitro, Cyano, $(C_1-C_6)$-Alkyl, Halogen-$(C_1-C_6)$-alkyl, $(C_3-C_6)$-Cycloalkyl, $S(O)_n$-$(C_1-C_6)$-Alkyl, $(C_1-C_6)$-Alkoxy, Halogen-$(C_1-C_6)$-alkoxy und $(C_1-C_6)$-Alkoxy-$(C_1-C_4)$-alkyl substituiertes $(C_3-C_7)$-Cycloalkyl, Heteroaryl, Heterocyclyl oder Phenyl,

Q bedeutet O, S, oder $NR^3$,

$R^1$ bedeutet Wasserstoff, $(C_1-C_6)$-Alkyl, $(C_2-C_6)$-Alkenyl, $(C_2-C_6)$-Alkinyl, $(C_3-C_6)$-Cycloalkyl, $(C_3-C_6)$-Cycloalkyl-$(C_1-C_6)$-alkyl, $(C_1-C_6)$-Alkyl-O-$(C_1-C_6)$-alkyl, Phenyl, Phenyl-$(C_1-C_6)$-alkyl, Heteroaryl, $(C_1-C_6)$-Alkyl-Heteroaryl, Heterocyclyl, $(C_1-C_6)$-Alkyl-Heterocyclyl, $(C_1-C_6)$-Alkyl-O-Heteroaryl, $(C_1-C_6)$-Alkyl-O-Heterocyclyl, $(C_1-C_6)$-Alkyl-$NR^3$-Heteroaryl oder $(C_1-C_6)$-Alkyl-$NR^3$-Heterocyclyl, wobei die sechzehn letztgenannten Reste jeweils durch s Reste aus der Gruppe bestehend aus Cyano, Halogen, Nitro, $OR^3$, $S(O)_nR^4$, $N(R^3)_2$, $NR^3OR^3$, $COR^3$, $OCOR^3$, $NR^3COR^3$, $NR^3SO_2R^4$, $CO_2R^3$, $CON(R^3)_2$ und $(C_1-C_4)$-Alkoxy-$(C_2-C_6)$-alkoxycarbonyl substituiert sind, und wobei Heterocyclyl n Oxogruppen trägt,

$R^2$ bedeutet $(C_1-C_6)$-Alkyl, $(C_2-C_6)$-Alkenyl, $(C_2-C_6)$-Alkinyl, $(C_3-C_6)$-Cycloalkyl, $(C_3-C_6)$-Cycloalkyl-$(C_1-C_6)$-alkyl, $(C_1-C_6)$-Alkyl-O-$(C_1-C_6)$-alkyl, Phenyl, Phenyl-$(C_1-C_6)$-alkyl, Heteroaryl, $(C_1-C_6)$-Alkyl-Heteroaryl, Heterocyclyl, $(C_1-C_6)$-Alkyl-Heterocyclyl, $(C_1-C_6)$-Alkyl-O-Heteroaryl, $(C_1-C_6)$-Alkyl-O-Heterocyclyl, $(C_1-C_6)$-Alkyl-$NR^3$-Heteroaryl oder $(C_1-C_6)$-Alkyl-$NR^3$-Heterocyclyl, wobei diese sechzehn letztgenannten Reste jeweils durch s Reste aus der Gruppe bestehend aus Cyano, Halogen, Nitro, $OR^3$, $S(O)_nR^4$, $N(R^3)_2$, $NR^3OR^3$, $NR^3SO_2R^4$, $COR^3$, $OCOR^3$, $NR^3COR^3$, $CO_2R^3$, $CON(R^3)_2$ und $(C_1-C_4)$-Alkoxy-$(C_2-C_6)$-alkoxycarbonyl substituiert sind, und wobei Heterocyclyl n Oxogruppen trägt,

$R^3$ bedeutet Wasserstoff, $(C_1-C_6)$-Alkyl, $(C_2-C_6)$-Alkenyl, $(C_2-C_6)$-Alkinyl, $(C_3-C_6)$-Cycloalkyl oder $(C_3-C_6)$-Cycloalkyl-$(C_1-C_6)$-alkyl,

$R^4$ bedeutet ($C_1$-$C_6$)-Alkyl, ($C_2$-$C_6$)-Alkenyl oder ($C_2$-$C_6$)-Alkinyl,

$R^5$ bedeutet Methyl oder Ethyl,

$R^6$ und $R^7$ bedeuten unabhängig voneinander jeweils ($C_1$-$C_6$)-Alkyl, Halogen-($C_1$-$C_6$)-alkyl, ($C_3$-$C_6$)-Cycloalkyl, Halogen-($C_3$-$C_6$)-cycloalkyl, ($C_3$-$C_6$)-Cycloalkyl-($C_1$-$C_6$)-alkyl, Halogen-($C_3$-$C_6$)-cycloalkyl-($C_1$-$C_6$)-alkyl, ($C_1$-$C_6$)-Alkoxy-($C_1$-$C_6$)-alkyl, Halogen-($C_1$-$C_6$)-alkoxy-($C_1$-$C_6$)-alkyl, Phenyl, Heteroaryl oder Heterocyclyl, wobei die drei letztgenannten Reste jeweils durch s Reste aus der Gruppe bestehend aus Nitro, Halogen, ($C_1$-$C_6$)-Alkyl, Halogen-($C_1$-$C_6$)-alkyl, $R^1O(O)C$, $(R^1)_2N(O)C$, $R^1O$, $(R^1)_2N$, $R^2(O)_nS$, und $R^1O$-($C_1$-$C_6$)-Alkyl substituiert sind, und wobei Heterocyclyl n Oxogruppen trägt,

oder $R^6$ und $R^7$ bilden gemeinsam mit dem Schwefel-Atom, an dem sie gebunden sind, einen 3- bis 8-gliedrigen ungesättigten, teilgesättigten oder gesättigten Ring, der außer den Kohlenstoffatomen und außer dem Schwefelatom der Sulfoximinogruppe jeweils m Ringglieder aus der Gruppe bestehend aus $N(R^1)$, O und $S(O)_n$ enthält, und wobei dieser Ring jeweils durch s Reste aus der Gruppe bestehend aus Halogen, ($C_1$-$C_6$)-Alkyl, Halogen-($C_1$-$C_6$)-alkyl, $R^1O(O)C$, $(R^1)_2N(O)C$, $R^1O$, $(R^1)_2N$, $R^2(O)_nS$, $R^1O(O)_2S$, $(R^1)_2N(O)_2S$ und $R^1O$-($C_1$-$C_6$)-Alkyl, substituiert ist, und wobei dieser Ring n Oxogruppen trägt,

$R^8$ bedeutet jeweils durch s Reste aus der Gruppe bestehend aus Halogen, Cyano, ($C_3$-$C_6$)-Cycloalkyl, $R^1(O)C$, $R^1(R^1ON{=})C$, $R^1O(O)C$, $(R^1)_2N(O)C$, $R^2(O)_2S(R^1)N(O)C$, $R^1O$, $(R^1)_2N$, $R^1(O)C(R^1)N$, $R^2(O)_2S(R^1)N$, $R^2O(O)C(R^1)N$, $(R^1)_2N(O)C(R^1)N$, $R^2(O)_nS$, $R^1O(O)_2S$, $(R^1)_2N(O)_2S$, $R^1(O)C(R^1)N(O)_2S$, $R^2O(O)C(R^1)N(O)_2S$ und $(R^1)_2N(O)C(R^1)N(O)_2S$ substituiertes ($C_1$-$C_6$)-Alkyl oder

jeweils durch s Reste aus der Gruppe bestehend aus Halogen, ($C_1$-$C_6$)-Alkyl, Halogen-($C_1$-$C_6$)-alkyl, ($C_3$-$C_6$)-Cycloalkyl, $R^1O(O)C$ und $(R^1)_2N(O)C$ substituiertes ($C_3$-$C_6$)-Cycloalkyl,

$R^9$ bedeutet Wasserstoff, Nitro, Cyano, ($C_1$-$C_6$)-Alkyl, Halogen-($C_1$-$C_6$)-alkyl, ($C_3$-$C_6$)-Cycloalkyl, Halogen-($C_3$-$C_6$)-cycloalkyl, ($C_3$-$C_6$)-Cycloalkyl-($C_1$-$C_6$)-alkyl, Halogen-($C_3$-$C_6$)-cycloalkyl-($C_1$-$C_6$)-alkyl, $R^1(O)C$, $R^2O(O)C$, $(R^1)_2N(O)C$, $R^2(O)_2S$, $R^1(O)C$-($C_1$-$C_6$)-Alkyl, $R^1O(O)C$-($C_1$-$C_6$)-Alkyl, $(R^1)_2N(O)C$-($C_1$-$C_6$)-Alkyl, $R^1O$-($C_1$-$C_6$)-Alkyl, $(R^1)_2N$-($C_1$-$C_6$)-Alkyl oder $R^2(O)_nS$-($C_1$-$C_6$)-Alkyl,

m bedeutet 0, 1 oder 2,

n bedeutet 0, 1 oder 2,

s bedeutet 0, 1, 2 oder 3.

**3.** N-(Tetrazol-5-yl)- und N-(Triazol-5-yl)arylcarbonsäurethioamide nach Anspruch 1 oder 2, worin

A bedeutet N oder CY,

B bedeutet N oder CH,

X bedeutet Nitro, Halogen, Cyano, ($C_1$-$C_6$)-Alkyl, Halogen-($C_1$-$C_6$)-alkyl, ($C_3$-$C_6$)-Cycloalkyl, $OR^1$, $S(O)_nR^2$, ($C_1$-$C_6$)-Alkyl-$S(O)_nR^2$, ($C_1$-$C_6$)-Alkyl-$OR^1$, ($C_1$-$C_6$)-Alkyl-$CON(R^1)_2$, ($C_1$-$C_6$)-Alkyl-$SO_2N(R^1)_2$, ($C_1$-$C_6$)-Alkyl-$NR^1COR^1$, ($C_1$-$C_6$)-Alkyl-$NR^1SO_2R^2$, ($C_1$-$C_6$)-Alkyl-Heteroaryl oder ($C_1$-$C_6$)-Alkyl-Heterocyclyl, wobei die beiden letztgenannten Reste jeweils durch s Reste Halogen, ($C_1$-$C_6$)-Alkyl, Halogen-($C_1$-$C_6$)-alkyl, $S(O)_n$-($C_1$-$C_6$)-Alkyl, ($C_1$-$C_6$)-Alkoxy und Halogen-($C_1$-$C_6$)-alkoxy substituiert sind, und wobei Heterocyclyl n Oxogruppen trägt,

Y Wasserstoff, Nitro, Halogen, Cyano, ($C_1$-$C_6$)-Alkyl, ($C_1$-$C_6$)-Halogenalkyl, $OR^1$, $S(O)_nR^2$, $SO_2N(R^1)2$, $NS(O)R^6R^7$, $S(O)R^8NR^9$, $N(R^1)_2$, $CHNOR^1$, $CH_2ONC(R^3)_2$, $NR^1SO_2R^2$, $NR^1COR^1$, ($C_1$-$C_6$)-Alkyl-$S(O)_nR^2$, ($C_1$-$C_6$)-Alkyl-$OR^1$, ($C_1$-$C_6$)-Alkyl-$CON(R^1)_2$, ($C_1$-$C_6$)-Alkyl-$SO_2N(R^1)_2$, ($C_1$-$C_6$)-Alkyl-$NR^1COR^1$, ($C_1$-$C_6$)-Alkyl-$NR^1SO_2R^2$, ($C_1$-$C_6$)-Alkyl-Phenyl, ($C_1$-$C_6$)-Alkyl-Heteroaryl, ($C_1$-$C_6$)-Alkyl-Heterocyclyl, Phenyl, Heteroaryl oder Heterocyclyl, wobei die sechs letztgenannten Reste jeweils durch s Reste aus der Gruppe bestehend Halogen, Nitro, Cyano, ($C_1$-$C_6$)-Alkyl, Halogen-($C_1$-$C_6$)-alkyl, ($C_3$-$C_6$)-Cycloalkyl, $S(O)_n$-($C_1$-$C_6$)-Alkyl, ($C_1$-$C_6$)-Alkoxy, Halogen-($C_1$-$C_6$)-alkoxy, ($C_1$-$C_6$)-Alkoxy-($C_1$-$C_4$)-alkyl und Cyanomethyl substituiert sind, und wobei Heterocyclyl n Oxogruppen trägt,

Z bedeutet Halogen, Cyano, Halogen-($C_1$-$C_6$)-alkyl, ($C_3$-$C_6$)-Cycloalkyl, $S(O)_nR^2$, 1,2,4-Triazol-1-yl,

oder

Z kann auch Wasserstoff, Methyl, Methoxy oder Ethoxy bedeuten, falls Y für den Rest $S(O)_nR^2$ steht,

W bedeutet Wasserstoff, Methyl, Ethyl, Methoxymethyl, Methoxy, Fluor, Chlor oder $S(O)_nCH_3$,

R bedeutet ($C_1$-$C_8$)-Alkyl, Halogen-($C_1$-$C_8$)-alkyl, ($C_2$-$C_8$)-Alkenyl, Halogen-($C_2$-$C_8$)-alkenyl, ($C_2$-$C_8$)-Alkinyl, Halogen-($C_2$-$C_8$)-alkinyl, wobei diese sechs vorstehend genannten Reste jeweils durch s Reste aus der Gruppe bestehend aus Cyano, $S(O)_n$-($C_1$-$C_6$)-Alkyl, ($C_1$-$C_6$)-Alkoxy, Halogen-($C_1$-$C_6$)-alkoxy, $COR^3$, $COOR^3$, $OCOR^3$, $NR^3COR^3$, $NR^3SO_2R^4$, ($C_3$-$C_6$)-Cycloalkyl, Heteroaryl, Heterocyclyl und Phenyl substituiert sind, wobei die drei letztgenannten Reste jeweils durch s Reste aus der Gruppe bestehend aus Methyl, Ethyl, Methoxy, Trifluormethyl, Cyano und Halogen substituiert sind, und wobei Heterocyclyl 0 bis 2 Oxogruppen trägt,

oder

R bedeutet durch s Reste aus der Gruppe bestehend aus Halogen, Nitro, Cyano, ($C_1$-$C_6$)-Alkyl, Halo-

gen-($C_1$-$C_6$)-alkyl, ($C_3$-$C_6$)-Cycloalkyl, $S(O)_n$-($C_1$-$C_6$)-Alkyl, ($C_1$-$C_6$)-Alkoxy, Halogen-($C_1$-$C_6$)-alkoxy, ($C_1$-$C_6$)-Alkoxy-($C_1$-$C_4$)-alkyl substituiertes Phenyl,
$R^1$ bedeutet Wasserstoff, ($C_1$-$C_6$)-Alkyl, ($C_2$-$C_6$)-Alkenyl, ($C_2$-$C_6$)-Alkinyl, ($C_3$-$C_6$)-Cycloalkyl, ($C_3$-$C_6$)-Cycloalkyl-($C_1$-$C_6$)-alkyl, ($C_1$-$C_6$)-Alkyl-O-($C_1$-$C_6$)-alkyl, Phenyl, Phenyl-($C_1$-$C_6$)-alkyl, Heteroaryl, ($C_1$-$C_6$)-Alkyl-Heteroaryl, Heterocyclyl, ($C_1$-$C_6$)-Alkyl-Heterocyclyl, ($C_1$-$C_6$)-Alkyl-O-Heteroaryl, ($C_1$-$C_6$)-Alkyl-O-Heterocyclyl, ($C_1$-$C_6$)-Alkyl-$NR^3$-Heteroaryl oder ($C_1$-$C_6$)-Alkyl-$NR^3$-Heterocyclyl, wobei die sechzehn letztgenannten Reste jeweils durch s Reste aus der Gruppe bestehend aus Cyano, Halogen, Nitro, $OR^3$, $S(O)_nR^4$, $N(R^3)_2$, $NR^3OR^3$, $COR^3$, $OCOR^3$, $NR^3COR^3$, $NR^3SO_2R^4$, $CO_2R^3$, $CON(R^3)_2$ und ($C_1$-$C_4$)-Alkoxy-($C_2$-$C_6$)-alkoxycarbonyl substituiert sind, und wobei Heterocyclyl n Oxogruppen trägt,
$R^2$ bedeutet jeweils durch s Reste aus der Gruppe bestehend aus Halogen und $OR^3$ substituiertes ($C_1$-$C_6$)-Alkyl, ($C_3$-$C_6$)-Cycloalkyl oder ($C_3$-$C_6$)-Cycloalkyl-($C_1$-$C_6$)-alkyl,
$R^3$ bedeutet Wasserstoff oder ($C_1$-$C_6$)-Alkyl,
$R^4$ bedeutet ($C_1$-$C_6$)-Alkyl,
$R^6$ und $R^7$ bedeuten unabhängig voneinander jeweils Methyl, Ethyl oder n-Propyl, oder
$R^6$ und $R^7$ bilden gemeinsam mit dem Schwefel-Atom, an dem sie gebunden sind, einen 5- oder 6-gliedrigen gesättigten Ring, der außer den Kohlenstoffatomen und außer dem Schwefelatom der Sulfoximinogruppe m Sauerstoffatome enthält,
$R^8$ bedeutet Methyl, Ethyl oder n-Propyl,
$R^9$ bedeutet Wasserstoff oder Cyano,
m bedeutet 0 oder 1,
n bedeutet 0, 1 oder 2,
s bedeutet 0, 1, 2 oder 3.

**4.** Herbizide Mittel, **gekennzeichnet durch** einen herbizid wirksamen Gehalt an mindestens einer Verbindung der Formel (I) gemäß einem der Ansprüche 1 bis 3.

**5.** Herbizide Mittel nach Anspruch 4 in Mischung mit Formulierungshilfsmitteln.

**6.** Herbizide Mittel nach Anspruch 4 oder 5 enthaltend mindestens einen weiteren pestizid wirksamen Stoff aus der Gruppe Insektizide, Akarizide, Herbizide, Fungizide, Safener und Wachstumsregulatoren.

**7.** Herbizide Mittel nach Anspruch 6 enthaltend einen Safener.

**8.** Herbizide Mittel nach Anspruch 7 enthaltend cyprosulfamid, cloquintocet-mexyl, mefenpyr-diethyl oder isoxadifen-ethyl.

**9.** Herbizide Mittel nach einem der Ansprüche 6 bis 8 enthaltend ein weiteres Herbizid.

**10.** Verfahren zur Bekämpfung unerwünschter Pflanzen, **dadurch gekennzeichnet, daß** man eine wirksame Menge mindestens einer Verbindung der Formel (I) gemäß einem der Ansprüche 1 bis 3 oder eines herbiziden Mittels nach einem der Ansprüche 4 bis 9 auf die Pflanzen oder auf den Ort des unerwünschten Pflanzenwachstums appliziert.

**11.** Verwendung von Verbindungen der Formel (I) gemäß einem der Ansprüche 1 bis 3 oder von herbiziden Mitteln nach einem der Ansprüche 4 bis 9 zur Bekämpfung unerwünschter Pflanzen.

**12.** Verwendung nach Anspruch 11, **dadurch gekennzeichnet, daß** die Verbindungen der Formel (I) zur Bekämpfung unerwünschter Pflanzen in Kulturen von Nutzpflanzen eingesetzt werden.

**13.** Verwendung nach Anspruch 12, **dadurch gekennzeichnet, daß** die Nutzpflanzen transgene Nutzpflanzen sind.

## Claims

**1.** N-(Tetrazol-5-yl)- and N-(triazol-5-yl)arylcarboxylic acid thioamides of the formula (I) or salts thereof

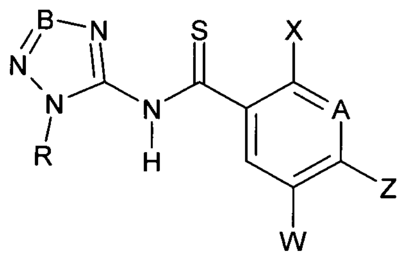

(I),

in which

A represents N or CY,
B represents N or CH,
X represents nitro, halogen, cyano, formyl, thiocyanato, $(C_1-C_6)$-alkyl, halo-$(C_1-C_6)$-alkyl, $(C_2-C_6)$-alkenyl, halo-$(C_2-C_6)$-alkenyl, $(C_2-C_6)$-alkynyl, halo-$(C_3-C_6)$-alkynyl, $(C_3-C_6)$-cycloalkyl, halo-$(C_3-C_6)$-cycloalkyl, $(C_3-C_6)$-cycloalkyl-$(C_1-C_6)$-alkyl, halo-$(C_3-C_6)$-cycloalkyl-$(C_1-C_6)$-alkyl, $COR^1$, $COOR^1$, $OCOOR^1$,$NR^1COOR^1$, $C(O)N(R^1)_2$, $NR^1C(O)N(R^1)_2$, $OC(O)N(R^1)_2$, $C(O)NR^1OR^1$, $OR^1$, $OCOR^1$, $OSO_2R^2$, $S(O)_nR^2$, $SO_2OR^1$, $SO_2N(R^1)_2$, $NR_1SO_2R^2$, $NR^1COR^1$, $(C_1-C_6)$-alkyl-$S(O)_nR^2$, $(C_1-C_6)$-alkyl-$OR^1$, $(C_1-C_6)$-alkyl-$OCOR^1$, $(C_1-C_6)$-alkyl-$OSO_2R^2$, $(C_1-C_6)$-alkyl-$CO_2R^1$, $(C_1-C_6)$-alkyl-$SO_2OR^1$, $(C_1-C_6)$-alkyl-$CON(R^1)_2$, $(C_1-C_6)$-alkyl-$SO_2N(R^1)_2$, $(C_1-C_6)$-alkyl-$NR^1COR^1$, $(C_1-C_6)$-alkyl-$NR^1SO_2R^2$, $NR_1R_2$, $P(O)(OR^5)_2$, $CH_2P(O)(OR^5)_2$, $(C_1-C_6)$-alkylheteroaryl or $(C_1-C_6)$-alkylheterocyclyl, where the two last-mentioned radicals are each substituted by s radicals from the group consisting of halogen, $(C_1-C_6)$-alkyl, halo-$(C_1-C_6)$-alkyl, $S(O)_n$-$(C_1-C_6)$-alkyl, $(C_1-C_6)$-alkoxy and halo-$(C_1-C_6)$-alkoxy, and where heterocyclyl carries n oxo groups,
Y represents hydrogen, nitro, halogen, cyano, thiocyanato, $(C_1-C_6)$-alkyl, halo-$(C_1-C_6)$-alkyl, $(C_2-C_6)$-alkenyl, halo-$(C_2-C_6)$-alkenyl, $(C_2-C_6)$-alkynyl, halo-$(C_2-C_6)$-alkynyl, $(C_3-C_6)$-cycloalkyl, $(C_3-C_6)$-cycloalkenyl, halo-$(C_3-C_6)$-cycloalkyl, $(C_3-C_6)$-cycloalkyl-$(C_1-C_6)$-alkyl, halo-$(C_3-C_6)$-cycloalkyl-$(C_1-C_6)$-alkyl, $COR^1$, $COOR^1$, $OCOOR^1$, $NR^1COOR^1$, $C(O)N(R^1)_2$, $NR^1C(O)N(R^1)_2$, $OC(O)N(R^1)_2$, $CO(NOR^1)R^1$, $CHNOR^1$, $CH_2ONC(R^3)_2$, $NR^1SO_2R^2$, $NR^1COR^1$, $OR^1$, $OSO_2R^2$, $S(O)_nR^2$, $SO_2OR^1$, $SO_2N(R^1)_2$ $(C_1-C_6)$-alkyl-$S(O)_nR^2$, $NS(O)R^6R^7$, $S(O)R^8NR^9$, $(C_1-C_6)$-alkyl-$OR^1$, $(C_1-C_6)$-alkyl-$OCOR^1$, $(C_1-C_6)$-alkyl-$OSO_2R^2$, $(C_1-C_6)$-alkyl-$CO_2R^1$, $(C_1-C_6)$-alkyl-CN, $(C_1-C_6)$-alkyl-$SO_2OR^1$, $(C_1-C_6)$-alkyl-$CON(R^1)2$, $(C_1-C_6)$-alkyl-$SO_2N(R^1)_2$, $(C_1-C_6)$-alkyl-$NR^1COR^1$, $(C_1-C_6)$-alkyl-$NR^1SO_2R^2$, $N(R^1)_2$, $P(O)(OR^5)_2$, $CH_2P(O)(OR^5)_2$, $(C_1-C_6)$-alkylphenyl, $(C_1-C_6)$-alkyl-heteroaryl, $(C_1-C_6)$-alkylheterocyclyl, phenyl, heteroaryl or heterocyclyl, where the six last-mentioned radicals are each substituted by s radicals from the group consisting of halogen, nitro, cyano, $(C_1-C_6)$-alkyl, halo-$(C_1-C_6)$-alkyl, $(C_3-C_6)$-cycloalkyl, $S(O)_n$-$(C_1-C_6)$-alkyl, $(C_1-C_6)$-alkoxy, halo-$(C_1-C_6)$-alkoxy, $(C_1-C_6)$-alkoxy-$(C_1-C_4)$-alkyl and cyanomethyl, and where heterocyclyl carries n oxo groups,
Z represents halogen, cyano, nitro, thiocyanato, halo-$(C_1-C_6)$-alkyl, $(C_2-C_6)$-alkenyl, halo-$(C_2-C_6)$-alkenyl, $(C_2-C_6)$-alkynyl, halo-$(C_2-C_6)$-alkynyl, $(C_3-C_6)$-cycloalkyl, halo-$(C_3-C_6)$-cycloalkyl, $(C_3-C_6)$-cycloalkyl-$(C_1-C_6)$-alkyl, halo-$(C_3-C_6)$-cycloalkyl-$(C_1-C_6)$-alkyl, $COR^1$, $COOR^1$, $OCOOR^1$, $NR^1COOR^1$, $C(O)N(R^1)_2$, $NR^1C(O)N(R^1)_2$, $OC(O)N(R^1)_2$, $C(O)NR^1OR^1$, $OSO_2R^2$, $S(O)_nR^2$, $SO_2OR^1$, $SO_2N(R^1)_2$, $NR^1SO_2R^2$, $NR^1COR^1$, $(C_1-C_6)$-alkyl-$S(O)_nR^2$, $(C_1-C_6)$-alkyl-$OR^1$, $(C_1-C_6)$-alkyl-$OCOR^1$, $(C_1-C_6)$-alkyl-$OSO_2R^2$, $(C_1-C_6)$-alkyl-$CO_2R^1$, $(C_1-C_6)$-alkyl-$SO_2OR^1$, $(C_1-C_6)$-alkyl-$CON(R^1)_2$, $(C_1-C_6)$-alkyl-$SO_2N(R^1)_2$, $(C_1-C_6)$-alkyl-$NR^1COR^1$, $(C_1-C_6)$-alkyl-$NR^1SO_2R^2$, $N(R^1)_2$, $P(O)(OR^5)_2$, heteroaryl, heterocyclyl or phenyl, where the three last-mentioned radicals are each substituted by s radicals from the group consisting of halogen, nitro, cyano, $(C_1-C_6)$-alkyl, halo-$(C_1-C_6)$-alkyl, $(C_3-C_6)$-cycloalkyl, $S(O)_n$-$(C_1-C_6)$-alkyl, $(C_1-C_6)$-alkoxy and halo-$(C_1-C_6)$-alkoxy, and where heterocyclyl carries n oxo groups, or
Z may also represent hydrogen, $(C_1-C_6)$-alkyl or $(C_1-C_6)$-alkoxy if Y represents the $S(O)_nR^2$ radical,
W represents hydrogen, $(C_1-C_6)$-alkyl, halo-$(C_1-C_6)$-alkyl, $(C_2-C_6)$-alkenyl, halo-$(C_2-C_6)$-alkenyl, $(C_2-C_6)$-alkynyl, halo-$(C_2-C_6)$-alkynyl, $(C_3-C_7)$-cycloalkyl, $(C_3-C_7)$-halocycloalkyl, $(C_1-C_6)$-alkoxy, $(C_1-C_6)$-haloalkoxy, $S(O)_n$-$(C_1-C_6)$-alkyl, $S(O)_n$-$(C_1-C_6)$-haloalkyl, $(C_1-C_6)$-alkoxy-$(C_1-C_4)$-alkyl, $(C_1-C_6)$-alkoxy-$(C_1-C_4)$-haloalkyl, halogen, nitro, $NR^3COR^3$ or cyano,
R represents $(C_1-C_8)$-alkyl, halo-$(C_1-C_8)$-alkyl, $(C_2-C_8)$-alkenyl, halo-$(C_2-C_8)$-alkenyl, $(C_2-C_8)$-alkynyl or halo-$(C_2-C_8)$-alkynyl, where these six abovementioned radicals are each substituted by s radicals from the group consisting of hydroxy, nitro, cyano, $SiR^5_3$, $PO(OR^5)_2$, $S(O)_n$-$(C_1-C_6)$-alkyl, $S(O)_n$-$(C_1-C_6)$-haloalkyl, $(C_1-C_6)$-alkoxy, halo-$(C_1-C_6)$-alkoxy, $N(R^3)_2$, $COR^3$, $COOR^3$, $OCOR^3$, $NR^3COR^3$, $NR^3SO_2R^4$, $O(C_1-C_2)$-alkyl-$(C_3-C_6)$-cycloalkyl, $(C_3-C_6)$-cycloalkyl, heteroaryl, heterocyclyl, phenyl, Q-heteroaryl, Q-heterocyclyl, Q-phenyl and Q-benzyl, where the seven last-mentioned radicals are each substituted by s radicals from the group consisting of methyl, ethyl, methoxy, trifluoromethyl, cyano and halogen, and where heterocyclyl

carries n oxo groups, or

R represents ($C_3$-$C_7$)-cycloalkyl, heteroaryl, heterocyclyl or phenyl, each of which is substituted by s radicals from the group consisting of halogen, nitro, cyano, ($C_1$-$C_6$)-alkyl, halo-($C_1$-C6)-alkyl, ($C_3$-$C_6$)-cycloalkyl, $S(O)_n$-($C_1$-$C_6$)-alkyl, ($C_1$-$C_6$)-alkoxy, halo-($C_1$-$C_6$)-alkoxy and ($C_1$-$C_6$)-alkoxy-($C_1$-$C_6$)-alkyl, where heterocyclyl carries n oxo groups,

Q represents O, S or $NR^3$,

$R^1$ represents hydrogen, ($C_1$-$C_6$)-alkyl, ($C_1$-$C_6$)-haloalkyl, ($C_2$-$C_6$)-alkenyl, ($C_2$-$C_6$)-haloalkenyl, ($C_2$-$C_6$)-alkynyl, ($C_2$-$C_6$)-haloalkynyl, ($C_3$-$C_6$)-cycloalkyl, ($C_3$-$C_6$)-cycloalkenyl, ($C_3$-$C_6$)-halocycloalkyl, ($C_1$-$C_6$)-alkyl-O-($C_1$-$C_6$)-alkyl, ($C_3$-$C_6$)-cycloalkyl-($C_1$-$C_6$)-alkyl, phenyl, phenyl-($C_1$-$C_6$)-alkyl, heteroaryl, ($C_1$-$C_6$)-alkylheteroaryl, heterocyclyl, ($C_1$-$C_6$)-alkylheterocycl, ($C_1$-$C_6$)-alkyl-O-heteroaryl, ($C_1$-$C_6$)-alkyl-O-heterocyclyl, ($C_1$-$C_6$)-alkyl-$NR^3$-heteroaryl or ($C_1$-$C_6$)-alkyl-$NR^3$-heterocyclyl, where the 21 last-mentioned radicals are each substituted by s radicals from the group consisting of cyano, halogen, nitro, thiocyanato, $OR^3$, $S(O)_nR^4$, $N(R^3)_2$, $NR^3OR^3$, $COR^3$, $OCOR^3$, $SCOR^4$, $NR^3COR^3$, $NR^3SO_2R^4$, $CO_2R^3$, $COSR^4$, $CON(R^3)_2$ and ($C_1$-$C_4$)-alkoxy-($C_2$-$C_6$)-alkoxycarbonyl, and where heterocyclyl carries n oxo groups,

$R^2$ represents ($C_1$-$C_6$)-alkyl, ($C_1$-$C_6$)-haloalkyl, ($C_2$-$C_6$)-alkenyl, ($C_2$-$C_6$)-haloalkenyl, ($C_2$-$C_6$)-alkynyl, ($C_2$-$C_6$)-haloalkynyl, ($C_3$-$C_6$)-cycloalkyl, ($C_3$-$C_6$)-cycloalkenyl, ($C_3$-$C_6$)-halocycloalkyl, ($C_1$-$C_6$)-alkyl-O-($C_1$-$C_6$)-alkyl, ($C_3$-$C_6$)-cycloalkyl-($C_1$-$C_6$)-alkyl, phenyl, phenyl-($C_1$-$C_6$)-alkyl, heteroaryl, ($C_1$-$C_6$)-alkylheteroaryl, heterocyclyl, ($C_1$-$C_6$)-alkylheterocyclyl, ($C_1$-$C_6$)-alkyl-O-heteroaryl, ($C_1$-$C_6$)-alkyl-O-heterocyclyl, ($C_1$-$C_6$)-alkyl-$NR^3$-heteroaryl or ($C_1$-$C_6$)-alkyl-$NR^3$-heterocyclyl, where the 21 last-mentioned radicals are each substituted by s radicals from the group consisting of cyano, halogen, nitro, thiocyanato, $OR^3$, $S(O)_nR^4$, $N(R^3)_2$, $NR^3OR^3$, $COR^3$, $OCOR^3$, $SCOR^4$, $NR^3COR^3$, $NR^3SO_2R^4$, $CO_2R^3$, $COSR^4$, $CON(R^3)_2$ and ($C_1$-$C_4$)-alkoxy-($C_2$-$C_6$)-alkoxycarbonyl, and where heterocyclyl carries n oxo groups,

$R^3$ represents hydrogen, ($C_1$-$C_6$)-alkyl, ($C_2$-$C_6$)-alkenyl, ($C_2$-$C_6$)-alkynyl, ($C_3$-$C_6$)-cycloalkyl, ($C_3$-$C_6$)-cycloalkyl-($C_1$-$C_6$)-alkyl or phenyl,

$R^4$ represents ($C_1$-$C_6$)-alkyl, ($C_2$-$C_6$)-alkenyl, ($C_2$-$C_6$)-alkynyl, ($C_3$-$C_6$)-cycloalkyl, ($C_3$-$C_6$)-cycloalkyl-($C_1$-C6)-alkyl or phenyl,

$R^5$ represents ($C_1$-$C_4$)-alkyl,

$R^6$ and $R^7$ each independently of one another represent ($C_1$-$C_6$)-alkyl, halo-($C_1$-$C_6$)-alkyl, ($C_2$-$C_6$)-alkenyl, halo-($C_2$-$C_6$)-alkenyl, ($C_2$-$C_6$)-alkynyl, halo-($C_3$-$C_6$)-alkynyl, ($C_3$-$C_6$)-cycloalkyl, halo-($C_3$-$C_6$)-cycloalkyl, ($C_3$-$C_6$)-cycloalkyl-($C_1$-$C_6$)-alkyl, halo-($C_3$-$C_6$)-cycloalkyl-($C_1$-$C_6$)-alkyl, ($C_1$-$C_6$)-alkoxy-($C_1$-$C_6$)-alkyl, halo-($C_1$-$C_6$)-alkoxy-($C_1$-$C_6$)-alkyl, phenyl, heteroaryl or heterocyclyl, where the three last-mentioned radicals are each substituted by s radicals from the group consisting of nitro, halogen, cyano, thiocyanato, ($C_1$-$C_6$)-alkyl, halo-($C_1$-$C_6$)-alkyl, ($C_3$-$C_6$)-cycloalkyl, $R^1O(O)C$, $(R^1)_2N(O)C$, $R^1O$, $(R^1)_2N$, $R^2(O)_nS$, $R^1O(O)_2S$, $(R^1)_2N(O)_2S$ and $R^1O$-($C_1$-$C_6$)-alkyl, and where heterocyclyl carries n oxo groups, or

$R^6$ and $R^7$ together with the sulfur atom to which they are bonded form a 3- to 8-membered unsaturated, semisaturated or saturated ring which contains, apart from the carbon atoms and apart from the sulfur atom of the sulfoximino group, in each case m ring members from the group consisting of $N(R^1)$, O and $S(O)_n$, and where this ring in each case is substituted by s radicals from the group consisting of nitro, halogen, cyano, thiocyanato, ($C_1$-$C_6$)-alkyl, halo-($C_1$-$C_6$)-alkyl, ($C_3$-$C_6$)-cycloalkyl, $R^1O(O)C$, $(R^1)_2N(O)C$, $R^1O$, $(R^1)_2N$, $R^2(O)_nS$, $R^1O(O)_2S$, $(R^1)_2N(O)_2S$ and $R^1O$-($C_1$-$C_6$)-alkyl, and where this ring bears n oxo groups,

$R^8$ represents ($C_1$-$C_6$)-alkyl, ($C_2$-$C_6$)-alkenyl or ($C_2$-$C_6$)-alkynyl, each of which is substituted by s radicals from the group consisting of nitro, halogen, cyano, thiocyanato, ($C_3$-$C_6$)-cycloalkyl, $R^1(O)C$, $R^1(R^1ON=)C$, $R^1O(O)C$, $(R^1)_2N(O)C$, $R^1(R^1O)N(O)C$, $R^2(O)_2S(R^1)N(O)C$, $R^1O(O)_2S(R^1)N(O)C$, $(R^1)_2N(O)_2S(R^1)N(O)C$, $R^1S(O)C$, $R^1O$, $R^1(O)CO$, $R^2(O)_2SO$, $R^2O(O)CO$, $(R^1)_2N(O)CO$, $(R^1)_2N$, $R^1O(R^1)N$, $R^1(O)C(R^1)N$, $R^2(O)_2S(R^1)N$, $R^2O(O)C(R^1)N$, $(R^1)_2N(O)C(R^1)N$, $R^1O(O)_2S(R^1)N$, $(R^1)_2N(O)_2S(R^1)N$,$R^2(O)_nS$, $R^1C(O)S$, $R^1O(O)_2S$, $(R^1)_2N(O)_2S$, $R^1(O)C(R^1)N(O)_2S$, $R^2O(O)C(R^1)N(O)_2S$, $(R^1)_2N(O)C(R^1)N(O)_2S$ and $(R^5O)_2(O)P$,

or

($C_3$-$C_6$)-cycloalkyl, ($C_3$-$C_6$)-cycloalkenyl, phenyl, phenyl-($C_1$-$C_6$)-alkyl, heteroaryl, heteroaryl-($C_1$-$C_6$)-alkyl, heterocyclyl, heterocyclyl-($C_1$-$C_6$)-alkyl, phenyl-O-($C_1$-$C_6$)-alkyl, heteroaryl-O-($C_1$-$C_6$)-alkyl, heterocyclyl-O-($C_1$-$C_6$)-alkyl, phenyl-$N(R^1)$-($C_1$-$C_6$)-alkyl, heteroaryl- $N(R^1)$-($C_1$-$C_6$)-alkyl, heterocyclyl- $N(R^1)$-($C_1$-$C_6$)-alkyl, phenyl-$S(O)_n$-($C_1$-$C_6$)-alkyl, heteroaryl-$S(O)_n$-($C_1$-$C_6$)-alkyl or heterocyclyl-$S(O)_n$-($C_1$-$C_6$)-alkyl, each of which is substituted in the cyclic moiety by s radicals from the group consisting of nitro, halogen, cyano, thiocyanato, ($C_1$-$C_6$)-alkyl, halo-($C_1$-$C_6$)-alkyl, ($C_3$-$C_6$)-cycloalkyl, $R^1(O)C$, $R^1(R^1ON=)C$, $R^1O(O)C$, $(R^1)_2N(O)C$, $R^1(R^1O)N(O)C$, $R^2(O)_2S(R^1)N(O)C$, $R^1O(O)_2S(R^1)N(O)C$, $(R^1)_2N(O)_2S(R^1)N(O)C$, $R^1S(O)C$, $R^1O$, $R^1(O)CO$, $R^2(O)_2SO$, $R^2O(O)CO$,$(R^1)_2N(O)CO$,$(R^1)_2N$, $R^1O(R^1)N$, $R^1(O)C(R^1)N$, $R^2(O)_2S(R^1)N$, $R^2O(O)C(R^1)N$, $(R^1)_2N(O)C(R^1)N$, $R^1O(O)_2S(R^1)N$, $(R^1)_2N(O)_2S(R^1)N$, $R^2(O)_nS$, $R^1C(O)S$, $R^1O(O)_2S$, $(R^1)_2N(O)_2S$, $R^1(O)C(R^1)N(O)_2S$, $R^2O(O)C(R^1)N(O)_2S$, $(R^1)_2N(O)C(R^1)N(O)_2S$, $(R^5O)_2(O)P$ and $R^1O$-($C_1$-$C_6$)-alkyl, and where heterocyclyl carries n oxo groups,

$R^9$ represents hydrogen, nitro, halogen, cyano, $(C_1-C_6)$-alkyl, halo-$(C_1-C_6)$-alkyl, $(C_3-C_6)$-alkenyl, halo-$(C_3-C_6)$-alkenyl, $(C_2-C_6)$-alkynyl, halo-$(C_3-C_6)$-alkynyl, $(C_3-C_6)$-Cycloalkyl, halo-$(C_3-C_6)$-cycloalkyl, $(C_3-C_6)$-cycloalkyl-$(C_1-C_6)$-alkyl, halo-$(C_3-C_6)$-cycloalkyl-$(C_1-C_6)$-alkyl, $R^1(O)C$, $R^2O(O)C$, $(R^1)_2N(O)C$, $R^2S(O)C$, $(R^1)_2N(S)C$, $R^1(R^1O)N(O)C$, $R^2(O)_2S(R^1)N(O)C$, $(R^1)_2N(O)_2S(R^1)N(O)C$, $R^1O$, $(R^1)_2N$, $R^2(O)_nS$, $(R^2)_3Si$-$(C_1-C_6)$-alkyl-(O)nS, $R^1O(O)_2S$, $(R^1)_2N(O)_2S$, $R^1(O)C(R^1)N(O)_2S$, $R^2O(O)C(R^1)N(O)_2S$, $(R^1)_2N(O)C(R^1)N(O)_2S$, $R^2(O)_2S(R^1)N(O)_2S$, $(R^5O)_2(O)P$, $(R^2)_3Si$, $R^1(O)C$-$(C_1-C_6)$-alkyl, $R^1O(O)C$-$(C_1-C_6)$-alkyl, $(R^1)_2N(O)C$-$(C_1-C_6)$-alkyl, $(R^1O)(R^1)N(O)C$-$(C_1-C_6)$-alkyl, $R^2(O)_2S(R^1)N(O)C$-$(C_1-C_6)$-alkyl, $R^1O(O)_2S(R^1)N(O)C$-$(C_1-C_6)$-alkyl, $(R^1)_2N(O)_2S(R^1)N(O)C$-$(C_1-C_6)$-alkyl, $R^1O$-$(C_1-C_6)$-alkyl, $R^1(O)CO$-$(C_1-C_6)$-alkyl, $R^2(O)_2SO$-$(C_1-C_6)$-alkyl, $R^2O(O)CO$-$(C_1-C_6)$-alkyl, $(R^1)_2N(O)CO$-$(C_1-C_6)$-alkyl, $(R^1)_2N$-$(C_1-C_6)$-alkyl, $R^1(O)C(R^1)N$-$(C_1-C_6)$-alkyl, $R^2(O)_2S(R^1)N$-$(C_1-C_6)$-alkyl, $R^2O(O)C(R^1)N$-$(C_1-C_6)$-alkyl, $(R^1)_2N(O)C(R^1)N$-$(C_1-C_6)$-alkyl, $R^1O(O)_2S(R^1)N$-$(C_1-C_6)$-alkyl, $(R^1)_2N(O)_2S(R^1)N$-$(C_1-C_6)$-alkyl, $R^2(O)_nS$-$(C_1-C_6)$-alkyl, $R^1O(O)_2S$-$(C_1-C_6)$-alkyl, $(R^1)_2N(O)_2S$-$(C_1-C_6)$-alkyl, $R^1(O)C(R^1)N(O)_2S$-$(C_1-C_6)$-alkyl, $R^2O(O)C(R^1)N(O)_2S$-$(C_1-C_6)$-alkyl, $(R^1)_2N(O)C(R^1)N(O)_2S$-$(C_1-C_6)$-alkyl, $(R^5O)_2(O)P$-$(C_1-C_6)$-alkyl, $(R^2)_3Si$-$(C_1-C_6)$-alkyl,

or

phenyl, heteroaryl, heterocyclyl, phenyl-$(C_1-C_6)$-alkyl, heteroaryl-$(C_1-C_6)$-alkyl or heterocyclyl-$(C_1-C_6)$-alkyl, each of which is substituted in the cyclic moiety by s radicals from the group consisting of nitro, halogen, cyano, thiocyanato, $(C_1-C_6)$-alkyl, halo-$(C_1-C_6)$-alkyl, $(C_3-C_6)$-cycloalkyl, $R^1O(O)C$, $(R^1)_2N(O)C$, $R^1O$, $(R^1)_2N$, $R^2(O)_nS$, $R^1O(O)_2S$, $(R^1)_2N(O)_2S$ and $R^1O$-$(C_1-C_6)$-alkyl, and where heterocyclyl carries n oxo groups,

m represents 0, 1, 2, 3 or 4,

n represents 0, 1 or 2,

s represents 0, 1, 2 or 3.

**2.** N-(Tetrazol-5-yl)- and N-(triazol-5-yl)arylcarboxylic acid thioamides according to Claim 1 in which

A represents N or CY,

B represents N or CH,

X represents nitro, halogen, cyano, thiocyanato, $(C_1-C_6)$-alkyl, halo-$(C_1-C_6)$-alkyl, $(C_2-C_6)$-alkenyl, halo-$(C_2-C_6)$-alkenyl, $(C_2-C_6)$-alkynyl, halo-$(C_3-C_6)$-alkynyl, $(C_3-C_6)$-cycloalkyl, halo-$(C_3-C_6)$-cycloalkyl, $(C_1-C_6)$-alkyl-O-$(C_1-C_6)$-alkyl, $(C_3-C_6)$-cycloalkyl-$(C_1-C_6)$-alkyl, halo-$(C_3-C_6)$-cycloalkyl-$(C_1-C_6)$-alkyl, $COR^1$, $OR^1$, $OCOR^1$, $OSO_2R^2$, $S(O)_nR^2$, $SO_2OR^1$, $SO_2N(R^1)_2$, $NR^1SO_2R^2$, $NR^1COR^1$, $(C_1-C_6)$-alkyl-$S(O)_nR^2$, $(C_1-C_6)$-alkyl-$OR^1$, $(C_1-C_6)$-alkyl-$OCOR^1$, $(C_1-C_6)$-alkyl-$OSO_2R^2$, $(C_1-C_6)$-alkyl-$CO_2R^1$, $(C_1-C_6)$-alkyl-$SO_2OR^1$, $(C_1-C_6)$-alkyl-$CON(R^1)_2$, $(C_1-C_6)$-alkyl-$SO_2N(R^1)_2$, $(C_1-C_6)$-alkyl-$NR^1COR^1$ or $(C_1-C_6)$-alkyl-$NR^1SO_2R^2$, $(C_1-C_6)$-alkyl-heteroaryl or $(C_1-C_6)$-alkyl-heterocyclyl, where the two last-mentioned radicals are each substituted by s radicals from the group consisting of halogen, $(C_1-C_6)$-alkyl, halo-$(C_1-C_6)$-alkyl, $S(O)_n$-$(C_1-C_6)$-alkyl, $(C_1-C_6)$-alkoxy and halo-$(C_1-C_6)$-alkoxy, and where heterocyclyl carries n oxo groups,

Y represents hydrogen, nitro, halogen, cyano, thiocyanato, $(C_1-C_6)$-alkyl, halo-$(C_1-C_6)$-alkyl, $(C_2-C_6)$-alkenyl, halo-$(C_2-C_6)$-alkenyl, $(C_2-C_6)$-alkynyl, halo-$(C_3-C_6)$-alkynyl, $(C_3-C_6)$-cycloalkyl, $(C_3-C_6)$-cycloalkenyl, halo-$(C_3-C_6)$-cycloalkyl, $(C_3-C_6)$-cycloalkyl-$(C_1-C_6)$-alkyl, halo-$(C_3-C_6)$-cycloalkyl-$(C_1-C_6)$-alkyl, $COR^1$, $OR^1$, $COOR^1$, $CHNOR^1$, $CH_2ONC(R^3)_2$, $OSO_2R^2$, $S(O)_nR^2$, $SO_2OR^1$, $SO_2N(R^1)_2$, $NS(O)R^6R^7$, $S(O)R^8NR^9$, $N(R^1)_2$, $NR^1SO_2R^2$, $NR^1COR^1$, $(C_1-C_6)$-alkyl-$S(O)_nR^2$, $(C_1-C_6)$-alkyl-$OR^1$, $(C_1-C_6)$-alkyl-$OCOR^1$, $(C_1-C_6)$-alkyl-$OSO_2R^2$, $(C_1-C_6)$-alkyl-$CO_2R^1$, $(C_1-C_6)$-alkyl-$SO_2OR^1$, $(C_1-C_6)$-alkyl-$CON(R^1)2$, $(C_1-C_6)$-alkyl-$SO_2N(R^1)_2$, $(C_1-C_6)$-alkyl-$NR^1COR^1$, $(C_1-C_6)$-alkyl-$NR^1SO_2R^2$, $(C_1-C_6)$-alkyl-phenyl, $(C_1-C_6)$-alkyl-heteroaryl, $(C_1-C_6)$-alkyl-heterocyclyl, phenyl, heteroaryl or heterocyclyl, where the six last-mentioned radicals are each substituted by s radicals from the group consisting of halogen, nitro, cyano, $(C_1-C_6)$-alkyl, halo-$(C_1-C_6)$-alkyl, $(C_3-C_6)$-cycloalkyl, $S(O)_n$-$(C_1-C_6)$-alkyl, $(C_1-C_6)$-alkoxy, halo-$(C_1-C_6)$-alkoxy, $(C_1-C_6)$-alkoxy-$(C_1-C_4)$-alkyl and cyanomethyl, and where heterocyclyl carries n oxo groups,

Z represents halogen, cyano, nitro, thiocyanato, halo-$(C_1-C_6)$-alkyl, $(C_2-C_6)$-alkenyl, halo-$(C_2-C_6)$-alkenyl, $(C_2-C_6)$-alkynyl, halo-$(C_3-C_6)$-alkynyl, $(C_3-C_6)$-cycloalkyl, halo-$(C_3-C_6)$-cycloalkyl, $(C_3-C_6)$-cycloalkyl-$(C_1-C_6)$-alkyl, halo-$(C_3-C_6)$-cycloalkyl-$(C_1-C_6)$-alkyl, $COR^1$, $COOR^1$, $C(O)N(R^1)_2$, $C(O)NR^1OR^1$, $OSO_2R^2$, $S(O)_nR^2$, $SO_2OR^1$, $SO_2N(R^1)_2$, $NR^1SO_2R^2$, $NR^1COR^1$, $(C_1-C_6)$-alkyl-S(O)n$R^2$, $(C_1-C_6)$-alkyl-$OR^1$, $(C_1-C_6)$-alkyl-$OCOR^1$, $(C_1-C_6)$-alkyl-$OSO_2R^2$, $(C_1-C_6)$-alkyl-$CO_2R^1$, $(C_1-C_6)$-alkyl-$SO_2OR^1$, $(C_1-C_6)$-alkyl-$CON(R^1)_2$, $(C_1-C_6)$-alkyl-$SO_2N(R^1)_2$, $(C_1-C_6)$-alkyl-$NR^1COR^1$, $(C_1-C_6)$-alkyl-$NR^1SO_2R^2$ or 1,2,4-triazol-1-yl,

or

Z may also represent hydrogen, $(C_1-C_6)$-alkyl or $(C_1-C_6)$-alkoxy if Y represents the $S(O)_nR^2$ radical,

W represents hydrogen, $(C_1-C_6)$-alkyl, halo-$(C_1-C_6)$-alkyl, $(C_1-C_6)$-alkoxy, $(C_1-C_6)$-haloalkoxy, $S(O)_n$-$(C_1-C_6)$-alkyl, $S(O)_n$-$(C_1-C_6)$-haloalkyl, $(C_1-C_6)$-alkoxy-$(C_1-C_4)$-alkyl, halogen, nitro or cyano,

R represents $(C_1-C_8)$-alkyl, halo-$(C_1-C_8)$-alkyl, $(C_2-C_8)$-alkenyl, halo-$(C_2-C_8)$-alkenyl, $(C_2-C_8)$-alkynyl, ha-

lo-($C_2$-$C_8$)-alkynyl, where these six abovementioned radicals are each substituted by s radicals from the group consisting of nitro, cyano, $SiR^5_3$, $P(OR^5)_3$, $S(O)_n$-($C_1$-$C_6$)-alkyl, ($C_1$-$C_6$)-alkoxy, halo-($C_1$-$C_6$)-alkoxy, $N(R^3)_2$, $COR^3$, $COOR^3$, $OCOR^3$, $NR^3COR^3$, $NR^3SO_2R^4$, ($C_3$-$C_6$)-cycloalkyl, heteroaryl, heterocyclyl, phenyl, Q-heteroaryl, Q-heterocyclyl, Q-phenyl and Q-benzyl, where the seven last-mentioned radicals are each substituted by s radicals from the group consisting of methyl, ethyl, methoxy, trifluoromethyl, cyano and halogen, and where heterocyclyl carries n oxo groups, or

R represents ($C_3$-$C_7$)-cycloalkyl, heteroaryl, heterocyclyl or phenyl, each of which is substituted by s radicals from the group consisting of halogen, nitro, cyano, ($C_1$-$C_6$)-alkyl, halo-($C_1$-$C_6$)-alkyl, ($C_3$-$C_6$)-cycloalkyl, $S(O)_n$-($C_1$-$C_6$)-alkyl, ($C_1$-$C_6$)-alkoxy, halo-($C_1$-$C_6$)-alkoxy and ($C_1$-$C_6$)-alkoxy-($C_1$-$C_4$)-alkyl,

Q represents O, S or $NR^3$,

$R^1$ represents hydrogen, ($C_1$-$C_6$)-alkyl, ($C_2$-$C_6$)-alkenyl, ($C_2$-$C_6$)-alkynyl, ($C_3$-$C_6$)-cycloalkyl, ($C_3$-$C_6$)-cycloalkyl-($C_1$-$C_6$)-alkyl, ($C_1$-$C_6$)-alkyl-O-($C_1$-$C_6$)-alkyl, phenyl, phenyl-($C_1$-$C_6$)-alkyl, heteroaryl, ($C_1$-$C_6$)-alkyl-heteroaryl, heterocyclyl, ($C_1$-$C_6$)-alkylheterocyclyl, ($C_1$-$C_6$)-alkyl-O-heteroaryl, ($C_1$-$C_6$)-alkyl-O-heterocyclyl, ($C_1$-$C_6$)-alkyl-$NR^3$-heteroaryl or ($C_1$-$C_6$)-alkyl-$NR^3$-heterocyclyl, where the sixteen last-mentioned radicals are each substituted by s radicals from the group consisting of cyano, halogen, nitro, $OR^3$, $S(O)_nR^4$, $N(R^3)_2$, $NR^3OR^3$, $COR^3$, $OCOR^3$, $NR^3COR^3$, $NR^3SO_2R^4$, $CO_2R^3$, $CON(R^3)_2$ and ($C_1$-$C_4$)-alkoxy-($C_2$-$C_6$)-alkoxycarbonyl, and where heterocyclyl carries n oxo groups,

$R^2$ represents ($C_1$-$C_6$)-alkyl, ($C_2$-$C_6$)-alkenyl, ($C_2$-$C_6$)-alkynyl, ($C_3$-$C_6$)-cycloalkyl, ($C_3$-$C_6$)-cycloalkyl-($C_1$-$C_6$)-alkyl, ($C_1$-$C_6$)-alkyl-O-($C_1$-$C_6$)-alkyl, phenyl, phenyl-($C_1$-$C_6$)-alkyl, heteroaryl, ($C_1$-$C_6$)-alkyl-heteroaryl, heterocyclyl, ($C_1$-$C_6$)-alkylheterocyclyl, ($C_1$-$C_6$)-alkyl-O-heteroaryl, ($C_1$-$C_6$)-alkyl-O-heterocyclyl, ($C_1$-$C_6$)-alkyl-$NR^3$-heteroaryl or ($C_1$-$C_6$)-alkyl-$NR^3$-heterocyclyl, where these sixteen last-mentioned radicals are each substituted by s radicals from the group consisting of cyano, halogen, nitro, $OR^3$, $S(O)_nR^4$, $N(R^3)_2$, $NR^3OR^3$, $NR^3SO_2R^4$, $COR^3$, $OCOR^3$, $NR^3COR^3$, $CO_2R^3$, $CON(R^3)_2$ and ($C_1$-$C_4$)-alkoxy-($C_2$-$C_6$)-alkoxycarbonyl, and where heterocyclyl carries n oxo groups,

$R^3$ represents hydrogen, ($C_1$-$C_6$)-alkyl, ($C_2$-$C_6$)-alkenyl, ($C_2$-$C_6$)-alkynyl, ($C_3$-$C_6$)-cycloalkyl or ($C_3$-$C_6$)-cycloalkyl-($C_1$-$C_6$)-alkyl,

$R^4$ represents ($C_1$-$C_6$)-alkyl, ($C_2$-$C_6$)-alkenyl or ($C_2$-$C_6$)-alkynyl,

$R^5$ represents methyl or ethyl,

$R^6$ and $R^7$ each independently of one another represent ($C_1$-$C_6$)-alkyl, halo-($C_1$-$C_6$)-alkyl, ($C_3$-$C_6$)-cycloalkyl, halo-($C_3$-$C_6$)-cycloalkyl, ($C_3$-$C_6$)-cycloalkyl-($C_1$-$C_6$)-alkyl, halo-($C_3$-$C_6$)-cycloalkyl-($C_1$-$C_6$)-alkyl, ($C_1$-$C_6$)-alkoxy-($C_1$-$C_6$)-alkyl, halo-($C_1$-$C_6$)-alkoxy-($C_1$-$C_6$)-alkyl, phenyl, heteroaryl or heterocyclyl, where the three last-mentioned radicals are each substituted by s radicals from the group consisting of nitro, halogen, ($C_1$-$C_6$)-alkyl, halo-($C_1$-$C_6$)-alkyl, $R^1O(O)C$, $(R^1)_2N(O)C$, $R^1O$, $(R^1)_2N$, $R^2(O)_nS$ and $R^1O$-($C_1$-$C_6$)-alkyl, and where heterocyclyl carries n oxo groups,

or $R^6$ and $R^7$ together with the sulfur atom to which they are attached form a 3- to 8-membered unsaturated, partially saturated or saturated ring which contains, in addition to the carbon atoms and in addition to the sulfur atom of the sulfoximino group, in each case m ring members from the group consisting of $N(R^1)$, O and $S(O)_n$, and where this ring is in each case substituted by s radicals from the group consisting of halogen, ($C_1$-$C_6$)-alkyl, halo-($C_1$-$C_6$)-alkyl, $R^1O(O)C$, $(R^1)_2N(O)C$, $R^1O$, $(R^1)_2N$, $R^2(O)_nS$, $R^1O(O)_2S$, $(R^1)_2N(O)_2S$ and $R^1O$-($C_1$-$C_6$)-alkyl, and where this ring carries n oxo groups,

$R^8$ represents ($C_1$-$C_6$)-alkyl which is in each case substituted by s radicals from the group consisting of halogen, cyano, ($C_3$-$C_6$)-cycloalkyl, $R^1(O)C$, $R^1(R^1ON{=})C$, $R^1O(O)C$, $(R^1)_2N(O)C$, $R^2(O)_2S(R^1)N(O)C$, $R^1O$, $(R^1)_2N$, $R^1(O)C(R^1)N$, $R^2(O)_2S(R^1)N$, $R^2O(O)C(R^1)N$, $(R^1)_2N(O)C(R^1)N$, $R^2(O)_nS$, $R^1O(O)_2S$, $(R^1)_2N(O)_2S$, $R^1(O)C(R^1)N(O)_2S$, $R^2O(O)C(R^1)N(O)_2S$ and $(R^1)_2N(O)C(R^1)N(O)_2S$ or ($C_3$-$C_6$)-cycloalkyl which is in each case substituted by s radicals from the group consisting of halogen, ($C_1$-$C_6$)-alkyl, halo-($C_1$-$C_6$)-alkyl, ($C_3$-$C_6$)-cycloalkyl, $R^1O(O)C$ and $(R^1)_2N(O)C$,

$R^9$ represents hydrogen, nitro, cyano, ($C_1$-$C_6$)-alkyl, halo-($C_1$-$C_6$)-alkyl, ($C_3$-$C_6$)-cycloalkyl, halo-($C_3$-$C_6$)-cycloalkyl, ($C_3$-$C_6$)-cycloalkyl-($C_1$-$C_6$)-alkyl, halo-($C_3$-$C_6$)-cycloalkyl-($C_1$-$C_6$)-alkyl, $R^1(O)C$, $R^2O(O)C$, $(R^1)_2N(O)C$, $R^2(O)_2S$, $R^1(O)C$-($C_1$-$C_6$)-alkyl, $R^1O(O)C$-($C_1$-$C_6$)-alkyl, $(R^1)_2N(O)C$-($C_1$-$C_6$)-alkyl, $R^1O$-($C_1$-$C_6$)-alkyl, $(R^1)_2N$-($C_1$-$C_6$)-alkyl or $R^2(O)_nS$-($C_1$-$C_6$)-alkyl,

m represents 0, 1 or 2,

n represents 0, 1 or 2,

s represents 0, 1, 2 or 3.

**3.** N-(Tetrazol-5-yl)- and N-(triazol-5-yl)arylcarboxylic acid thioamides according to Claim 1 or 2 in which

A represents N or CY,

B represents N or CH,

X represents nitro, halogen, cyano, $(C_1-C_6)$-alkyl, halo-$(C_1-C_6)$-alkyl, $(C_3-C_6)$-cycloalkyl, $OR^1$, $S(O)_nR^2$, $(C_1-C_6)$-alkyl-S(O)n$R^2$, $(C_1-C_6)$-alkyl-$OR^1$, $(C_1-C_6)$-alkyl-$CON(R^1)_2$, $(C_1-C_6)$-alkyl-$SO_2N(R^1)_2$, $(C_1-C_6)$-alkyl-$NR^1COR^1$, $(C_1-C_6)$-alkyl-$NR^1SO_2R^2$, $(C_1-C_6)$-alkylheteroaryl or $(C_1-C_6)$-alkylheterocyclyl, where the two last-mentioned radicals are each substituted by s halogen, $(C_1-C_6)$-alkyl, halo-$(C_1-C_6)$-alkyl, $S(O)_n$-$(C_1-C_6)$-alkyl, $(C_1-C_6)$-alkoxy and halo-$(C_1-C_6)$-alkoxy radicals, and where heterocyclyl carries n oxo groups,

Y represents hydrogen, nitro, halogen, cyano, $(C_1-C_6)$-alkyl, $(C_1-C_6)$-haloalkyl, $OR^1$, $S(O)_nR^2$, $SO_2N(R^1)_2$, $NS(O)R^6R^7$, $S(O)R^8NR^9$, $N(R^1)_2$, $CHNOR^1$, $CH_2ONC(R^3)_2$, $NR^1SO_2R^2$, $NR^1COR^1$, $(C_1-C_6)$-alkyl-$S(O)_nR^2$, $(C_1-C_6)$-alkyl-$OR^1$, $(C_1-C_6)$-alkyl-$CON(R^1)_2$, $(C_1-C_6)$-alkyl-$SO_2N(R^1)_2$, $(C_1-C_6)$-alkyl-$NR^1COR^1$, $(C_1-C_6)$-alkyl-$NR^1SO_2R^2$, $(C_1-C_6)$-alkyl-phenyl, $(C_1-C_6)$-alkyl-heteroaryl, $(C_1-C_6)$-alkyl-heterocyclyl, phenyl, heteroaryl or heterocyclyl, where the six last-mentioned radicals are each substituted by s radicals from the group consisting of halogen, nitro, cyano, $(C_1-C_6)$-alkyl, halo-$(C_1-C_6)$-alkyl, $(C_3-C_6)$-cycloalkyl, $S(O)_n$-$(C_1-C_6)$-alkyl, $(C_1-C_6)$-alkoxy, halo-$(C_1-C_6)$-alkoxy, $(C_1-C_6)$-alkoxy-$(C_1-C_4)$-alkyl and cyanomethyl, and where heterocyclyl carries n oxo groups,

Z represents halogen, cyano, halo-$(C_1-C_6)$-alkyl, $(C_3-C_6)$-cycloalkyl, $S(O)_nR^2$, 1,2,4-triazol-1-yl,

or

Z may also represent hydrogen, methyl, methoxy or ethoxy if Y represents the $S(O)_nR^2$ radical,

W represents hydrogen, methyl, ethyl, methoxymethyl, methoxy, fluorine, chlorine or $S(O)_nCH_3$,

R represents $(C_1-C_8)$-alkyl, halo-$(C_1-C_8)$-alkyl, $(C_2-C_8)$-alkenyl, halo-$(C_2-C_8)$-alkenyl, $(C_2-C_8)$-alkynyl, halo-$(C_2-C_8)$-alkynyl, where these six abovementioned radicals are each substituted by s radicals from the group consisting of cyano, $S(O)_n$-$(C_1-C_6)$-alkyl, $(C_1-C_6)$-alkoxy, halo-$(C_1-C_6)$-alkoxy, $COR^3$, $COOR^3$, $OCOR^3$, $NR^3COR^3$, $NR^3SO_2R^4$, $(C_3-C_6)$-cycloalkyl, heteroaryl, heterocyclyl and phenyl, where the three last-mentioned radicals are each substituted by s radicals from the group consisting of methyl, ethyl, methoxy, trifluoromethyl, cyano and halogen, and where heterocyclyl carries 0 to 2 oxo groups,

or

R represents phenyl which is substituted by s radicals from the group consisting of halogen, nitro, cyano, $(C_1-C_6)$-alkyl, halo-$(C_1-C_6)$-alkyl, $(C_3-C_6)$-cycloalkyl, $S(O)_n$-$(C_1-C_6)$-alkyl, $(C_1-C_6)$-alkoxy, halo-$(C_1-C_6)$-alkoxy and $(C_1-C_6)$-alkoxy-$(C_1-C_4)$-alkyl,

$R^1$ represents hydrogen, $(C_1-C_6)$-alkyl, $(C_2-C_6)$-alkenyl, $(C_2-C_6)$-alkynyl, $(C_3-C_6)$-cycloalkyl, $(C_3-C_6)$-cycloalkyl-$(C_1-C_6)$-alkyl, $(C_1-C_6)$-alkyl-O-$(C_1-C_6)$-alkyl, phenyl, phenyl-$(C_1-C_6)$-alkyl, heteroaryl, $(C_1-C_6)$-alkyl-heteroaryl, heterocyclyl, $(C_1-C_6)$-alkylheterocyclyl, $(C_1-C_6)$-alkyl-O-heteroaryl, $(C_1-C_6)$-alkyl-O-heterocyclyl, $(C_1-C_6)$-alkyl-$NR^3$-heteroaryl or $(C_1-C_6)$-alkyl-$NR^3$-heterocyclyl, where the sixteen last-mentioned radicals are each substituted by s radicals from the group consisting of cyano, halogen, nitro, $OR^3$, $S(O)_nR^4$, $N(R^3)_2$, $NR^3OR^3$, $COR^3$, $OCOR^3$, $NR^3COR^3$, $NR^3SO_2R^4$, $CO_2R^3$, $CON(R^3)_2$ and $(C_1-C_4)$-alkoxy-$(C_2-C_6)$-alkoxycarbonyl, and where heterocyclyl carries n oxo groups,

$R^2$ represents $(C_1-C_6)$-alkyl, $(C_3-C_6)$-cycloalkyl or $(C_3-C_6)$-cycloalkyl-$(C_1-C_6)$-alkyl, each substituted by s radicals from the group consisting of halogen and $OR^3$,

$R^3$ represents hydrogen or $(C_1-C_6)$-alkyl,

$R^4$ represents $(C_1-C_6)$-alkyl,

$R^6$ and $R^7$ independently of one another each represent methyl, ethyl or n-propyl, or

$R^6$ and $R^7$ together with the sulfur atom to which they are attached form a 5- or 6-membered saturated ring which, in addition to the carbon atoms and in addition to the sulfur atom of the sulfoximino group, contains m oxygen atoms,

$R^8$ represents methyl, ethyl or n-propyl,

$R^9$ represents hydrogen or cyano,

m represents 0 or 1,

n represents 0, 1 or 2,

s represents 0, 1, 2 or 3.

4. Herbicidal compositions, **characterized by** a herbicidally effective amount of at least one compound of the formula (I) according to any of Claims 1 to 3.

5. Herbicidal compositions according to Claim 4 in a mixture with formulation auxiliaries.

6. Herbicidal compositions according to Claim 4 or 5, comprising at least one further pesticidally active substance from the group of insecticides, acaricides, herbicides, fungicides, safeners and growth regulators.

7. Herbicidal compositions according to Claim 6, comprising a safener.

**8.** Herbicidal compositions according to Claim 7, comprising cyprosulfamide, cloquintocet-mexyl, mefenpyr-diethyl or isoxadifen-ethyl.

**9.** Herbicidal compositions according to any of Claims 6 to 8, comprising a further herbicide.

**10.** Method for controlling unwanted plants, **characterized in that** an effective amount of at least one compound of the formula (I) according to any of Claims 1 to 3 or of a herbicidal composition according to any of Claims 4 to 9 is applied to the plants or to the site of the unwanted vegetation.

**11.** Use of compounds of the formula (I) according to any of Claims 1 to 3 or of herbicidal compositions according to any of Claims 4 to 9 for controlling unwanted plants.

**12.** Use according to Claim 11, **characterized in that** the compounds of the formula (I) are used for controlling unwanted plants in crops of useful plants.

**13.** Use according to Claim 12, **characterized in that** the useful plants are transgenic useful plants.

## Revendications

**1.** Thioamides de l'acide N-(tétrazol-5-yl)-arylcarboxylique et de l'acide N-(triazol-5-yl)-arylcarboxylique de formule (I) ou leurs sels

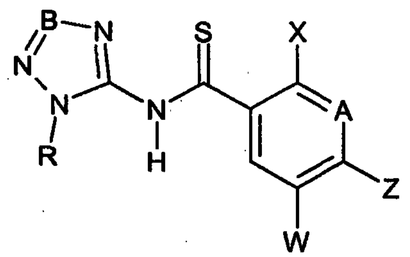
(I),

dans laquelle

A signifie N ou CY,
B signifie N ou CH,
X signifie nitro, halogéno, cyano, formyle, thiocyano, $(C_1-C_6)$ -alkyle, halogéno- $(C_1-C_6)$ -alkyle, $(C_2-C_6)$ -alcényle, halogéno- $(C_2-C_6)$ -alcényle, $(C_2-C_6)$ -alcynyle, halogéno- $(C_3-C_6)$ -alcynyle, $(C_3-C_6)$-cycloalkyle, halogéno-$(C_3-C_6)$ -cycloalkyle, $(C_3-C_6)$-cycloalkyl-$(C_1-C_6)$-alkyle, halogéno-$(C_3-C_6)$-cycloalkyl-$(C_1-C_6)$-alkyle, $COR^1$, $COOR^1$, $OCOOR^1$, $NR^1COOR^1$, $C(O)N(R^1)_2$, $NR^1C(O)N(R^1)_2$, $OC(O)N(R^1)_2$, $C(O)NR^1OR^1$, $OR^1$, $OCOR^1$, $OSO_2R^2$, $S(O)_nR^2$, $SO_2OR^1$, $SO_2N(R^1)_2$, $NR^1SO_2R^2$, $NR^1COR^1$, $(C_1-C_6)$-alkyl-$S(O)_nR^2$, $(C_1-C_6)$-alkyl-$OR^1$, $(C_1-C_6)$-alkyl-$OCOR^1$, $(C_1-C_6)$-alkyl-$OSO_2R^2$, $(C_1-C_6)$ -alkyl-$CO_2R^1$, $(C_1-C_6)$-alkyl-$SO_2OR^1$, $(C_1-C_6)$-alkyl-CON $(R^1)$ $_2$, $(C_1-C_6)$-alkyl-$SO_2N(R^1)_2$, $(C_1-C_6)$-alkyl-$NR^1COR^1$, $(C_1-C_6)$-alkyl-$NR^1SO_2R^2$, $NR_1R_2$, $P(O)(OR^5)_2$, $CH_2P(O)(OR^5)_2$, $(C_1-C_6)$-alkyl-hétéroaryle ou $(C_1-C_6)$-alkyl-hétérocyclyle, les deux derniers radicaux mentionnés étant à chaque fois substitués par s radicaux du groupe constitué par halogéno, $(C_1-C_6)$-alkyle, halogéno-$(C_1-C_6)$-alkyle, S $(O)_n$- $(C_1-C_6)$-alkyle, $(C_1-C_6)$-alcoxy et halogéno-$(C_1-C_6)$-alcoxy, et hétérocyclyle portant n groupes oxo,
Y signifie hydrogène, nitro, halogéno, cyano, thiocyano, $(C_1-C_6)$-alkyle, halogéno- $(C_1-C_6)$ -alkyle, $(C_2-C_6)$-alcényle, halogéno-$(C_2-C_6)$-alcényle, $(C_2-C_6)$-alcynyle, halogéno-$(C_2-C_6)$-alcynyle, $(C_3-C_6)$-cycloalkyle, $(C_3-C_6)$-cycloalcényle, halogéno-$(C_3-C_6)$ -cycloalkyle, $(C_3-C_6)$ -cycloalkyl- $(C_1-C_6)$ -alkyle, halogéno- $(C_3-C_6)$ -cycloalkyl- $(C_1-C_6)$ -alkyle, $COR^1$, $COOR^1$, $OCOOR^1$, $NR^1COOR^1$, $C(O)N(R^1)_2$, $NR^1C(O)N(R^1)_2$, $OC(O)N(R^1)_2$, $CO(NOR^1)R^1$, $CHNOR^1$, $CH_2ONC(R^3)_2$, $NR^1SO_2R^2$, $NR^1COR^1$, $OR^1$, $OSO_2R^2$, $S(O)_nR^2$, $SO_2OR^1$, $SO_2N(R^1)_2$ $(C_1-C_6)$-alkyl-$S(O)nR^2$, $NS(O)R^6R^7$, $S(O)R^8NR^9$, $(C_1-C_6)$-alkyl-$OR^1$, $(C_1-C_6)$-alkyl-$OCOR^1$, $(C_1-C_6)$-alkyl-$OSO_2R^2$, $(C_1-C_6)$-alkyl-$CO_2R^1$, $(C_1-C_6)$-alkyl-CN, $(C_1-C_6)$-alkyl-$SO_2OR^1$, $(C_1-C_6)$-alkyl-$CON(R^1)_2$, $(C_1-C_6)$-alkyl-$SO_2N(R^1)2$, $(C_1-C_6)$-alkyl-$NR^1COR^1$, $(C_1-C_6)$-alkyl-$NR^1SO_2R^2$, $N(R^1)_2$, $P(O)(OR^5)^2$, $CH_2P(O)(OR^5)_2$, $(C_1-C_6)$ -alkyl-phényle, $(C_1-C_6)$ -alkyl-hétéroaryle, $(C_1-C_6)$-alkyl-hétérocyclyle, phényle, hétéroaryle ou hétérocyclyle, les six derniers radicaux mentionnés étant à chaque fois substitués par s radicaux du

groupe constitué par halogéno, nitro, cyano, $(C_1-C_6)$-alkyle, halogéno-$(C_1-C_6)$ -alkyle, $(C_3-C_6)$-cycloalkyle, $S(O)_n$-$(C_1-C_6)$ -alkyle, $(C_1-C_6)$-alcoxy, halogéno-$(C_1-C_6)$-alcoxy, $(C_1-C_6)$-alcoxy-$(C_1-C_4)$-alkyle et cyanométhyle, et hétérocyclyle portant n groupes oxo,

Z signifie halogéno, cyano, nitro, thiocyano, halogéno-$(C_1-C_6)$-alkyle, $(C_2-C_6)$-alcényle, halogéno-$(C_2-C_6)$-alcényle, $(C_2-C_6)$-alcynyle, halogéno-$(C_2-C_6)$-alcynyle, $(C_3-C_6)$-cycloalkyle, halogéno-$(C_3-C_6)$-cycloalkyle, $(C_3-C_6)$ -cycloalkyl- $(C_1-C_6)$ -alkyle, halogéno-$(C_3-C_6)$-cycloalkyl-$(C_1-C_6)$-alkyle, $COR^1$, $COOR^1$, $OCOOR^1$, $NR^1COOR^1$, $C(O)N(R^1)_2$, $NR^1C(O)N(R^1)_2$, $OC(O)N(R^1)_2$, $C(O)NR^1OR^1$, $OSO_2R^2$, $S(O)_nR^2$, $SO_2OR^1$, $SO_2N(R^1)_2$, $NR^1SO_2R^2$, $NR^1COR^1$, $(C_1-C_6)$-alkyl-$S(O)_nR^2$, $(C_1-C_6)$-alkyl-$OR^1$, $(C_1-C_6)$-alkyl-$OCOR^1$, $(C_1-C_6)$-alkyl-$OSO_2R^2$, $(C_1-C_6)$ -alkyl-$CO_2R^1$, $(C_1-C_6)$-alkyl-$SO_2OR^1$, $(C_1-C_6)$-alkyl-$CON(R^1)_2$, $(C_1-C_6)$-alkyl-$SO_2N(R^1)_2$, $(C_1-C_6)$-alkyl-$NR^1COR^1$, $(C_1-C_6)$-alkyl-$NR^1SO_2R^2$, $N(R^1)_2$, $P(O)(OR^5)_2$, hétéroaryle, hétérocyclyle ou phényle, les trois derniers radicaux mentionnés étant à chaque fois substitués par s radicaux du groupe constitué par halogéno, nitro, cyano, $(C_1-C_6)$-alkyle, halogéno-$(C_1-C_6)$-alkyle, $(C_3-C_6)$-cycloalkyle, $S(O)_n$-$(C_1-C_6)$ -alkyle, $(C_1-C_6)$-alcoxy ou halogéno-$(C_1-C_6)$-alcoxy, et hétérocyclyle portant n groupes oxo, ou

Z peut également signifier hydrogène, $(C_1-C_6)$-alkyle ou $(C_1-C_6)$-alcoxy si Y représente le radical $S(O)_nR^2$,

W signifie hydrogène, $(C_1-C_6)$-alkyle, halogéno-$(C_1-C_6)$-alkyle, $(C_2-C_6)$-alcényle, halogéno-$(C_2-C_6)$-alcényle, $(C_2-C_6)$-alcynyle, halogéno-$(C_2-C_6)$-alcynyle, $(C_3-C_7)$-cycloalkyle, $(C_3-C_7)$-halogénocycloalkyle, $(C_1-C_6)$-alcoxy, $(C_1-C_6)$-halogénoalcoxy, $S(O)_n$-$(C_1-C_6)$-alkyle, $S(O)_n$-$(C_1-C_6)$-halogénoalkyle, $(C_1-C_6)$ -alcoxy- $(C_1-C_4)$ -alkyle, $(C_1-C_6)$-alcoxy-$(C_1-C_4)$-halogénoalkyle, halogéno, nitro, $NR^3COR^3$ ou cyano,

R signifie $(C_1-C_8)$-alkyle, halogéno-$(C_1-C_8)$-alkyle, $(C_2-C_8)$-alcényle, halogéno-$(C_2-C_8)$-alcényle, $(C_2-C_8)$-alcynyle ou halogéno-$(C_2-C_8)$-alcynyle, ces six radicaux mentionnés ci-dessus étant à chaque fois substitués par s radicaux du groupe constitué par hydroxy, nitro, cyano, $SiR^5_3$, $PO(OR^5)_2$, $S(O)_n$-$(C_1-C_6)$-alkyle, $S(O)_n$-$(C_1-C_6)$-halogénoalkyle, $(C_1-C_6)$-alcoxy, halogéno-$(C_1-C_6)$-alcoxy, $N(R^3)_2$, $COR^3$, $COOR^3$, $OCOR^3$, $NR^3COR^3$, $NR^3SO_2R^4$, 0 $(C_1-C_2)$-alkyl-$(C_3$-Ce)-cycloalkyle, $(C_3-C_6)$-cycloalkyle, hétéroaryle, hétérocyclyle, phényle, Q-hétéroaryle, Q-hétérocyclyle, Q-phényle et Q-benzyle, les sept derniers radicaux mentionnés étant à chaque fois substitués par s radicaux du groupe constitué par méthyle, éthyle, méthoxy, trifluorométhyle, cyano et halogéno, et hétérocyclyle portant n groupes oxo, ou

R signifie $(C_3-C_7)$-cycloalkyle, hétéroaryle, hétérocyclyle ou phényle à chaque fois substitué par s radicaux du groupe constitué par halogéno, nitro, cyano, $(C_1-C_6)$-alkyle, halogéno-$(C_1-C_6)$-alkyle, $(C_3-C_6)$-cycloalkyle, $S(O)_n$- $(C_1-C_6)$ -alkyle, $(C_1-C_6)$-alcoxy, halogéno-$(C_1-C_6)$-alcoxy et $(C_1-C_6)$-alcoxy-$(C_1-C_4)$-alkyle, hétérocyclyle portant n groupes oxo,

Q signifie 0, S ou $NR^3$,

$R^1$ signifie hydrogène, $(C_1-C_6)$-alkyle, $(C_1-C_6)$-halogénoalkyle, $(C_2-C_6)$-alcényle, $(C_2-C_6)$-halogénoalcényle, $(C_2-C_6)$-alcynyle, $(C_2-C_6)$-halogénoalcynyle, $(C_3-C_6)$-cycloalkyle, $(C_3-C_6)$-cycloalcényle, $(C_3-C_6)$-halogénocycloalkyle, $(C_1-C_6)$-alkyl-O- $(C_1-C_6)$ -alkyle, $(C_3-C_6)$ -cycloalkyl- $(C_1-C_6)$-alkyle, phényle, phényl-$(C_1-C_6)$-alkyle, hétéroaryle, $(C_1-C_6)$-alkyl-hétéroaryle, hétérocyclyle, $(C_1-C_6)$ -alkyl-hétérocyclyle, $(C_1-C_6)$-alkyl-O-hétéroaryle, $(C_1-C_6)$-alkyl-O-hétérocyclyle, $(C_1-C_6)$-alkyl-$NR^3$-hétéroaryle ou $(C_1-C_6)$-alkyl-$NR^3$-hétérocyclyle, les 21 derniers radicaux mentionnés étant à chaque fois substitués par s radicaux du groupe constitué par cyano, halogéno, nitro, thiocyano, $OR^3$, $S(O)_nR^4$, $N(R^3)_2$, $NR^3OR^3$, $COR^3$, $OCOR^3$, $SCOR^4$, $NR^3COR^3$, $NR^3SO_2R^4$, $CO_2R^3$, $COSR^4$, $CON(R^3)_2$ et $(C_1-C_4)$ -alcoxy- $(C_2-C_6)$ -alcoxycarbonyle, et hétérocyclyle portant n groupes oxo,

$R^2$ signifie $(C_1-C_6)$-alkyle, $(C_1-C_6)$-halogénoalkyle, $(C_2-C_6)$-alcényle, $(C_2-C_6)$-halogénoalcényle, $(C_2-C_6)$-alcynyle, $(C_2-C_6)$-halogénoalcynyle, $(C_3-C_6)$-cycloalkyle, $(C_3-C_6)$-cycloalcényle, $(C_3-C_6)$-halogénocycloalkyle, $(C_1-C_6)$ -alkyl-O- $(C_1-C_6)$ -alkyle, $(C_3-C_6)$-cycloalkyl-$(C_1-C_6)$-alkyle, phényle, phényl-$(C_1-C_6)$-alkyle, hétéroaryle, $(C_1-C_6)$-alkyl-hétéroaryle, hétérocyclyle, $(C_1-C_6)$-alkyl-hétérocyclyle, $(C_1-C_6)$ -alkyl-O-hétéroaryle, $(C_1-C_6)$-alkyl-O-hétérocyclyle, $(C_1-C_{6*})$-alkyl-$NR^3$-hétéroaryle ou $(C_1-C_6)$-alkyl-$NR^3$-hétérocyclyle, les 21 derniers radicaux mentionnés étant à chaque fois substitués par s radicaux du groupe cyano, halogéno, nitro, thiocyano, $OR^3$, $S(O)_nR^4$, $N(R^3)_2$, $NR^3OR^3$, $COR^3$, $OCOR^3$, $SCOR^4$, $NR^3COR^3$, $NR^3SO_2R^4$, $CO_2R^3$, $COSR^4$, $CON(R^3)_2$ et $(C_1-C_4)$-alcoxy-$(C_2-C_6)$-alcoxycarbonyle, et hétérocyclyle portant n groupes oxo,

$R^3$ signifie hydrogène, $(C_1-C_6)$-alkyle, $(C_2-C_6)$-alcényle, $(C_2-C_6)$-alcynyle, $(C_3-C_6)$-cycloalkyle, $(C_3-C_6)$-cycloalkyl-$(C_1-C_6)$-alkyle ou phényle,

$R^4$ signifie $(C_1-C_6)$-alkyle, $(C_2-C_6)$-alcényle, $(C_2-C_6)$-alcynyle, $(C_3-C_6)$-cycloalkyle, $(C_3-C_6)$-cycloalkyl-$(C_1-C_6)$-alkyle ou phényle,

$R^5$ signifie $(C_1-C_4)$-alkyle,

$R^6$ et $R^7$ signifient, indépendamment l'un de l'autre, à chaque fois $(C_1-C_6)$-alkyle, halogéno-$(C_1-C_6)$-alkyle, $(C_2-C_6)$-alcényle, halogéno-$(C_2-C_6)$-alcényle, $(C_2-C_6)$-alcynyle, halogéno-$(C_3-C_6)$-alcynyle, $(C_3-C_6)$-cycloalkyle, halogéno-$(C_3-C_6)$-cycloalkyle, $(C_3-C_6)$-cycloalkyl- $(C_1-C_6)$-alkyle, halogéno-$(C_3-C_6)$-cycloalkyl- $(C_1-C_6)$ -alkyle, $(C_1-C_6)$ -alcoxy- $(C_1-C_6)$-alkyle, halogéno-$(C_1-C_6)$-alcoxy-$(C_1-C_6)$-alkyle, phényle, hétéroaryle ou hétérocyclyle, les trois derniers radicaux mentionnés étant à chaque fois substitués par s radicaux du groupe constitué par nitro, halogéno, cyano, thiocyano, $(C_1-C_6)$-alkyle, halogéno- $(C_1-C_6)$ -alkyle, $(C_3-C_6)$-cycloalkyle, $R^1O(O)C$,

$(R^1)_2N(O)C$, $R^1O$, $(R^1)_2N$, $R^2(O)_nS$, $R^1O(O)_2S$, $(R^1)_2N(O)_2S$ et $R^1O$-($C_1$-$C_6$)-alkyle ; et hétérocyclyle portant n groupes oxo, ou

$R^6$ et $R^7$ forment, ensemble avec l'atome de soufre auquel ils sont liés, un cycle de 3 à 8 chaînons, insaturé, partiellement saturé ou saturé qui contient, outre les atomes de carbone et outre l'atome de soufre du groupe sulfoximino, à chaque fois m chaînons de cycle du groupe constitué par N($R^1$), 0 et $S(O)_n$ ; et ce cycle étant substitué à chaque fois par s radicaux du groupe constitué par nitro, halogéno, cyano, thiocyano, ($C_1$-$C_6$)-alkyle, halogéno- ($C_1$-$C_6$) -alkyle, ($C_3$-$C_6$)-cycloalkyle, $R^1O(O)C$, $(R^1)_2N(O)C$, $R^1O$, $(R^1)_2N$, $R^2(O)_nS$, $R^1O(O)_2S$, $(R^1)_2N(O)_2S$ et $R^1O$-($C_1$-$C_6$)-alkyle ; et ce cycle portant n groupes oxo,

$R^8$ signifie ($C_1$-$C_6$)-alkyle, ($C_2$-$C_6$)-alcényle ou ($C_2$-$C_6$)-alcynyle à chaque fois substitué par s radicaux du groupe constitué par nitro, halogéno, cyano, thiocyano, ($C_3$-$C_6$)-cycloalkyle, $R^1(O)C$, $R^1(R^1ON=)C$, $R^1O(O)C$, $(R^1)_2N(O)C$, $R^1(R^1O)N(O)C$, $R^2(O)_2S(R^1)N(O)C$, $R^1O(O)_2S(R^1))N(O)C$, $(R^1)_2N(O)_2S(R^1)N(O)C$, $R^1S(O)C$, $R^1O$, $R^1(O)CO$, $R^2(O)_2SO$, $R^2O(O)CO$, $(R^1)_2N(O)CO$, $(R^1)_2N$, $R^1O(R^1)N$, $R^1(O)C(R^1)N$, $R^2(O)_2S(R^1)N$, $R^2O(O)C(R^1)N$, $(R^1)_2N(O)C(R^1)N$, $R^{10}(O)_2S(R^1)N$, $(R^1)_2N(O)_2S(R^1)N$, $R^2(O)_nS$, $R^1C(O)S$, $R^1O(O)_2S$, $(R^1)_2N(O)_2S$, $R^1(O)C(R^1)N(O)_2S$, $R^2O(O)C(R^1)N(O)_2S$, $(R^1)_2N(O)C(R^1)N(O)_2S$ et $(R^5O)_2(O)P$ ; ou ($C_3$-$C_6$)-cycloalkyle, ($C_3$-$C_6$)-cycloalcényle, phényle, phényl-($C_1$-$C_6$)-alkyle, hétéroaryle, hétéroaryl-($C_1$-$C_6$)-alkyle, hétérocyclyle, hétérocyclyl-($C_1$-$C_6$)-alkyle, phényl-O-($C_1$-$C_6$)-alkyle, hétéroaryl-O-($C_1$-$C_6$)-alkyle, hétérocyclyl-O-($C_1$-$C_6$)-alkyle, phényl-N($R^1$)-($C_1$-$C_6$)-alkyle, hétéroaryl-N($R^1$)-($C_1$-$C_6$)-alkyle, hétérocyclyl-N-($R^1$)-($C_1$-$C_6$)-alkyle, phényl-$S(O)_n$-($C_1$-$C_6$)-alkyle, hétéroaryl-$S(O)_n$-($C_1$-$C_6$)-alkyle ou hétérocyclyl-$S(O)_n$-($C_1$-$C_6$)-alkyle à chaque fois substitué dans la partie cyclique par s radicaux du groupe constitué par nitro, halogéno, cyano, thiocyano, ($C_1$-$C_6$)-alkyle, halogéno- ($C_1$-$C_6$) -alkyle, ($C_3$-$C_6$) -cycloalkyle, $R^1(O)C$, $R^1(R^1ON=)C$, $R^1O(O)C$, $(R^1)_2N(O)C$, $R^1(R^1O)N(O)C$, $R^2(O)_2S(R^1)N(O)C$, $R^1O(O)_2S(R^1)N(O)C$, $(R^1)_2N(O)_2S(R^1)N(O)C$, $R^1S(O)C$, $R^1O$, $R^1(O)CO$, $R^2(O)_2SO$, $R^2O(O)CO$, $(R^1)_2N(O)CO$, $(R^1)_2N$, $R^1O(R^1)N$, $R^1(O)C(R^1)N$, $R^2(O)_2S(R^1)N$, $R^2O(O)C(R^1)N$, $(R^1)_2N(O)C(R^1)N$, $R^1O(O)_2S(R^1)N$, $(R^1)_2N(O)_2S(R^1)N$, $R^2(O)_nS$, $R^1C(O)S$, $R^1O(O)_2S$, $(R^1)_2N(O)_2S$, $R^1(O)C(R^1)N(O)_2S$, $R^2O(O)C(R^1)N(O)_2S$, $(R^1)_2N(O)C(R^1)N(O)_2S$, $(R^5O)_2(O)P$ et $R^1O$-($C_1$-$C_6$)-alkyle, et hétérocyclyle portant n groupes oxo,

$R^9$ signifie hydrogène, nitro, halogéno, cyano, ($C_1$-$C_6$)-alkyle, halogéno-($C_1$-$C_6$)-alkyle, ($C_3$-$C_6$)-alcényle, halogéno- ($C_3$-$C_6$) -alcényle, ($C_2$-$C_6$)-alcynyle, halogéno- ($C_3$-$C_6$) -alcynyle, ($C_3$-$C_6$)-cycloalkyle, halogéno-($C_3$-$C_6$) -cycloalkyle, ($C_3$-$C_6$)-cycloalkyl-($C_1$-$C_6$)-alkyle, halogéno-($C_3$-$C_6$)-cycloalkyl-($C_1$-$C_6$)-alkyle, $R^1(O)C$, $R^2O(O)C$, $(R^1)_2N(O)C$, $R^2S(O)C$, $(R^1)_2N(S)C$, $R^1(R^1O)N(O)C$, $R^2(O)_2S(R^1)N(O)C$, $(R^1)_2N(O)_2S(R^1)N(O)C$, $R^1O$, $(R^1)_2N$, $R^2(O)_nS$, $(R^2)_3Si$-($C_1$-$C_6$)-alkyl-$(O)_nS$, $R^1O(O)_2S$, $(R^1)_2N(O)_2S$, $R^1(O)C(R^1)N(O)_2S$, $R^2O$ (0) C ($R^1$) N (O) $_2S$, $(R^1)_2N(O)C(R^1)N(O)_2S$, $R^2(O)_2S(R^1)N(O)_2S$, $(R^5O)(O)P$, $(R^2)_3Si$, $R^1(O)C$-($C_1$-$C_6$)-alkyle, $R^1O(O)C$-($C_1$-$C_6$)-alkyle, $(R^1)_2N(O)C$- ($C_1$-$C_6$) -alkyle, $(R^1O)(R^1)N(O)C$-($C_1$-$C_6$)-alkyle, $R^2(O)_2S(R^1)N(O)C$-($C_1$-$C_6$)-alkyle, $R^1O(O)_2S(R^1)N(O)C$-($C_1$-$C_6$)-alkyle, $(R^1)_2N(O)_2S(R^1)N(O)C$-($C_1$-$C_6$)-alkyle, $R^1O$-($C_1$-$C_6$)-alkyle, $R^1(O)CO$-($C_1$-$C_6$)-alkyle, $R^2(O)_2SO$-($C_1$-$C_6$)-alkyle, $R^2O(O)CO$-($C_1$-$C_6$)-alkyle, $(R^1)_2N(O)CO$-($C_1$-$C_6$)-alkyle, $(R^1)_2N$-($C_1$-$C_6$)-alkyle, $R^1(O)C(R^1)N$-($C_1$-$C_6$)-alkyle, $R^2(O)_2S(R^1)N$-($C_1$-$C_6$) -alkyle, $R^2O(O)C(R^1)N$-($C_1$-$C_6$)-alkyle, $(R^1)_2N(O)C(R^1)N$-($C_1$-$C_6$) -alkyle, $R^1O(O)_2S$ ($R^1$)N-($C_1$-$C_6$)-alkyle, $(R^1)_2N(O)_2S(R^1)N$-($C_1$-$C_6$)-alkyle, $R^2(O)_nS$-($C_1$-$C_6$)-alkyle, $R^1O(O)_2S$-($C_1$-$C_6$)-alkyle, $(R^1)_2N(O)_2S$-($C_1$-$C_6$) -alkyle, $R^1(O)C(R^1)N(O)_2S$-($C_1$-$C_6$)-alkyle, $R^2O(O)C(R^1)N(O)_2S$-($C_1$-$C_6$)-alkyle, $(R^1)_2N(O)C(R^1)N(O)_2S$-($C_1$-$C_6$)-alkyle, $(R^5O)_2$ (O) P- ($C_1$-$C_6$)-alkyle, $(R^2)_3Si$-($C_1$-$C_6$)-alkyle ; ou phényle, hétéroaryle, hétérocyclyle, phényl-($C_1$-$C_6$)-alkyle, hétéroaryl-($C_1$-$C_6$)-alkyle ou hétérocyclyl-($C_1$-$C_6$)-alkyle à chaque fois substitué dans la partie cyclique par s radicaux du groupe constitué par nitro, halogéno, cyano, thiocyano, ($C_1$-$C_6$)-alkyle, halogéno- ($C_1$-$C_6$)-alkyle, ($C_3$-$C_6$) -cycloalkyle, $R^1O(O)C$, $(R^1)_2N(O)C$, $R^1O$, $(R^1)_2N$, $R^2(O)_nS$, $R^1O(O)_2S$, $(R^1)_2N(O)_2S$ et $R^1O$-($C_1$-$C_6$)-alkyle, et hétérocyclyle portant n groupes oxo,

m vaut 0, 1, 2, 3 ou 4,

n vaut 0, 1 ou 2,

s vaut 0, 1, 2 ou 3.

**2.** Thioamides de l'acide N-(tétrazol-5-yl)-arylcarboxylique et de l'acide N-(triazol-5-yl)-arylcarboxylique selon la revendication 1, dans lesquels

A signifie N ou CY,

B signifie N ou CH,

X signifie nitro, halogéno, cyano, thiocyano, ($C_1$-$C_6$)-alkyle, halogéno- ($C_1$-$C_6$) -alkyle, ($C_2$-$C_6$)-alcényle, halogéno- ($C_2$-$C_6$) -alcényle, ($C_2$-$C_6$)-alcynyle, halogéno- ($C_3$-$C_6$) -alcynyle, ($C_3$-$C_6$)-cycloalkyle, halogéno- ($C_3$-$C_6$) -cycloalkyle, ($C_1$-$C_6$)-alkyl-O-($C_1$-$C_6$) -alkyle, ($C_3$-$C_6$)-cycloalkyl-($C_1$-$C_6$)-alkyle, halogéno-($C_3$-$C_6$)-cycloalkyl-($C_1$-$C_6$)-alkyle, $COR^1$, $OR^1$, $OCOR^1$, $OSO_2R^2$, $S(O)_nR^2$, $SO_2OR^1$, $SO_2N(R^1)_2$, $NR^1SO_2R^2$, $NR^1COR^1$, ($C_1$-$C_6$)-alkyl-$S(O)_nR^2$, ($C_1$-$C_6$)-alkyl-$OR^1$, ($C_1$-$C_6$)-alkyl-$OCOR^1$, ($C_1$-$C_6$)-alkyl-$OSO_2R^2$, ($C_1$-$C_6$) -alkyl-$CO_2R^1$, ($C_1$-$C_6$) -alkyl-$SO_2OR^1$, ($C_1$-$C_6$)-alkyl-$CON(R^1)_2$, ($C_1$-$C_6$)-alkyl-$SO_2N(R^1)_2$, ($C_1$-$C_6$)-alkyl-$NR^1COR^1$ ou

$(C_1-C_6)$-alkyl-$NR^1SO_2R^2$, $(C_1-C_6)$-alkyl-hétéroaryle ou $(C_1-C_6)$-alkyl-hétérocyclyle, les deux derniers radicaux mentionnés étant à chaque fois substitués par s radicaux du groupe constitué par halogéno, $(C_1-C_6)$-alkyle, halogéno-$(C_1-C_6)$-alkyle, $S(O)_n$-$(C_1-C_6)$ -alkyle, $(C_1-C_6)$-alcoxy et halogéno-$(C_1-C_6)$-alcoxy, et hétérocyclyle portant n groupes oxo,

Y signifie hydrogène, nitro, halogéno, cyano, thiocyano, $(C_1-C_6)$-alkyle, halogéno- $(C_1-C_6)$ -alkyle, $(C_2-C_6)$-alcényle, halogéno-$(C_2-C_6)$-alcényle, $(C_2-C_6)$-alcynyle, halogéno-$(C_3-C_6)$-alcynyle, $(C_3-C_6)$-cycloalkyle, $(C_3-C_6)$-cycloalcényle, halogéno-$(C_3-C_6)$-cycloalkyle, $(C_3-C_6)$ -cycloalkyl-$(C_1-C_6)$-alkyle, halogéno- $(C_3-C_6)$ -cycloalkyl-$(C_1-C_6)$-alkyle, $COR^1$, $OR^1$, $COOR^1$, $CHNOR^1$, $CH_2ONC(R^3)_2$, $OSO_2R^2$, $S(O)_nR^2$, $SO_2OR^1$, $SO_2N(R^1)_2$, $NS(O)R^6R^7$, $S(O)R^8NR^9$, $N(R^1)_2$, $NR^1SO_2R^2$, $NR^1COR^1$, $(C_1-C_6)$-alkyl-$S(O)_nR^2$, $(C_1-C_6)$-alkyl-$OR^1$, $(C_1-C_6)$ -alkyl-$OCOR^1$, $(C_1-C_6)$-alkyl-$OSO_2R^2$, $(C_1-C_6)$-alkyl-$CO_2R^1$, $(C_1-C_6)$-alkyl-$SO_2OR^1$, $(C_1-C_6)$-alkyl-$CON(R^1)_2$, $(C_1-C_6)$-alkyl-$SO_2N(R^1)_2$, $(C_1-C_6)$-alkyl-$NR^1COR^1$, $(C_1-C_6)$ -alkyl-$NR^1SO_2R^2$, $(C_1-C_6)$-alkyl-phényle, $(C_1-C_6)$-alkyl-hétéroaryle, $(C_1-C_6)$-alkyl-hétérocyclyle, phényle, hétéroaryle ou hétérocyclyle, les six derniers radicaux mentionnés étant à chaque fois substitués par s radicaux du groupe constitué par halogéno, nitro, cyano, $(C_1-C_6)$-alkyle, halogéno-$(C_1-C_6)$-alkyle, $(C_3-C_6)$-cycloalkyle, $S(O)_n$-$(C_1-C_6)$-alkyle, $(C_1-C_6)$-alcoxy, halogéno-$(C_1-C_6)$-alcoxy, $(C_1-C_6)$-alcoxy-$(C_1-C_4)$-alkyle et cyanométhyle, et hétérocyclyle portant n groupes oxo,

Z signifie halogéno, cyano, nitro, thiocyano, halogéno-$(C_1-C_6)$-alkyle, $(C_2-C_6)$-alcényle, halogéno-$(C_2-C_6)$-alcényle, $(C_2-C_6)$-alcynyle, halogéno-$(C_3-C_6)$-alcynyle, $(C_3-C_6)$-cycloalkyle, halogéno-$(C_3-C_6)$-cycloalkyle, $(C_3-C_6)$ -cycloalkyl-$(C_1-C_6)$-alkyle, halogéno- $(C_3-C_6)$ -cycloalkyl-$(C_1-C_6)$-alkyle, $COR^1$, $COOR^1$, $C(O)N(R^1)_2$, $C(O)NR^1OR^1$, $OSO_2R^2$, $S(O)_nR^2$, $SO_2OR^1$, $SO_2N(R^1)_2$, $NR^1SO_2R^2$, $NR^1COR^1$, $(C_1-C_6)$-alkyl-$S(O)_nR^2$, $(C_1-C_6)$-alkyl-$OR^1$, $(C_1-C_6)$-alkyl-$OCOR^1$, $(C_1-C_6)$ -alkyl-$OSO_2R^2$, $(C_1-C_6)$ -alkyl-$CO_2R^1$, $(C_1-C_6)$-alkyl-$SO_2OR^1$, $(C_1-C_6)$-alkyl-$CON(R^1)_2$, $(C_1-C_6)$-alkyl-$SO_2N(R^1)_2$, $(C_1-C_6)$ -alkyl-$NR^1COR^1$, $(C_1-C_6)$-alkyl-$NR^1SO_2R^2$, 1,2,4-triazol-1-yle, ou

Z peut également signifier hydrogène, $(C_1-C_6)$-alkyle ou $(C_1-C_6)$-alcoxy si Y représente le radical $S(O)_nR^2$,

W signifie hydrogène, $(C_1-C_6)$-alkyle, halogéno-$(C_1-C_6)$-alkyle, $(C_1-C_6)$-alcoxy, $(C_1-C_6)$-halogénoalcoxy, $S(O)_n$-$(C_1-C_6)$-alkyle, $S(O)_n$-$(C_1-C_6)$-halogénoalkyle, $(C_1-C_6)$-alcoxy-$(C_1-C_4)$-alkyle, halogéno, nitro ou cyano,

R signifie $(C_1-C_8)$-alkyle, halogéno-$(C_1-C_8)$-alkyle, $(C_2-C_8)$-alcényle, halogéno-$(C_2-C_{8)}$-alcényle, $(C_2-C_8)$-alcynyle, halogéno-$(C_2-C_8)$-alcynyle, ces six radicaux mentionnés ci-dessus étant à chaque fois substitués par s radicaux du groupe constitué par nitro, cyano, $SiR^5_3$, $P(OR^5)_3$, $S(O)_n$-$(C_1-C_6)$-alkyle, $(C_1-C_6)$-alcoxy, halogéno-$(C_1-C_6)$-alcoxy, $N(R^3)_2$, $COR^3$, $COOR^3$, $OCOR^3$, $NR^3COR^3$, $NR^3SO_2R^4$, $(C_3-C_6)$-cycloalkyle, hétéroaryle, hétérocyclyle, phényle, Q-hétéroaryle, Q-hétérocyclyle, Q-phényle et Q-benzyle, les sept derniers radicaux mentionnés étant à chaque fois substitués par s radicaux du groupe constitué par méthyle, éthyle, méthoxy, trifluorométhyle, cyano et halogéno, et hétérocyclyle portant n groupes oxo, ou

R signifie $(C_3-C_7)$-cycloalkyle, hétéroaryle, hétérocyclyle ou phényle à chaque fois substitué par s radicaux du groupe constitué par halogéno, nitro, cyano, $(C_1-C_6)$-alkyle, halogéno-$(C_1-C_6)$-alkyle, $(C_3-C_6)$-cycloalkyle, $S(O)_n$-$(C_1-C_6)$-alkyle, $(C_1-C_6)$-alcoxy, halogéno-$(C_1-C_6)$-alcoxy et $(C_1-C_6)$-alcoxy-$(C_1-C_4)$-alkyle,

Q signifie O, S ou $NR^3$,

$R^1$ signifie hydrogène, $(C_1-C_6)$-alkyle, $(C_2-C_6)$-alcényle, $(C_2-C_6)$-alcynyle, $(C_3-C_6)$-cycloalkyle, $(C_3-C_6)$ -cycloalkyl-$(C_1-C_6)$-alkyle, $(C_1-C_6)$-alkyl-O-$(C_1-C_6)$-alkyle, phényle, phényl-$(C_1-C_6)$-alkyle, hétéroaryle, $(C_1-C_6)$-alkyl-hétéroaryle, hétérocyclyle, $(C_1-C_6)$ -alkyl-hétérocyclyle, $(C_1-C_6)$-alkyl-O-hétéroaryle, $(C_1-C_6)$-alkyl-O-hétérocyclyle, $(C_1-C_6)$-alkyl-$NR^3$-hétéroaryle ou $(C_1-C_6)$-alkyl-$NR^3$-hétérocyclyle, les seize derniers radicaux mentionnés étant à chaque fois substitués par s radicaux du groupe constitué par cyano, halogéno, nitro, $OR^3$, $S(O)_nR^4$, $N(R^3)_2$, $NR^3OR^3$, $COR^3$, $OCOR^3$, $NR^3COR^3$, $NR^3SO_2R^4$, $CO_2R^3$, $CON(R^3)_2$ et $(C_1-C_4)$-alcoxy-$(C_2-C_6)$-alcoxycarbonyle, et hétérocyclyle portant n groupes oxo,

$R^2$ signifie $(C_1-C_6)$-alkyle, $(C_2-C_6)$-alcényle, $(C_2-C_6)$-alcynyle, $(C_3-C_6)$-cycloalkyle, $(C_3-C_6)$-cycloalkyl-$(C_1-C_6)$-alkyle, $(C_1-C_6)$ -alkyl-O- $(C_1-C_6)$ -alkyle, phényle, phényl-$(C_1-C_6)$-alkyle, hétéroaryle, $(C_1-C_6)$ -alkyl-hétéroaryle, hétérocyclyle, $(C_1-C_6)$-alkyl-hétérocyclyle, $(C_1-C_6)$-alkyl-O-hétéroaryle, $(C_1-C_6)$ -alkyl-O-hétérocyclyle, $(C_1-C_6)$ -alkyl-$NR^3$-hétéroaryle ou $(C_1-C_6)$-alkyl-$NR^3$-hétérocyclyle, ces seize derniers radicaux mentionnés étant à chaque fois substitués par s radicaux du groupe constitué par cyano, halogéno, nitro, $OR^3$, $S(O)_nR^4$, $N(R^3)_2$, $NR^3OR^3$, $NR^3SO_2R^4$, $COR^3$, $OCOR^3$, $NR^3COR^3$, $CO_2R^3$, $CON(R^3)_2$ et $(C_1-C_4)$-alcoxy-$(C_2-C_6)$-alcoxycarbonyle, et hétérocyclyle portant n groupes oxo,

$R^3$ signifie hydrogène, $(C_1-C_6)$-alkyle, $(C_2-C_6)$-alcényle, $(C_2-C_6)$-alcynyle, $(C_3-C_6)$-cycloalkyle ou $(C_3-C_6)$-cycloalkyl-$(C_1-C_6)$-alkyle,

$R^4$ signifie $(C_1-C_6)$-alkyle, $(C_2-C_6)$-alcényle ou $(C_2-C_6)$-alcynyle,

$R^5$ signifie méthyle ou éthyle,

$R^6$ et $R^7$ signifient, indépendamment l'un de l'autre, à chaque fois $(C_1-C_6)$-alkyle, halogéno-$(C_1-C_6)$-alkyle, $(C_3-C_6)$-cycloalkyle, halogéno-$(C_3-C_6)$-cycloalkyle, $(C_3-C_6)$-cycloalkyl-$(C_1-C_6)$-alkyle, halogéno-$(C_3-C_6)$-cycloalkyl- $(C_1-C_6)$ -alkyle, $(C_1-C_6)$-alcoxy-$(C_1-C_6)$-alkyle, halogéno-$(C_1-C_6)$-alcoxy-$(C_1-C_6)$-alkyle, phényle, hé-

téroaryle ou hétérocyclyle, les trois derniers radicaux mentionnés étant à chaque fois substitués par s radicaux du groupe constitué par nitro, halogéno, ($C_1$-$C_6$)-alkyle, halogéno-($C_1$-$C_6$)-alkyle, $R^1O(O)C$, $(R^1)_2N(O)C$, $R^1O$, $(R^1)_2N$, $R^2(O)_nS$ et $R^1O$-($C_1$-$C_6$)-alkyle, et hétérocyclyle portant n groupes oxo, ou

$R^6$ et $R^7$ forment, ensemble avec l'atome de soufre auquel ils sont liés, un cycle de 3 à 8 chaînons, insaturé, partiellement saturé ou saturé qui contient, outre les atomes de carbone et outre l'atome de soufre du groupe sulfoximino, à chaque fois m chaînons de cycle du groupe constitué par $N(R^1)$, O et $S(O)_n$ ; et ce cycle étant substitué à chaque fois par s radicaux du groupe constitué par halogéno, ($C_1$-$C_6$)-alkyle, halogéno- ($C_1$-$C_6$) -alkyle, $R^1O(O)C$, $(R^1)_2N(O)C$, $R^1O$, $(R^1)_2N$, $R^2(O)_nS$, $R^1O(O)_2S$, $(R^1)_2N(O)_2S$ et $R^1O$-($C_1$-$C_6$)-alkyle ; et ce cycle portant n groupes oxo,

$R^8$ signifie ($C_1$-$C_6$)-alkyle à chaque fois substitué par s radicaux du groupe halogéno, cyano, ($C_3$-$C_6$)-cycloalkyle, $R^1(O)C$, $R^1(R^1ON{=})C$, $R^1O(O)C$, $(R^1)_2N(O)C$, $R^2(O)_2S(R^1)N(O)C$, $R^1O$, $(R^1)_2N$, $R^1(O)C(R^1)N$, $R^2(O)_2S(R^1)N$, $R^2O(O)C(R^1)N$, $(R^1)_2N(O)C(R^1)N$, $R^2(O)_nS$, $R^1O(O)_2S$, $(R^1)_2N(O)_2S$, $R^1(O)C(R^1)N(O)_2S$, $R^2O(O)C(R^1)N(O)_2S$ et $(R^1)_2N(O)C(R^1)N(O)_2S$ ; ou ($C_3$-$C_6$)-cycloalkyle à chaque fois substitué par s radicaux du groupe constitué par halogéno, ($C_1$-$C_6$)-alkyle, halogéno-($C_1$-$C_6$)-alkyle, ($C_3$-$C_6$)-cycloalkyle, $R^1O(O)C$ et $(R^1)_2N(O)C$,

$R^9$ signifie hydrogène, nitro, cyano, ($C_1$-$C_6$)-alkyle, halogéno-($C_1$-$C_6$)-alkyle, ($C_3$-$C_6$)-cycloalkyle, halogéno-($C_3$-$C_6$) -cycloalkyle, ($C_3$-$C_6$)-cycloalkyl-($C_1$-$C_6$)-alkyle, halogéno- ($C_3$-$C_6$) -cycloalkyl-($C_1$-$C_6$)-alkyle, $R^1(O)C$, $R^2O(O)C$, $(R^1)_2N(O)C$, $R^2(O)_2S$, $R^1(O)C$-($C_1$-$C_6$)-alkyle, $R^1O(O)C$-($C_1$-$C_6$) -alkyle, $(R^1)_2N(O)C$-($C_1$-$C_6$)-alkyle, $R^1O$-($C_1$-$C_6$) -alkyle, $(R^1)_2N$-($C_1$-$C_6$)-alkyle ou $R^2(O)_nS$-($C_1$-$C_6$)-alkyle,

m vaut 0, 1 ou 2,

n vaut 0, 1 ou 2,

s vaut 0, 1, 2 ou 3.

**3.** Thioamides de l'acide N-(tétrazol-5-yl)-arylcarboxylique et de l'acide N-(triazol-5-yl)-arylcarboxylique selon la revendication 1 ou 2, dans lesquels

A signifie N ou CY,

B signifie N ou CH,

X signifie nitro, halogéno, cyano, ($C_1$-$C_6$)-alkyle, halogéno-($C_1$-$C_6$)-alkyle, ($C_3$-$C_6$)-cycloalkyle, $OR^1$, $S(O)_nR^2$, ($C_1$-$C_6$)-alkyl-$S(O)_nR^2$, ($C_1$-$C_6$)-alkyl-$OR^1$, ($C_1$-$C_6$)-alkyl-$CON(R^1)_2$, ($C_1$-$C_6$)-alkyl-$SO_2N(R^1)_2$, ($C_1$-$C_6$)-alkyl-$NR^1COR^1$, ($C_1$-$C_6$)-alkyl-$NR^1SO_2R^2$, ($C_1$-$C_6$)-alkyl-hétéroaryle ou ($C_1$-$C_6$)-alkyl-hétérocyclyle, les deux derniers radicaux mentionnés étant à chaque fois substitués par s radicaux halogéno, ($C_1$-$C_6$)-alkyle, halogéno-($C_1$-$C_6$)-alkyle, $S(O)_n$-($C_1$-$C_6$)-alkyle, ($C_1$-$C_6$)-alcoxy et halogéno-($C_1$-$C_6$)-alcoxy, et hétérocyclyle portant n groupes oxo,

Y signifie hydrogène, nitro, halogéno, cyano, ($C_1$-$C_6$)-alkyle, ($C_1$-$C_6$)-halogénoalkyle, $OR^1$, $S(O)_nR^2$, $SO_2N(R^1)_2$, $NS(O)R^6R^7$, $S(O)R^8NR^9$, $N(R^1)_2$, $CHNOR^1$, $CH_2ONC(R^3)_2$, $NR^1SO_2R^2$, $NR^1COR^1$, ($C_1$-$C_6$)-alkyl-$S(O)_nR^2$, ($C_1$-$C_6$)-alkyl-$OR^1$, ($C_1$-$C_6$)-alkyl-$CON(R^1)_2$, ($C_1$-$C_6$)-alkyl-$SO_2N(R^1)_2$, ($C_1$-$C_6$)-alkyl-$NR^1COR^1$, ($C_1$-$C_6$)-alkyl-$NR^1SO_2R^2$, ($C_1$-$C_6$)-alkyl-phényle, ($C_1$-$C_6$)-alkyl-hétéroaryle, ($C_1$-$C_6$)-alkyl-hétérocyclyle, phényle, hétéroaryle ou hétérocyclyle, les six derniers radicaux mentionnés étant à chaque fois substitués par s radicaux du groupe constitué par halogéno, nitro, cyano, ($C_1$-$C_6$)-alkyle, halogéno-($C_1$-$C_6$)-alkyle, ($C_3$-$C_6$)-cycloalkyle, $S(O)_n$-($C_1$-$C_6$)-alkyle, ($C_1$-$C_6$)-alcoxy, halogéno-($C_1$-$C_6$)-alcoxy, ($C_1$-$C_6$)-alcoxy-($C_1$-$C_4$)-alkyle et cyanométhyle, et hétérocyclyle portant n groupes oxo,

Z signifie halogéno, cyano, halogéno-($C_1$-$C_6$)-alkyle, ($C_3$-$C_6$)-cycloalkyle, $S(O)_nR^2$, 1,2,4-triazol-1-yle, ou

Z peut également signifier hydrogène, méthyle, méthoxy ou éthoxy si Y représente le radical $S(O)_nR^2$,

W signifie hydrogène, méthyle, éthyle, méthoxyméthyle, méthoxy, fluor, chlore ou $S(O)_nCH_3$,

R signifie ($C_1$-$C_8$)-alkyle, halogéno-($C_1$-$C_8$)-alkyle, ($C_2$-$C_8$)-alcényle, halogène-($C_2$-$C_8$)-alcényle, ($C_2$-$C_8$)-alcynyle, halogène-($C_2$-$C_8$)-alcynyle, ces six radicaux mentionnés ci-dessus étant à chaque fois substitués par s radicaux du groupe constitué par cyano, S (O)$_n$-($C_1$-$C_6$) -alkyle, ($C_1$-$C_6$)-alcoxy, halogéno-($C_1$-$C_6$)-alcoxy, $COR^3$, $COOR^3$, $OCOR^3$, $NR^3COR^3$, $NR^3SO_2R^4$, ($C_3$-$C_6$)-cycloalkyle, hétéroaryle, hétérocyclyle et phényle, les trois derniers radicaux mentionnés étant à chaque fois substitués par s radicaux du groupe constitué par méthyle, éthyle, méthoxy, trifluorométhyle, cyano et halogéno, et hétérocyclyle portant 0 à 2 groupes oxo, ou

R signifie phényle substitué par s radicaux du groupe constitué par halogéno, nitro, cyano, ($C_1$-$C_6$)-alkyle, halogéno-($C_1$-$C_6$)-alkyle, ($C_3$-$C_6$)-cycloalkyle, $S(O)_n$-($C_1$-$C_6$)-alkyle, ($C_1$-$C_6$)-alcoxy, halogéno-($C_1$-$C_6$)-alcoxy, ($C_1$-$C_6$)-alcoxy-($C_1$-$C_4$)-alkyle,

$R^1$ signifie hydrogène, ($C_1$-$C_6$)-alkyle, ($C_2$-$C_6$)-alcényle, ($C_2$-$C_6$-alcynyle, ($C_3$-$C_6$-cycloalkyle, ($C_3$-$C_6$)-cycloalkyl-($C_1$-$C_6$)-alkyle, ($C_1$-$C_6$)-alkyl-O-($C_1$-$C_6$)-alkyle, phényle, phényl-($C_1$-$C_6$)-alkyle, hétéroaryle, ($C_1$-$C_6$)-alkyl-hétéroaryle, hétérocyclyle, ($C_1$-$C_6$)-alkyl-hétérocyclyle, ($C_1$-$C_6$)-alkyl-O-hétéroaryle, ($C_1$-$C_6$)-alkyl-O-hétérocyclyle, ($C_1$-$C_6$)-alkyl-$NR^3$-hétéroaryle ou ($C_1$-$C_6$)-alkyl-$NR^3$-hétérocyclyle, les seize derniers groupes mentionnés étant à chaque fois substitués par s radicaux du groupe constitué par cyano, halogéno, nitro, $OR^3$, $S(O)_nR^4$,

$N(R^3)_2$, $NR^3OR^3$, $COR^3$, $OCOR^3$, $NR^3COR^3$, $NR^3SO_2R^4$, $CO_2R^3$, $CON(R^3)_2$ et ($C_1$-$C_4$)-alcoxy-($C_2$-$C_6$)-alcoxy-carbonyle, et hétérocyclyle portant n groupes oxo,
$R^2$ signifie ($C_1$-$C_6$)-alkyle, ($C_3$-$C_6$-cycloalkyle ou ($C_3$-$C_6$)-cycloalkyl-($C_1$-$C_6$)-alkyle à chaque fois substitué par s radicaux du groupe constitué par halogéno et $OR^3$,
$R^3$ signifie hydrogène ou ($C_1$-$C_6$)-alkyle,
$R^4$ signifie ($C_1$-$C_6$)-alkyle,
$R^6$ et $R^7$ signifient, indépendamment l'un de l'autre, à chaque fois méthyle, éthyle ou n-propyle, ou
$R^6$ et $R^7$ forment, ensemble avec l'atome de soufre auquel ils sont liés, un cycle de 5 à 6 chaînons saturé qui contient, outre les atomes de carbone et outre l'atome de soufre du groupe sulfoximino, m atomes d'oxygène,
$R^8$ signifie méthyle, éthyle ou n-propyle,
$R^9$ signifie hydrogène ou cyano,
m vaut 0 ou 1,
n vaut 0, 1 ou 2,
s vaut 0, 1, 2 ou 3.

**4.** Agents herbicides, **caractérisés par** une teneur active en tant qu'herbicide en au moins un composé de formule (I) selon l'une quelconque des revendications 1 à 3.

**5.** Agents herbicides selon la revendication 4 en mélange avec des adjuvants de formulation.

**6.** Agents herbicides selon la revendication 4 ou 5 contenant au moins une autre substance active en tant que pesticide du groupe formé par les insecticides, les acaricides, les herbicides, les fongicides, les phytoprotecteurs et les régulateurs de croissance.

**7.** Agents herbicides selon la revendication 6 contenant un phytoprotecteur.

**8.** Agents herbicides selon la revendication 7, contenant du cyprosulfamide, du cloquintocet-mexyl, du méfenpyr-diéthyle ou de l'isoxadifen-éthyl.

**9.** Agents herbicides selon l'une quelconque des revendications 6 à 8 contenant un autre herbicide.

**10.** Procédé pour lutter contre des plantes non souhaitées, **caractérisé en ce qu'**on applique une quantité active d'au moins un composé de formule (I) selon l'une quelconque des revendications 1 à 3 ou d'un agent herbicide selon l'une quelconque des revendications 4 à 9 sur les plantes ou sur le lieu de la croissance non souhaitée des plantes.

**11.** Utilisation de composés de formule (I) selon l'une quelconque des revendications 1 à 3 ou d'agents herbicides selon l'une quelconque des revendications 4 à 9 pour lutter contre des plantes non souhaitées.

**12.** Utilisation selon la revendication 11, **caractérisée en ce que** les composés de formule (I) sont utilisés pour lutter contre des plantes non souhaitées dans les cultures de plantes utiles.

**13.** Utilisation selon la revendication 12, **caractérisée en ce que** les plantes utiles sont des plantes utiles transgéniques.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2012028579 A1
- EP 11158253 A
- EP 11159115 A
- EP 0221044 A
- EP 0131624 A
- WO 9211376 A
- WO 9214827 A
- WO 9119806 A
- EP 0242236 A
- EP 242246 A
- WO 9200377 A
- EP 0257993 A
- US 5013659 A
- EP 0142924 A
- EP 0193259 A
- WO 9113972 A
- EP 309862 A
- EP 0464461 A
- EP 0305398 A


### In der Beschreibung aufgeführte Nicht-Patentliteratur


- **D. TIEBES.** Combinatorial Chemistry - Synthesis, Analysis, Screening. Verlag Wiley, 1999, 1-34
- Polymer-Supported Scavengers and Reagents for Solution-Phase Synthesis. ChemFiles. Sigma-Aldrich, vol. 4
- **BARRY A. BUNIN.** The Combinatorial Index. Verlag Academic Press, 1998
- Combinatorial Chemistry - Synthesis, Analysis, Screening. Verlag Wiley, 1999
- Microwaves in Organic and Medicinal Chemistry. Verlag Wiley, 2005
- Gene Transfer to Plants, Springer Lab Manual. Springer Verlag Berlin, 1995
- **CHRISTOU.** *Trends in Plant Science,* 1996, vol. 1, 423-431
- **B. SAMBROOK et al.** Molecular Cloning, A Laboratory Manual. Cold Spring Harbor Laboratory Press, 1989
- **WINNACKER.** Gene und Klone. VCH Weinheim, 1996
- **BRAUN et al.** *EMBO J.,* 1992, vol. 11, 3219-3227
- **WOLTER et al.** *Proc. Natl. Acad. Sci. USA,* 1988, vol. 85, 846-850
- **SONNEWALD et al.** *Plant J.,* 1991, vol. 1, 95-106
- **WINNACKER-KÜCHLER.** Chemische Technologie. C. Hanser Verlag, 1986, vol. 7
- **WADE VAN VALKENBURG.** Pesticide Formulations. Marcel Dekker, 1973
- Spray Drying. **K. MARTENS.** Handbook. G. Goodwin Ltd, 1979
- **WATKINS.** Handbook of Insecticide Dust Diluents and Carriers. Darland Books
- **H.V. OLPHEN.** Introduction to Clay Colloid Chemistry. J. Wiley & Sons
- **C. MARSDEN.** Solvents Guide. Interscience, 1963
- **MCCUTCHEON'S.** Detergents and Emulsifiers Annual. MC Publ. Corp,
- **SISLEY ; WOOD.** Encyclopedia of Surface Active Agents. Chem. Publ. Co. Inc, 1964
- **SCHÖNFELDT.** Grenzflächenaktive Äthylenoxidaddukte. Wiss. Verlagsgesell, 1976
- **VERFAHREN.** Spray-Drying Handbook. G. Goodwin Ltd, 1979
- **J.E. BROWNING.** Agglomeration. *Chemical and Engineering,* 1967, 147 ff
- Perry's Chemical Engineer's Handbook. McGraw-Hill, 1973, 8-57
- **G.C. KLINGMAN.** Weed Control as a Science. John Wiley and Sons, Inc, 1961, 81-96
- **J.D. FREYER ; S.A. EVANS.** Weed Control Handbook. Blackwell Scientific Publications, 1968, 101-103